# EUROPEAN PATENT APPLICATION

(11) **EP 4 001 265 A1**
(43) Date of publication of application: **25.05.2022**
(21) Application number: 21198145.1
(22) Date of filing: 21.09.2021
(51) Int. Cl.: C07D 211/14, C07C 237/06, C07C 237/10, C07C 239/18, C07C 255/24, C07D 295/15, C07D 303/16, C07F 7/18

(54) **STRUCTURE OF ADJUSTABLE STERIC HINDRANCE WEAK BASIC LIGHT STABILIZER AND PREPARATION METHOD AND APPLICATION THEREOF**

(30) Priority: 11.11.2020 CN 202011250638
(71) Applicant: Shaoxing Ruikang Biotechnologies Co., Inc, Yuecheng District Shaoxing Zhejiang 312000 (CN)
(72) Inventor: MAO, Lijuan, SHAOXING, Zhejiang 312000 (CN); LIU, Shubai, SHAOXING, Zhejiang 312000 (CN); ZHAO, Chengshi, SHAOXING, Zhejiang 312000 (CN); YIN, Qiwei, SHAOXING, Zhejiang 312000 (CN); DING, Yifan, SHAOXING, Zhejiang 312000 (CN); WANG, Yifei, SHAOXING, Zhejiang 312000 (CN); LUO, Rui, SHAOXING, Zhejiang 312000 (CN); CHEN, Xiuying, SHAOXING, Zhejiang 312000 (CN); WANG, Jijiang, SHAOXING, Zhejiang 312000 (CN); LI, Jing, SHAOXING, Zhejiang 312000 (CN); XU, Chunjuan, SHAOXING, Zhejiang 312000 (CN); SONG, Jinge, SHAOXING, Zhejiang 312000 (CN); CHEN, Jun, SHAOXING, Zhejiang 312000 (CN); YU, Meiya, SHAOXING, Zhejiang 312000 (CN)
(74) Representative: JD&P Patent Attorneys Joanna Dargiewicz & Partners

(57) **Abstract**

The field of new compounds and synthesis methods thereof are related, and particularly to a structure of a steric hindrance adjustable weak base light stabilizer and a preparation method and application thereof. According to the innovative light stabilizer, a steric hindrance thereof is adjusted by establishing substituent generating the steric hindrance around nitrogen atoms; moreover, an electronegativity of the nitrogen atom can be influenced by adjusting a distance of a polar group, so that an alkalinity or a nucleophilicity of the nitrogen atom is adjusted. A desired effect is obtained by adjusting a steric hindrance and a nucleophilic property or an alkalinity where the nitrogen atom is located, so that an application range of the innovative light stabilizer is widened, and the innovative light stabilizer is suitable for PC, polyester, PVC and other slightly acidic or certain electrophilic polymer materials to serve as a light stability protecting aid.

## Description

### TECHNICAL FIELD

The invention belongs to the field of new compounds and synthesis methods thereof, and particularly relates to a structure of a steric hindrance adjustable weak base light stabilizer and a preparation method and application thereof.

### BACKGROUND

Polymer materials play an increasingly important role in the developed world today, and are ubiquitous from different industrial disposable products molded once simply to high-tech components used in space. Such diverse and complex applications require that different physical and chemical properties of the polymer material must meet the requirements arising from the diversity required by specific applications. Therefore, the polymer materials become more and more complex, and are not only composed of various basic polymers, but also need to be added with a large number of various additives, including different functional additives, which play a decisive role in endowing the polymer materials with unique properties. Among the polymer additives, an antioxidant stabilizer is the most important one, with a function of efficiently providing a polymer with resistance to degradation caused by heat, other environmental oxidation factors and ultraviolet light during machining and using. This is of great significance to the polymer materials, and according to the quality of the polymer antioxidant stabilizer, a service life of products may be directly predicted, and a vital negative impact caused by product failure may be avoided.

Light stabilizers are playing a more and more important role in the antioxidant stabilizers of the polymer materials, which can provide very effective thermal degradation and light degradation protection effects for the polymer.

The light stabilizer series are divided into three types in a working mechanism of polymer protection: protection: an ultra violet absorber (UVA), a hindered amine light stabilizer (HALS), and quenchers. In practical application, according to a light intensity and a required protection degree, these three light stabilizers may be used separately or as a mixture. The ultra violet absorber (UVA) absorbs and filters out harmful ultraviolet rays, and converts the same into heat energy, electromagnetic waves and harmless long-band light, which is beneficial for preventing the degradation of the polymer materials, especially the discoloration and delamination of light-sensitive coatings, adhesives and sealants.

For the hindered amine light stabilizer, LS-744 was first developed by Mitsubishi Corporation of Japan in 1970s, which was namely 2,2,6,6-tetramethylpiperidine benzoate, and a same product was synthesized by Ciba-Geigy Company of Switzerland in 1974. The protection effect of the light stabilizer polymer materials is more than 4 times that of traditional absorption-type, with a good compatibility. The annual consumption growth rate of the hindered amine light stabilizer in the world is 20% to 30%, and the total consumption has accounted for 44% of the total polymer stabilizers, ranking first among all kinds of stabilizers. According to the report of the third-party forecast of the global polymer stabilizer market in 2019, the light stabilizer reached 790 billion US dollars in 2019 in the market, and will reach 1,415.47 billion US dollars in 2027 in the market, with an annual growth rate of about 7.6%. In recent decades, due to the continuously expanding demand field of the hindered amine light stabilizer, the innovative research and development has been very active, and new products of small molecules and oligomeric molecules are constantly appearing, but the products are all based on a hindered amine parent core structure.

An active functional group structure of the hindered amine light stabilizer is as follows:

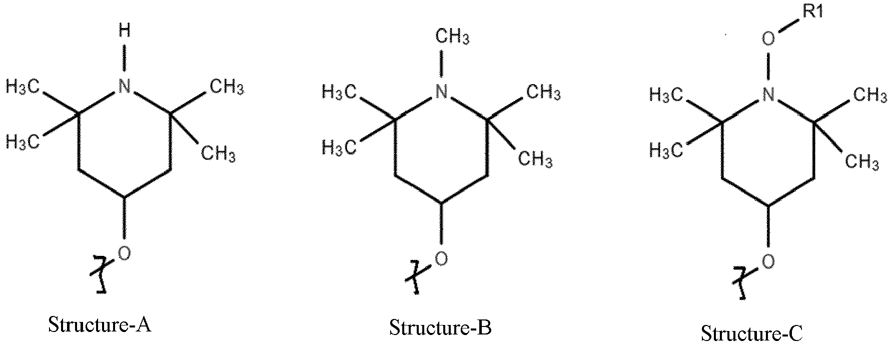

In the general structural formula of the active functional group of the hindered amine parent core above, in the most common hindered amine light stabilizers in the market, the hindered amine parent core structure - A-type light stabilizer has the lowest cost and is most widely used. The most common products of the hindered amine light stabilizer in the market are as follows.

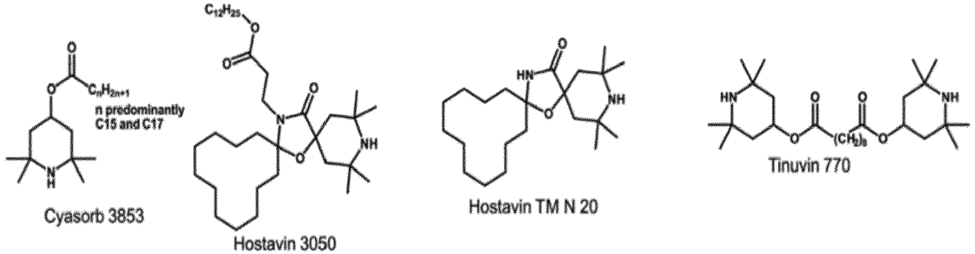

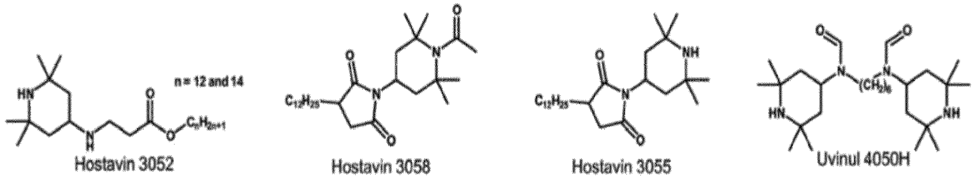

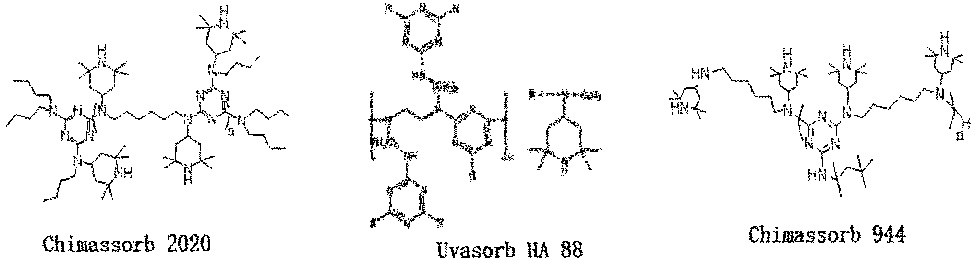

However, this kind of hindered amine light stabilizer with the hindered amine parent core structure cannot be applied to polymers such as PVC, PC, PU and polyester. The main reason is that an N-H bond in this kind of hindered amine parent core has relatively strong alkalinity and nucleophilicity, and nitrogen atoms with a nucleophilicity are easy to react with electrophilic or acidic functional groups on the polymers during machining and using, for example, a nucleophilic reaction with -CH2-Cl in PVC changes a property of PVC. Similar nucleophilic reactions may also occur on a carbonate bond in PC, an amide bond in PU and a carboxylate bond in polyester. Therefore, this kind of hindered amine light stabilizer with the relatively strong basicity cannot be applied to polymers with slight acidity or certain electrophilicity.

The only way to widen the application range of the hindered amine light stabilizer is to reduce the basicity or the nucleophilicity of the hindered amine HALS parent core. Up to now, there are two ways to reduce the basicity of nitrogen atoms in hindered amine HALS piperidine amine: (1) alkylation is carried out on an N-H bond of the piperidine amine to form an N-R bond, which increases an empty resistance effect around the nitrogen atoms in the piperidine amine, thus achieving the purpose of reducing the basicity of the piperidine amine. (2) Alkoxy is introduced into the N-H bond of the piperidine amine to form an N-OR bond, so that an alkalinity of the nitrogen atoms in the piperidine amine is reduced by double effects of electronegativity reduction and improvement of a steric hindrance effect around the nitrogen atoms.

The hindered amine HALS light stabilizer is a kind of free radical scavenger, with a very complex action mechanism, and a light protection effect is mainly achieved through a synergistic effect of the following mechanisms.
(1) Capturing of free radicals: the hindered amine functional groups are of an alicyclic amine structure, which may be converted into free radicals of nitroxide NO after absorbing light energy in an aerobic state. These free radicals of nitroxide can not only capture active free radicals of alkyl generated during photooxidation degradation of polymer materials, but also have a regeneration function during photostabilization, thus inhibiting a chain reaction to achieve the purpose of protection.
(2) Decomposition of hydroperoxide: amido is combined with hydrogen in a hydroperoxide and then decomposed into the free radicals of nitroxide, which then react with active free radicals to convert into a stable alcohol and ketone compound. The research results of Carlsson also confirm that the hindered amine has a concentration effect[5] around the hydroperoxide.
(3) Capturing of heavy metal: the nitrogen in the piperidine amine has lone pair electrons coordinated with metal, which can coordinate with metal ions in the polymer materials efficiently, thus playing a role in protecting the polymer materials.

It is reported in the literature how 2,2,6,6-tetramethylpiperidine amine and a derivative weak base light stabilizer protect a polymer-material-mechanism-based phenylethyl ester, wherein a Denisov circulation mechanism is widely recognized[7]. A DENISOV circulation protection mechanism of photodegradable polymers of a 2,2,6,6-tetramethylpiperidine amine light stabilizer is shown in the following drawing.

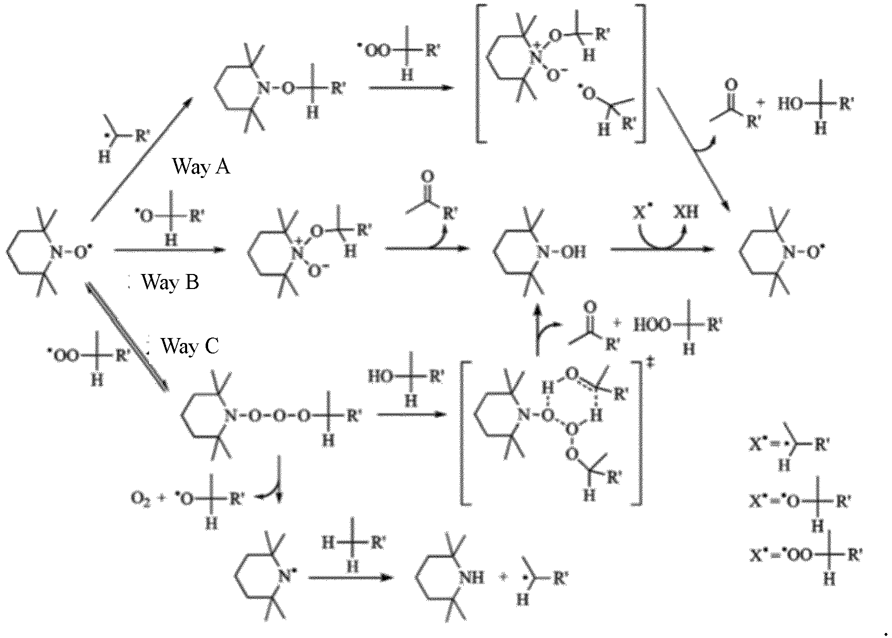

It can be seen from the DENISOV circulation protection mechanism that tetramethylpiperidine amine is an oxide, and how to transfer the free radicals on the polymers beginning to degrade through three possible ways A, B and C so that the polymer materials are protected is a working principle of circulating release of the piperidine amine monooxide.

Therefore, piperidylamine hindered amine light stabilizers with structure-B and structure-C patent cores are called a weak base hindered amine light stabilizer, and some N-R and N-OR light stabilizer products have been applied to the market as follows.

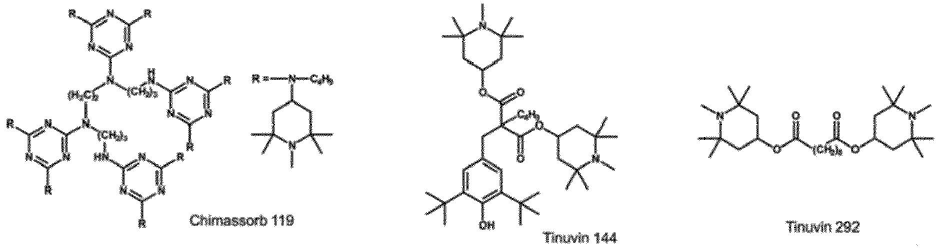

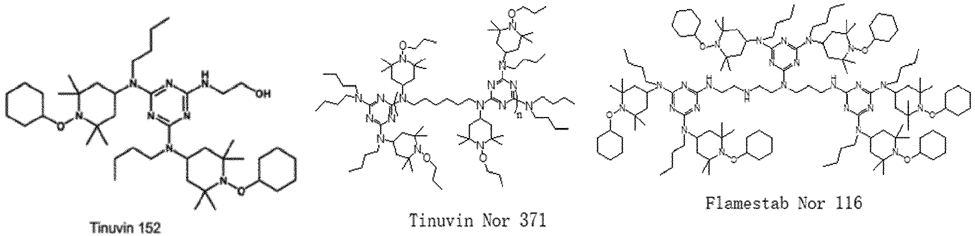

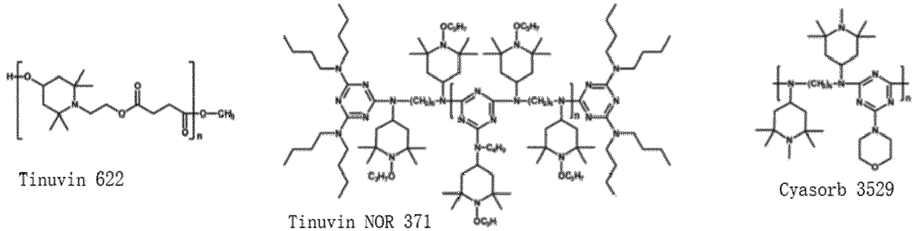

A structure of weak base hindered amine shown in the above structural formula is more complex than that of conventional hindered amine. In fact, both small molecule weak base hindered amine light stabilizer and oligomeric weak base hindered amine light stabilizer have to go through an additional synthesis step to obtain these products compared with the conventional hindered amine. Therefore, a cost price may be higher, and extra chemical synthesis may also bring larger pressure to green environmental protection.

An ultraviolet quencher is generally an organic metal compound or complex, which may restore excited polymers to a stable ground state by energy transfer, with a characteristic of small consumption, but a possible residual toxicity problem of heavy metal has not yet been determined. Therefore, the application has not been widely promoted.

### SUMMARY

The patent for invention reports an innovative structural light stabilizer with adjustable steric hindrance, controllable weak base property of nitrogen atoms and no 2,2,6,6-tetramethylpiperidine amine structural fragment for the first time. The patent for invention discloses seven series of innovative steric hindrance adjustable novel structural light stabilizers.

General formulas of an innovative small molecule light stabilizer are as follows:
Structural general formula 1: 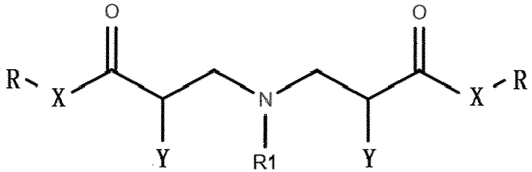
   wherein, in the structural formula 1, X is NH, NR3, or O;
   Y is H, methyl, or other alkyl;
   R is 5-22 linear alkyl or branched alkyl, and R may also be -(CH₂)nSi(OMe)₃, or -(CH₂)nSi(OEt)₃, and n is 2, 3, 4, or 5;
   R1 may be C1-C20 linear alkyl, or branched alkyl, and R1 may also be double-bond substituted alkyl, such as allyl; R1 may also be heteroatom substituted alkyl, such as hydroxyethyl and hydroxypropyl; R1 may also be hydroxyl (-OH) or alkoxy (OR); R1 may also be i-Pr, i-Bu, isoamyl, isooctyl, cyclohexyl, substituted cyclohexyl, cyclopentyl, benzyl, substituted benzyl, allyl, substituted allyl, double-bond contained alkyl, aryl substituted alkyl, or 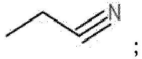 R1 may also be -(CH2)n-NR4R5; and R4 and R5 are methyl, ethyl, propyl, isopropyl, butyl, isobutyl, cyclohexyl, or benzyl, such as -CH2CH2NPrⁱPrⁱ or -CH2CH2NMe₂.
   R1 may also be -(CH2)n-Si(OEt)₃, and n is 2, 3, 4, or 5.
   When R1 is neither -(CH2)n-NR4R5 nor -(CH2)n-Si(OR)₃, R and R1 may be the same or different.
Structural general formula 2: 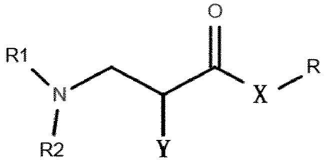
   wherein, in the structural formula 2, X is NH, NR3, or O;
   Y is H, methyl, ethyl, or other alkyl side chains;
   R is 5-22 carbon linear or branched alkyl;
   R1 and R2 may be linear alkyl, such as: -(CH2)nCH3, wherein n is a natural number ranging from 1 to 20, and may also be i-Pr, i-Bu, isoamyl, isooctyl, cyclohexyl, substituted cyclohexyl, cyclopentyl, benzyl, substituted benzyl, allyl, substituted allyl, double-bond contained alkyl, aryl substituted alkyl, or 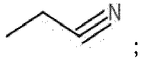 and R1 and R2 may both be 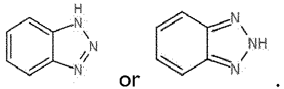
   R1 and R2 may be the same or different; and R may be the same as or different from R1 and R2.
   When R1 and R2 are different, R1 is Et, i-Pr, n-Pr, Bu, i-Bu, or C5-12 alkyl; and
   R2 is 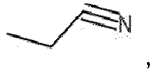 hydroxyethyl or hydroxypropyl.
Structural general formula 3: 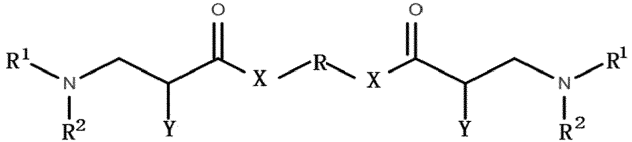
   wherein, in the structural formula 3, X is NH, NR3, or O;
   Y is H, methyl, ethyl, or other alkyl;
   R is -(CH2)n-, wherein n ranges from 2 to 22, and R may also be alkyl or aryl side chain substituted -(CH2)n-, or dibenzylamine.
   R1 and R2 may also be alkyl, such as: -(CH2)nCH3, wherein n is a natural number ranging from 0 to 20, and may also be i-Pr, i-Bu, isoamyl, isooctyl, cyclohexyl, substituted cyclohexyl, cyclopentyl, benzyl, substituted benzyl, allyl, substituted allyl, double-bond contained alkyl, aryl substituted alkyl, or 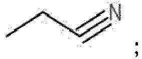 and R1 and R2 may both be 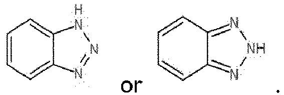
   R1 and R2 may be the same or different. When R1 and R2 are different, R1 is Et, i-Pr, n-Pr, Bu, i-Bu, or C5-12 alkyl; and
   R2 is 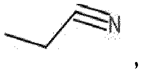 or hydroxyethyl or hydroxypropyl.
Structural general formula 4: 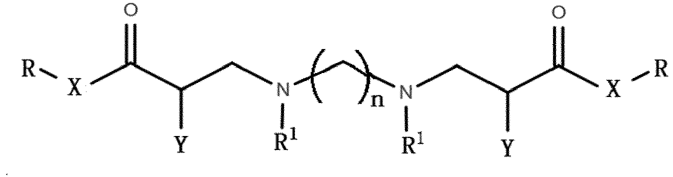
   wherein, in the structural formula 4, X is NH, NR3, or O;
   Y is H, methyl, ethyl, or other alkyl;
   n is 2-18 linear paraffin -(CH2)n-, and may also be side chain alkyl or aryl substituted alkane;
   R1 is methyl, ethyl, propyl, or butyl; and may also be i-Pr, i-Bu, cyclohexyl, substituted cyclohexyl, cyclopentyl, benzyl, substituted benzyl, allyl, substituted allyl, double-bond contained alkyl, or aryl substituted alkyl. R2 may also be 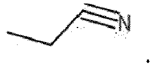
   R is C5-C20 linear or branched alkyl, and may also be alkyl side chain and aryl substituted alkyl; and R may also be -CH2CH2CH2-Si(OMe)₃ or -CH2CH2CH2-Si(OEt)₃.
Structural general formula 5: 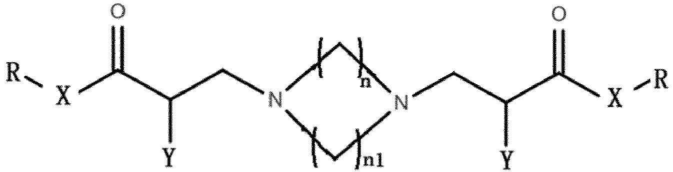
   wherein, in the structural formula 5, X is NH, NR3, or O;
   Y is H, methyl, or other alkyl;
   n is 1, 2, 3, 4, or 5; and n1 is 1, 2, 3, 4, or 5;
   n may be equal to n1, and n may not equal to n1;
   R is C5-C20 linear or branched alkyl, and may also be alkyl side chain and aryl substituted alkyl; and
   R may also be -CH2CH2CH2-Si(OMe)₃ or -CH2CH2CH2-Si(OEt)₃.

Structural general formulas of an innovative oligomer light stabilizer are as follows:
Structural general formula 6: 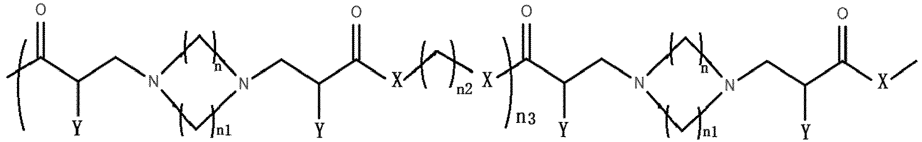
   wherein, in the structural formula 6, X is NH, NR3, or O;
   Y is H, methyl, ethyl, or other alkyl;
   n is 1, 2, 3, 4, or 5; and n1 is 1, 2, 3, 4, or 5;
   n may be equal to n1, and n may not equal to n1;
   n2 ranges from 2 to 18; and
   n3 may range from 2 to 35, which is a polymerization degree of an oligomer.
Structural general formula 7: 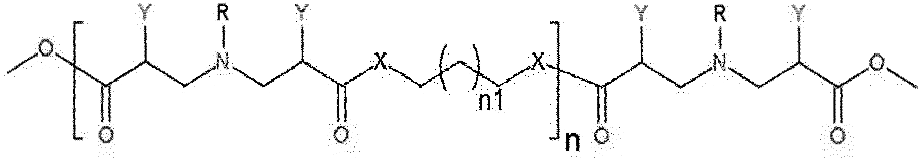
   wherein, in the structural formula 7, X is NH, NR3, or O;
   Y is H, methyl, ethyl, or other alkyl;
   n1 is 1, 2, 3, 4, 5, 6, 7, 8, 9, 10, 11, 12, 13, 14,15, 16, 17, 18,19, 20, 21, or 22;
   n ranges from 1 to 15, which is a polymerization degree of an oligomer.
   n may be equal to n1, and n may not equal to n1;
   R may be C2-C18 linear or branched alkane, such as n-propyl, n-butyl, n-pentyl, n-hexyl, n-nonyl, and n-octyl; and may also be isopropyl, isobutyl, isopentyl, isohexyl, isooctyl, isodecyl, etc.; and
   R may also be double-bond contained alkyl, such as allyl, etc.; may also be heteroatom contained substituent, such as hydroxyethyl, hydroxypropyl, etc.; and may also be -OH or -OR1 (R1 is alkoxy, and the alkyl herein may be linear or branched alkyl).

A preparation method of the structure of the above steric hindrance adjustable weak base light stabilizer includes the reaction formulas as follows:
structural general formula 1: 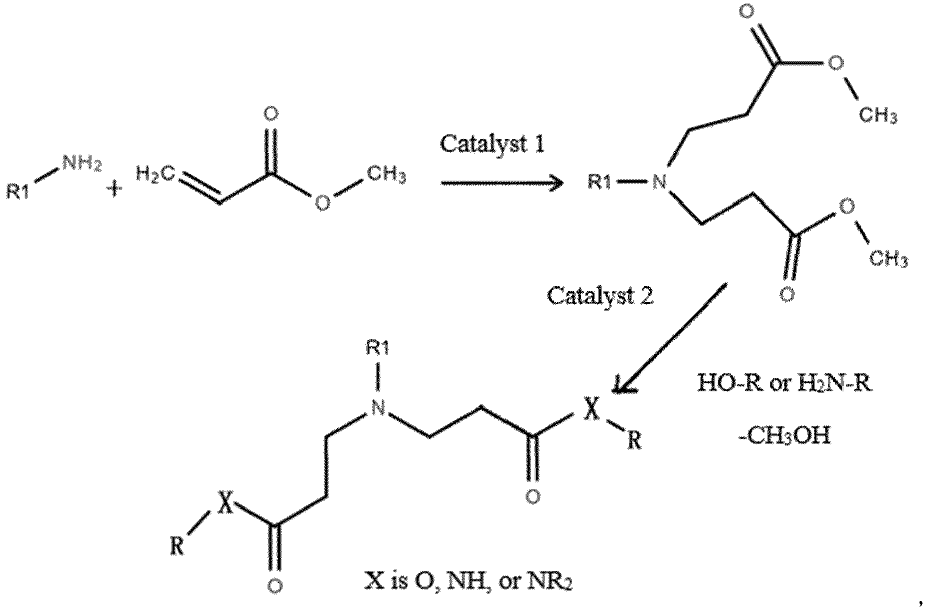
structural general formula 2: 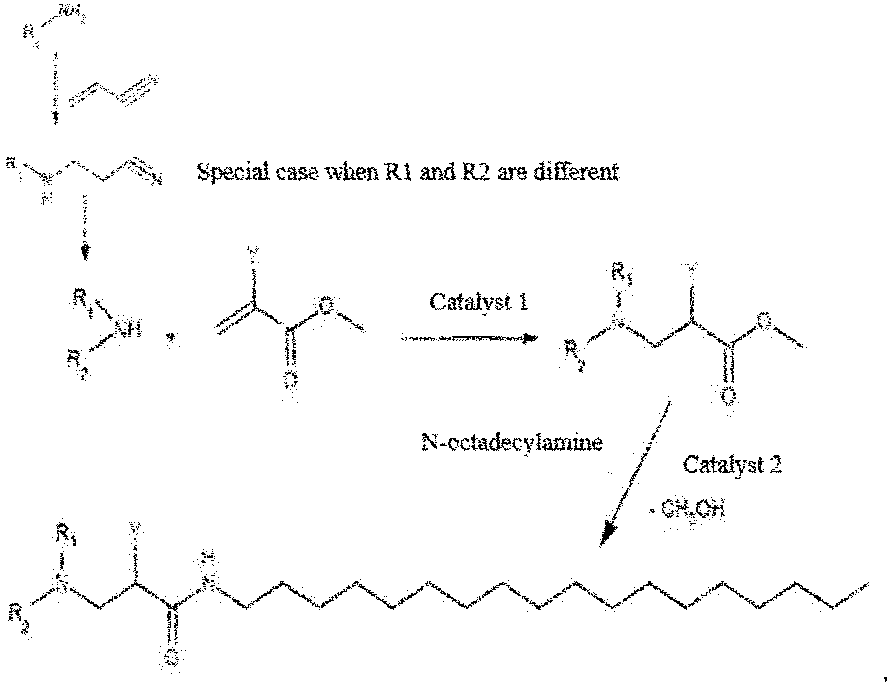
structural general formula 3: 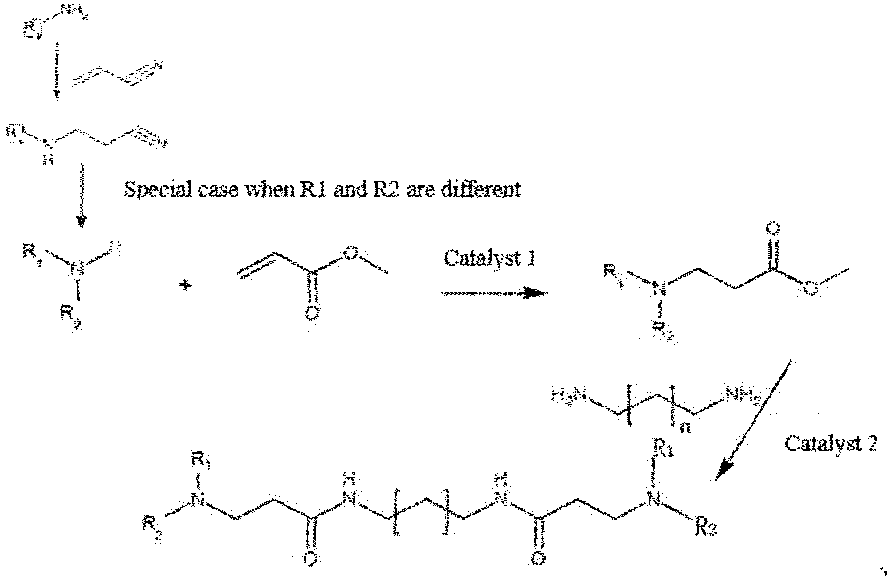
structural general formula 4: 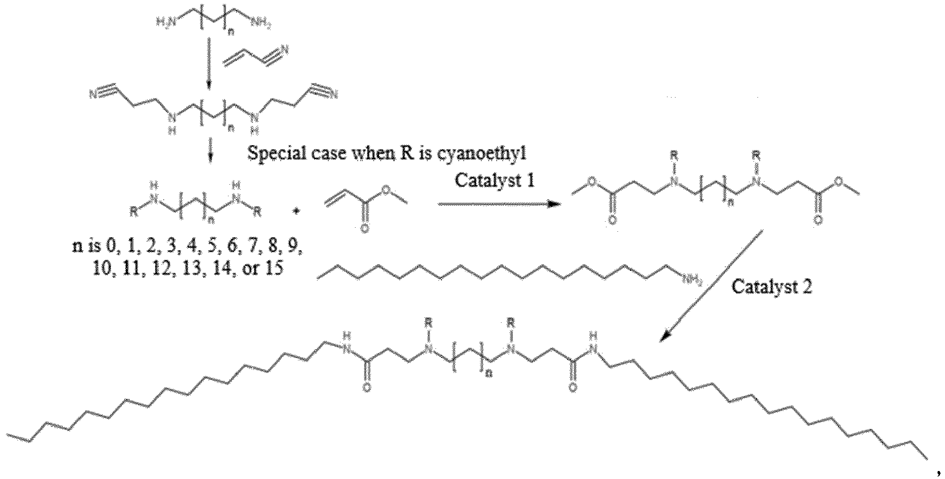
structural general formula 5: 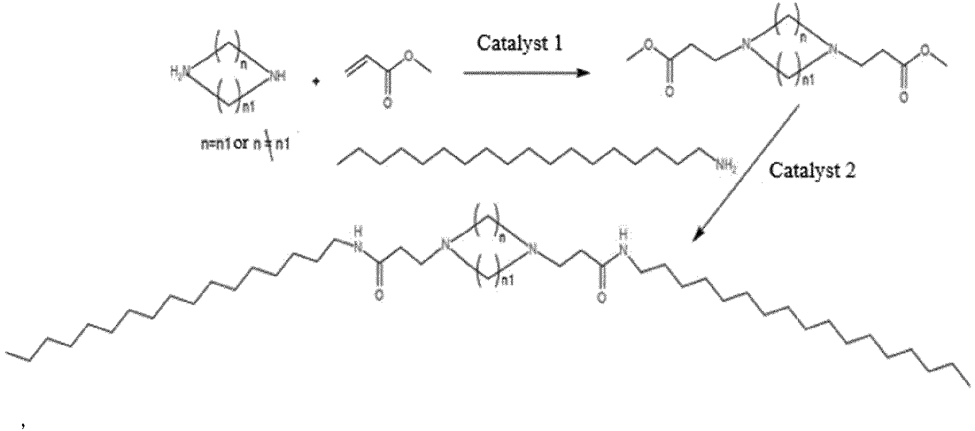
structural general formula 6: 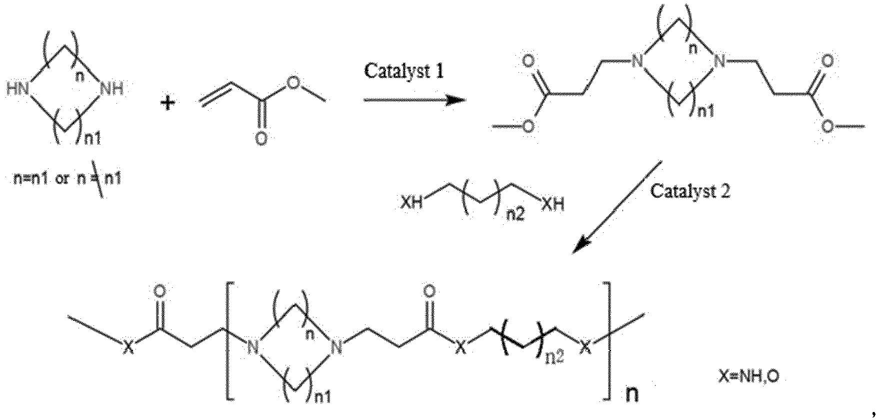
structural general formula 7:

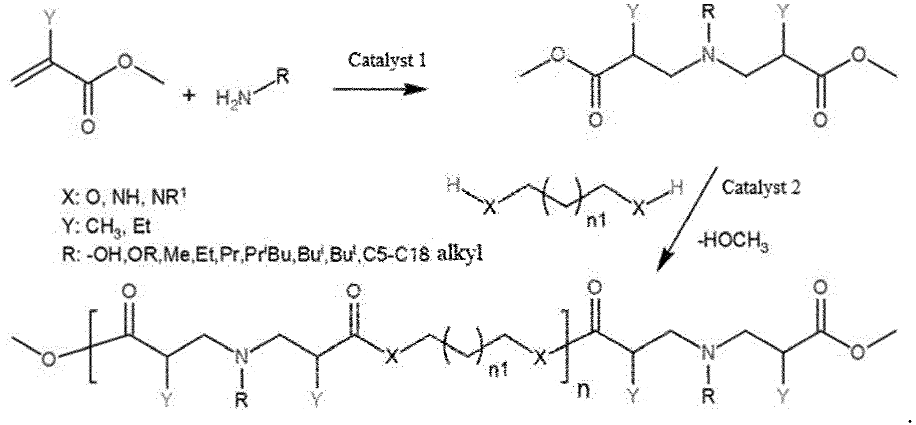

The preparation method of the structure of the above steric hindrance adjustable weak base light stabilizer includes the specific steps as follows:
a preparation method of the structural general formula 1:
   (1) under the protection of nitrogen, adding methyl acrylate or methyl methacrylate into a reaction flask, starting stirring, adding methanol, or ethanol, or acetone, or ethyl acetate, or dichloroethane, or DMF, or not adding any solvent, adding a catalyst 1, wherein the catalyst 1 is acetic acid, or acidic alumina, or silica gel, or ortho-methoxyhydroquinone, or 4,4'-benzophenone, or m-nitrophenol, or silica gel sulfate, cooling to 5°C to 10°C, and then dropwise adding first amine slowly; heating to a room temperature after dropwise adding, stirring, and heating to continue the reaction; monitoring the reaction process by TLC until the reaction is complete, removing excessive methyl acrylate under vacuum, and using the remaining reaction intermediate without further purification for next reaction;
   (2) under the protection of nitrogen and stirring, adding 0.1% to 5% of catalyst 2 into the intermediate obtained in the first step at a room temperature, wherein the catalyst 2 is sodium methoxide, sodium formate, diethyl tin oxide or aluminum isooctanol, then adding n-dodecylamine, or n-octadecylamine, or n-dodecanol, or n-octadecanol in batches, after finishing adding, heating to 40°C to 70°C for reaction for 5 hours to 16 hours, continuously heating to 85°C to 120°C for reaction for 30 hours to 96 hours, and monitoring the reaction process by TLC until the reaction is complete; and adding ethanol, or ethyl acetate or petroleum ether containing 5% to 15% of water for recrystallizing, filtering to yield a white powder solid product, and drying, wherein a yield ranges from 85% to 97%;
a preparation method of the structural general formula 2:
   (1) under the protection of nitrogen, adding methyl acrylate or methyl methacrylate into a reaction flask, starting stirring, then adding 1 part to 3 parts of methanol, or ethanol, or ethyl acetate, or dichloroethane, or acetone, or acetonitrile or DMF, or not adding any solvent, adding 0.02% to 30% of catalyst 1, wherein the catalyst 1 is acetic acid, or acidic alumina, or silica gel, or ortho-methoxyhydroquinone, or 4,4-diphenol hydroxy dibenzophenone, or m-nitrophenol, cooling to 5°C to 10°C, and then dropwise adding second amine slowly; heating to a room temperature after dropwise adding, stirring for 6 hours to 24 hours, and if the reaction needs to be continued, continuously heating to 40°C to 80°C to continue the reaction for 5 hours to 18 hours; monitoring the reaction process by TLC until the reaction is complete, removing excessive methyl acrylate under vacuum, and using the remaining reaction intermediate without further purification for next reaction;
   (2) under the protection of nitrogen and stirring, adding 0.1% to 5% of catalyst 2 into the intermediate obtained in the first step at a room temperature, wherein the catalyst 2 is sodium methoxide, sodium formate, diethyl tin oxide or aluminum isooctanol, then adding n-dodecylamine, or n-hexadecylamine, or n-octadecylamine, or n-dodecanol, or n-octadecanol in batches, after finishing adding, heating to 40°C to 70°C for reaction for 8 hours to 16 hours, continuously heating to 85°C to 140°C for reaction for 48 hours to 96 hours, and monitoring the reaction process by TLC until the reaction is complete; and removing the catalyst, adding ethanol, or methanol, or ethyl acetate, or petroleum ether containing 0.5% to 20% of water for recrystallizing, filtering to yield a white powder solid product, and drying, wherein a yield ranges from 87% to 96%;
a preparation method of the structural general formula 3:
   (1) under the protection of nitrogen, adding methyl acrylate or methyl methacrylate into a reaction flask, starting stirring, then adding 1 part to 3 parts of methanol, or ethanol, or ethyl acetate, or dichloroethane, or acetone, or acetonitrile, or DMF, or not adding any solvent, then adding 0.01% to 30% of catalyst 1, wherein the catalyst 1 is acetic acid, or acidic alumina, or silica gel, or ortho-methoxyhydroquinone, or 4,4-diphenol hydroxy dibenzophenone, or m-nitrophenol, cooling to 5°C to 20°C, and then dropwise adding second amine slowly; heating to a room temperature after dropwise adding, stirring for 5 hours to 24 hours, and if the reaction needs to be continued, continuously heating to 40°C to 80°C to continue the reaction for 5 hours to 18 hours; monitoring the reaction process by TLC until the reaction is complete, removing excessive methyl acrylate under vacuum, and using the remaining reaction intermediate without further purification for next reaction;
   (2) under the protection of nitrogen and stirring, adding alkyl diamine, such as ethylenediamine, or butanediamine, or hexamethylenediamine, or decanediamine into the intermediate obtained in the first step at a room temperature, then adding 0.1% to 5% of catalyst 2, wherein the catalyst 2 is sodium methoxide, sodium formate, diethyl tin oxide or aluminum isooctanol, after finishing adding, heating to 50°C to 70°C for reaction for 6 hours to 16 hours, continuously heating to 85°C to 120°C for reaction for 48 hours to 96 hours, and monitoring the reaction process by TLC until the reaction is complete; and removing the catalyst, adding ethanol, or methanol, or ethyl acetate, or petroleum ether containing 0.5% to 20% of water for recrystallizing, filtering to yield a white powder solid product, and drying, wherein a yield ranges from 85% to 95%;
a preparation method of the structural general formula 4:
   (1) under the protection of nitrogen, adding methyl acrylate or methyl methacrylate into a reaction flask, starting stirring, then adding 1 part to 3 parts of methanol, or ethanol, or ethyl acetate, or dichloroethane, or acetone, or acetonitrile, or DMF, or not adding any solvent, then adding 100 ppm to 1000 ppm o-methoxyhydroquinone, or 4,4-diphenol hydroxy dibenzophenone, or m-nitrophenol, cooling to 5°C to 20°C, and then dropwise adding second amine slowly; heating to a room temperature after dropwise adding, stirring for 8 hours to 32 hours, and if the reaction needs to be continued, continuously heating to 40°C to 60°C to continue the reaction for 5 hours to 24 hours; monitoring the reaction process by TLC until the reaction is complete, removing excessive methyl acrylate under vacuum, and using the remaining reaction intermediate without further purification for next reaction;
   (2) under the protection of nitrogen and stirring, adding dodecylamine, or hexadecylamine, or octadecylamine, or hexadecanol, or octadecanol, or dodecanol in batches first into the intermediate obtained in the first step at a room temperature, then adding 0.02% to 5% of catalyst 2, wherein the catalyst 2 is sodium methoxide, or sodium formate, or diethyl tin oxide, or aluminum isooctanol, after finishing adding, heating to 50°C to 70°C for reaction for 8 hours to 10 hours, continuously heating to 80°C to 120°C for reaction for 48 hours to 96 hours, and monitoring the reaction process by TLC until the reaction is complete; and removing the catalyst, adding ethanol, or methanol, or ethyl acetate, or petroleum ether containing 0.5% to 20% of water for recrystallizing, filtering to yield a white powder solid product, and drying, wherein a yield ranges from 83% to 96%;
a preparation method of the structural general formula 5:
   (1) under the protection of nitrogen, adding methyl acrylate or methyl methacrylate into a reaction flask, starting stirring, then adding 1 part to 3 parts of methanol, or ethanol, or ethyl acetate, or dichloroethane, or acetone, or acetonitrile, or DMF, or not adding any solvent, then adding 100 ppm to 1000 ppm o-methoxyhydroquinone, or 4,4-diphenol hydroxy dibenzophenone, or m-nitrophenol, cooling to 10°C to 20°C, and then dropwise adding cyclic alkyl diamine slowly; heating to a room temperature after dropwise adding, stirring for 3 hours to 5 hours, heating to 40°C to 60°C to continue the reaction for 10 hours to 24 hours; monitoring the reaction process by TLC until the reaction is complete, removing excessive methyl acrylate under vacuum, and using the remaining reaction intermediate without further purification for next reaction;
   (2) under the protection of nitrogen and stirring, adding octadecylamine, or dodecylamine, or hexadecylamine in batches first into the intermediate obtained in the first step at a room temperature, then adding 0.1% to 0.5% of sodium methoxide or sodium formate, or not adding any catalyst, after finishing adding, heating to 50°C to 60°C for reaction for 5 hours to 8 hours, continuously heating to 80°C to 120°C for reaction for 48 hours to 72 hours, and monitoring the reaction process by TLC until the reaction is complete; and removing the catalyst, adding ethanol or methanol containing 5% to 10% of water for recrystallizing, filtering to yield a white powder solid product, and drying, wherein a yield ranges from 83% to 92%;
a preparation method of the structural general formula 6:
   (1) under the protection of nitrogen, adding methyl acrylate or methyl methacrylate into a reaction flask, starting stirring, then adding 1 part to 3 parts of methanol, or ethanol, or ethyl acetate, or dichloroethane, or acetone, or acetonitrile, or DMF, or not adding any solvent, then adding 100 ppm to 1000 ppm o-methoxyhydroquinone, or 4,4-diphenol hydroxy dibenzophenone, or m-nitrophenol, cooling to 10°C to 20°C, and then dropwise adding alkyl cyclic diamine slowly; heating to a room temperature after dropwise adding, stirring for 5 hours to 8 hours, heating to 45°C to 70°C to continue the reaction for 10 hours to 24 hours; monitoring the reaction process by TLC until the reaction is complete, removing excessive methyl acrylate under vacuum, and using the remaining reaction intermediate without further purification for next reaction;
   (2) under the protection of nitrogen and stirring, adding pentanediamine, or hexamethylene diamine, or decanediamine, or hexanediol, or octanediol, or decanediol in batches first into the intermediate obtained in the first step at a room temperature, then adding 0.01% to 5% of sodium methoxide or sodium formate, or not adding any catalyst, or diethyl tin oxide, or aluminum alkoxide, after finishing adding, heating to 50°C to 70°C for reaction for 5 hours to 8 hours, continuously heating to 80°C to 130°C for reaction for 48 hours to 96 hours, and monitoring the reaction process by TLC until the reaction is complete; and removing the catalyst, adding ethanol or methanol containing 5% to 10% of water for recrystallizing, filtering to yield a white powder solid product, and drying, wherein a yield ranges from 83% to 91%; and
a preparation method of the structural general formula 7:
   (1) under the protection of nitrogen and stirring, adding alkylamine, or aryl substituted alkylamine, or hydroxylamine, or alkoxyamine, or aryl substituted alkoxyamine into a reaction flask, then adding 1 part to 3 parts of methanol, or ethanol, or ethyl acetate, or dichloroethane, or acetone, or acetonitrile, or DMF, or not adding any solvent, then adding 100 ppm to 1000 ppm o-methoxyhydroquinone, or 4,4-diphenol hydroxy dibenzophenone, or m-nitrophenol, or 0.1% to 5% of NaOH, or K2CO3, or 10% to 30% of silica gel, or acidic alumina, or not adding any catalyst, cooling to 5°C to 10°C, and then dropwise adding methyl acrylate or methyl methacrylate slowly; heating to a room temperature after dropwise adding, stirring for 5 hours to 18 hours, heating to 30°C to 70°C to continue the reaction for 5 hours to 24 hours; monitoring the reaction process by TLC until the reaction is complete, removing excessive methyl acrylate, solvent and catalyst under vacuum, and using the remaining reaction intermediate without further purification for next reaction;
   (2) under the protection of nitrogen and stirring, adding pentanediamine, or hexamethylene diamine, or decanediamine, or hexanediol, or octanediol, or decanediol in batches first into the intermediate obtained in the first step at a room temperature, then adding 0.01% to 5% of sodium methoxide or sodium formate, or not adding any catalyst, or diethyl tin oxide, or aluminum alkoxide, after finishing adding, heating to 50°C to 70°C for reaction for 10 hours to 18 hours, continuously heating to 80°C to 140°C for reaction for 24 hours to 96 hours, and monitoring the reaction process by TLC until the reaction is complete; and removing the catalyst, adding ethanol, or methanol, or ethyl acetate, or petroleum ether containing 5% to 10% of water for recrystallizing, filtering to yield a white powder solid product, and drying, wherein a yield ranges from 80% to 90%.

Compared with the hindered amine (HALS) light stabilizer product widely used in the market, the steric hindrance adjustable weak base light stabilizer product designed and invented by the patent has a more superior steric hindrance adjustable function, and the weak base property widens the application universality. Although both the products can provide different degrees of weather resistance protection for polymer materials, in terms of chemical functional group structure, the hindered amine (HALS) is a kind of light stabilizer that has been used for about half a century, wherein a structural characteristic is that: a molecular structure of each hindered amine light stabilizer product contains a 2,2,6,6-tetramethylpiperidine amine functional group structure 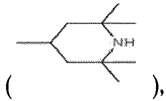 and the hindered amine functional structure may quickly transfer active free radicals generated by light-induced degradation on the polymers, thus playing an important role in weather resistance and protection of the polymer materials, and having a certain antioxidant effect at the same time.

The application of the hindered amine HALS light stabilizer that has been widely applied for about half a century in some polymer materials is limited due to slightly alkaline and nucleophilic functions defined by a specific chemical structure of tetramethylpiperidine amine, such as PVC, PC, polyester, PU, etc. Nucleophilic or alkaline tetramethylpiperidine amine nitrogen atoms may react with these polymer materials with certain acidity or electrophilicity, thus playing a role in degrading these polymer materials. The following reaction formula shows the mechanism how the hindered amine HALS participates in degradation of the PVC polymer material.

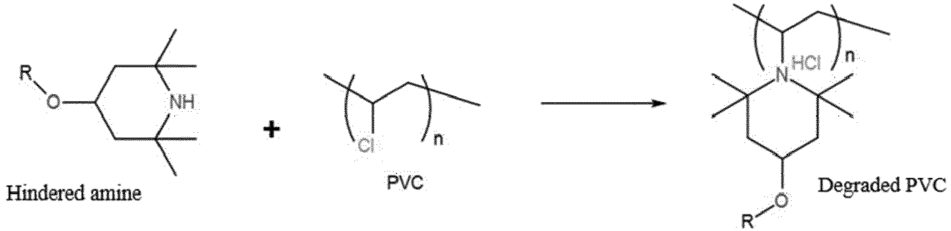

In order to weaken the nucleophilicity or the alkalinity of the hindered amine light stabilizer, there are usually two methods for modifying the nitrogen atoms of the hindered amine: (1) methyl or alkyl is introduced to the nitrogen atoms of the tetramethylpiperidine amine to increase the steric hindrance around the piperidine amine, thus weakening the alkalinity or the nucleophilicity; and (2) alkoxy is introduced to the nitrogen atoms of the tetramethylpiperidine amine, which can not only reduce the electronegativity of the nitrogen atoms of the piperidine amine, but also increase the steric hindrance around the nitrogen atoms, thus reducing the alkalinity and the nucleophilicity.

No matter whether alkyl is introduced to increase the steric hindrance and reduce an alkaline or nucleophilic attack ability, or alkoxy is introduced to reduce the electronegativity of the nitrogen atoms, it is necessary to undergo an additional chemical reaction of 1 to 3 steps to increase the steric hindrance and reduce the alkaline or nucleophilic attack ability at the same time, especially involving an oxidation, reduction or alkylation reaction. These reactions not only add extra burden to environmental protection, but also increase an additional product cost, so that a market price of the weak base hindered amine product is exceptionally high compared with that of the ordinary hindered amine product.

In the innovative photostable structure of the present invention, a 2,2,6,6-tetramethylpiperidine amine functional structure fragment is avoided from being continuously used, and the steric hindrance of substituent around the nitrogen atoms and the polarity control of the functional group are designed from a completely different new vision. By adjusting a size of the substituent around the nitrogen atoms, the steric hindrance around the nitrogen atoms is adjustable and controllable, so that a hindered amine compound with a lower alkalinity required may be provided by controlling the steric hindrance characteristic around the nitrogen atoms, thus being suitable for an application range of photodegradation protection of various polymer materials with a certain eletrophilicity. In addition, raw materials of the patent are easily available, and a designed green synthesis process greatly simplifies synthesis steps, reduces the three wastes generated by synthesis, and reduces a synthesis cost. In addition, an innovative design structure of the patent provides a new opportunity for selection of the light stabilizer of the polymer materials.

According to the innovative light stabilizer of the present invention, the steric hindrance thereof is adjusted by establishing the substituent generating the steric hindrance around the nitrogen atoms in the above seven structural general formulas, in addition, the electronegativity of the nitrogen atom can be influenced by adjusting a distance of a polar group, so that the alkalinity or the nucleophilicity of the nitrogen atom is adjusted. A desired effect is obtained by adjusting a steric hindrance and a nucleophilic property or an alkalinity in a circumstance where the nitrogen atom is located, so that an application range of the innovative light stabilizer is widened, and the innovative light stabilizer is suitable for PC, polyester, PVC and other slightly acidic or certain electrophilic polymer materials to serve as a light stability protecting aid.

In addition, raw materials of products of structures of the seven general formulas reported in the patent are easily available, and the green synthesis process is adopted, so that less three wastes are generated, and a best condition is provided for popularization and application.

The present invention aims to design and synthesize the steric hindrance adjustable weak base light stabilizer, which overcomes a defect that the hindered light stabilizer (HALS) is difficult to be applied to the slightly acidic or electrophilic polymers as the light stability protection aid up to now; meanwhile, due to the side chain property of the structural substituent and the adjustable polarity of other polar functional groups in molecules, a matching property with the polymers is improved; in addition, the innovative light stabilizer designed in the patent breaks a monopoly structure of a 2,2,6,6-tetramethylpiperidine amine fixed structure as the active functional group of the light stabilizer for nearly half a century, and the characteristic structure cannot be avoided in chemical synthesis, especially the additional chemical synthesis steps for converting the conventional hindered amine structure into the weak base hindered amine light stabilizer are inevitable, which makes a cost and an environment show an improvable state.

According to the present invention, the seven series of steric hindrance adjustable weak base light stabilizers have easily available raw materials, and all of the light stabilizers may synthesize expected products through the green environmental protection process, which greatly facilitates production and wide application of the light stabilizer, so that the light stabilizer has an opportunity to become the valuable light stability protecting aid for all polymer materials (including PVC, PC, PU, polyester, etc.).

The patent for invention reports an innovative structural light stabilizer with adjustable steric hindrance, controllable weak base property of nitrogen atoms and no 2,2,6,6-tetramethylpiperidine amine structural fragment for the first time. The patent for invention discloses seven series of innovative steric hindrance adjustable novel structural light stabilizers.

According to the patent for invention, the innovative chemical structure product may be directly applied to the polymer materials to provide effective light stability protection and antioxidant stability protection, may play a role in guaranteeing a quality and a color and maintaining a function of polymer material products for a long time in use, and may be applied to series products such as plastic, rubber, fiber, film, coating, paint, ink and petroleum, thus having a vast market.

The objectives of the present invention are as follows: (1) the steric hindrance adjustable weak base or nearly neutral light stabilizer is designed and synthesized, which may be applied to all polymer materials (including PVC, PC, PU, polyester, etc.) to provide wider and more valuable light stability protection. (2) More light stability functional group structures are provided to break a long-standing situation that tetramethylpiperidine is used as the only light stability functional group in the international market. (3) A greener and more environment-friendly synthesis method of the light stabilizer is provided for the market.

The present invention solves the following problems: (1) a state that 2,2,6,6-tetramethylpiperidine amine as the fixed steric hindrance light stabilizer is taken as the only choice throughout history is solved and changed. (2) For the newly designed light stabilizer structure, the steric hindrance and the alkalinity and the nucleophilicity of the light stabilizer are adjustable according to the needs, which widens the application range of the light stabilizer in the polymer materials. (3) The designed light stabilizer with a weak base and weak nucleophilicity structure provides a new opportunity for the polymer materials such as PVC, PC, polyester, PU, etc. the which cannot use hindered amine light stabilizer at present, and provides a better choice of an UV protectant for these polymer materials. (4) Mismatch between a tetramethylpiperidine amine base light stabilizer and an acid aid is solved. (5) A complicated weak base light stabilizer synthesis method in the market is broken through, and a new light stabilizer is synthesized by an optimized, simple and green synthesis method. (6) The steric hindrance adjustable weak base light stabilizer of the patent has better compatibility and matching with the polymers, thus improving an anti-yellowing property of thermal aging and prolonging a service life.

### BRIEF DESCRIPTION OF THE DRAWINGS

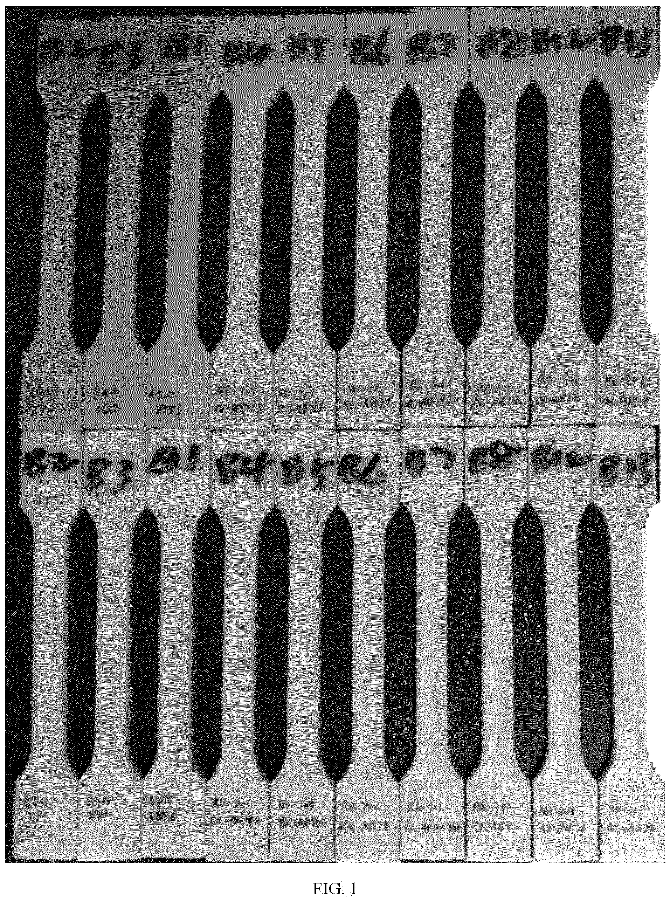

FIG. 1 shows comparison of results of thermal and light aging of 1PP-T20 splines (note: (1) splines in upper row are results of thermal aging in oven at 150°C for 192 hours, and splines in lower row are results of UVB ultraviolet light aging at 70°C for 157 hours; (2) testing and comparison of standard samples: B1 is 3853, B2 is 770, and B3 is 622; and (3) B4 to B13 are innovative antioxidants and steric hindrance adjustable light stabilizers of the patent).

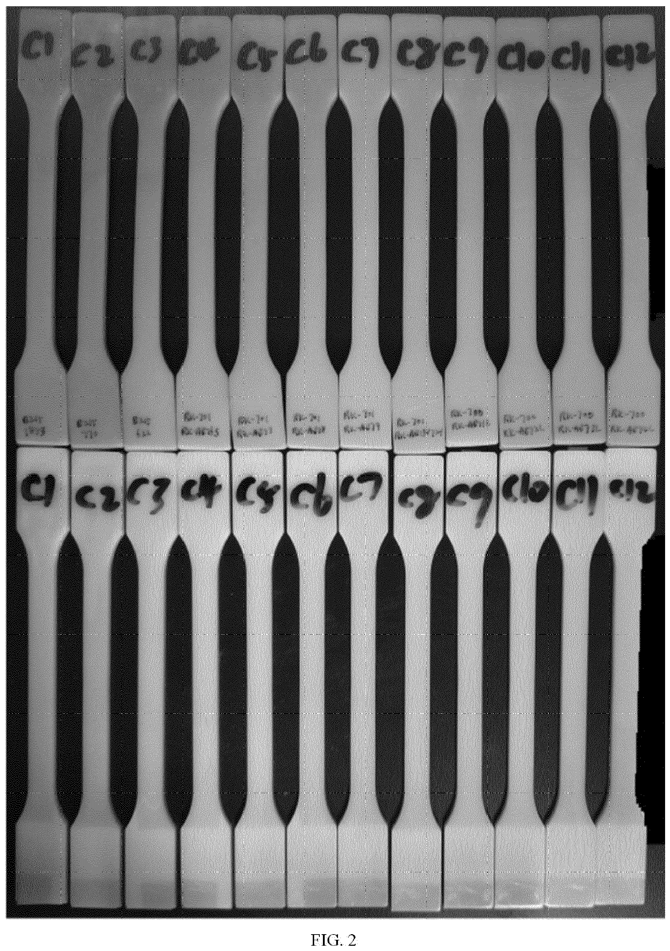

FIG. 2 shows comparison of results of thermal and light aging of ABS splines (note: (1) splines in upper row are results of thermal aging in oven at 110°C for 178 hours, and splines in lower row are results of UVB light aging at 70°C for 113 hours; (2) testing and comparison of standard samples: C1 is 3853, C2 is 770, and C3 is 622; and (3) C4 to C12 are innovative antioxidants and steric hindrance adjustable light stabilizers of Rycom).

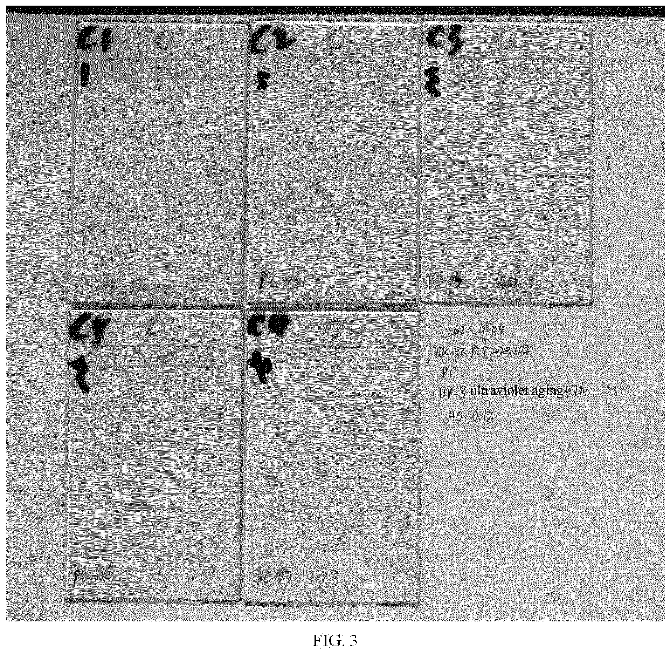

FIG. 3 shows results 1 of UVB ultraviolet light aging at 70°C for 47 hours of PC sample plates (antioxidant AO + light stabilizer (2: 1): 0.1%; and UV aging test device Q-Lab).

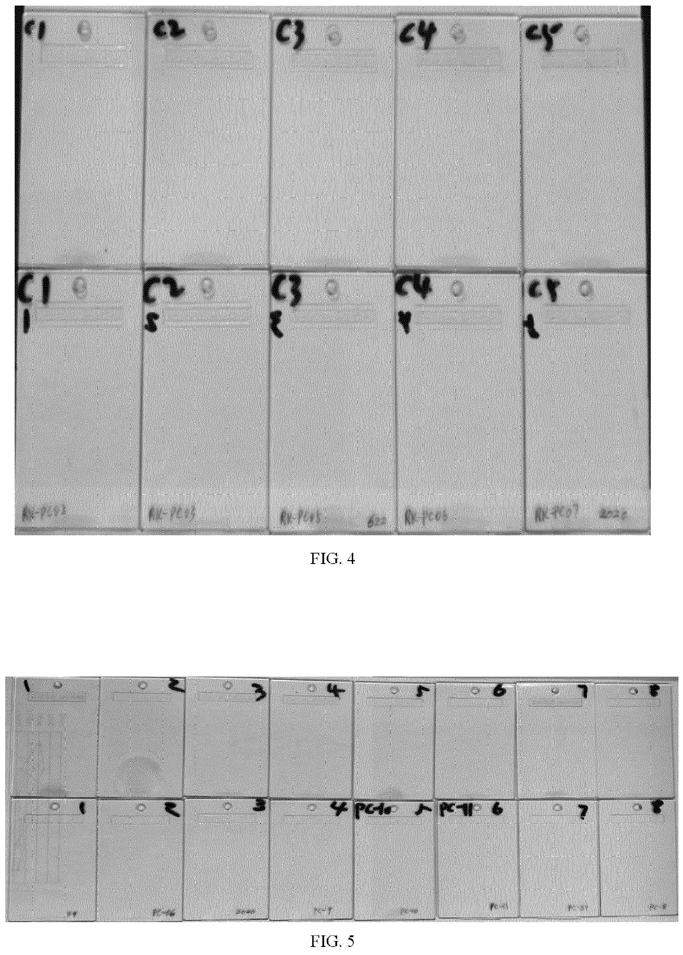

FIG. 4 shows results 2 of UVB ultraviolet light aging at 70°C for 17 hours of PC sample plates ((1) sample plates in upper row are results of thermal aging in oven at 150°C for 17 hours, and sample plates in lower row are results of UVB light aging at 70°C for 48 hours; (2) test and comparison of standard samples: C3 is 622, and C5 is 2020; (3) C1, C2 and C4 are innovative antioxidants and steric hindrance adjustable light stabilizers of Rycom; and (4) UVB light aging test device is Q-Lab UV light aging tester Suzhou Guangjun ZN-PB).
FIG. 5 shows results 3 of UVB ultraviolet light aging at 70°C of PC sample plates (note: (1) PC sample plates in lower row are PC sample plates before aging; (2) PC sample plates in upper row are sample plates subject to UVB ultraviolet light aging at 70°C for 24 hours; and (3) comparison of standard samples: UV2020(#3) and UV119(#1)).

### DETAILED DESCRIPTION

**Table 1: Example structure of steric hindrance adjustable innovative weak base light stabilizer**

| Mass spectrometry device: Thermo Finnigan LCQ Advantage ThermoFisher NMR device: Avance III 400 MHz Bruker in Switzerland | | | | |
|---|---|---|---|---|
| Seria I No. | Chemical structural formula | MS(ESI) | | ¹H-NMR /m.p.(°C) |
| | | Theore tical value | Detection value | 400MHZ |
| 1 | 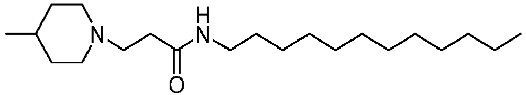 | 338 | MS⁺: 339.23 | N/A |
| | | | MS⁻: 337.25 | Mp: 52°C to 57°C |
| 2 | 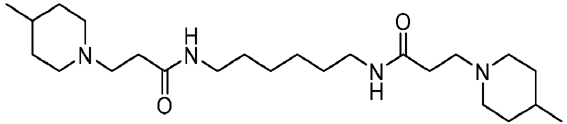 | 422 | MS⁺: 445.21; | N/A |
| | | | 867.16 | Mp: 91°C to 96°C |
| 3 | 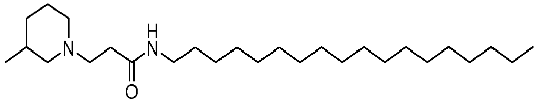 | 422 | MS+: 425.3 849.09 | Solvent: CDCl₃ 7.87 (s, 1H), 3.22 (q, J = 6.5 Hz, 1H), 2.86 (t, J = 5.3 Hz, 1H), 2.60 (t, J = 6.9 Hz, 1H), 2.40 - 2.29 (m, 1H), 1.47 (d, J = 6.4 Hz, 3H), 1.27 (d, J = 10.2 Hz, 18H), 0.88 (t, J = 6.6 Hz, 3H). |
| | | | | Mp: 67°C to 92°C |
| 4 | 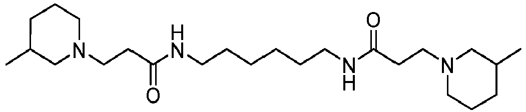 | 422 | MS+: 423.15 445.46 | 8.70 (s, 1H), 3.20 (s, 1H), 2.83 (s, 1H), 2.54 (s, 1H), 2.37 (d, J = 4.4 Hz, 1H), 1.91 (s, 1H), 1.54 (dd, J = 99.5, 42.2 Hz, 5H), 0.90 (s, 2H). |
| | | | | Mp: 79°C to 83°C |
| 5 | 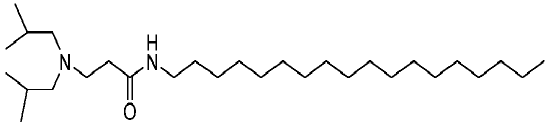 | 452 | MS⁺; 453.37 | N/A |
| | | | | Mp: 65°C to 69°C |
| 6 | 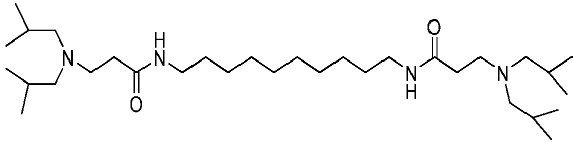 | 538 | MS⁺: 539.43 | N/A |
| | | | | Mp: 103°C to 109°C |
| 7 | 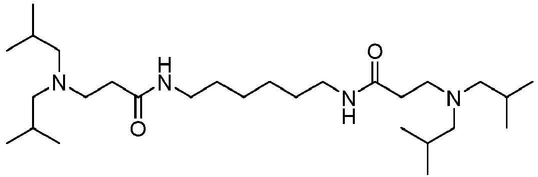 | 482 | MS+: 483.32,987.21 | N/A |
| | | | MS-: 481.38 | Mp:105°C to 110°C |
| 8 | 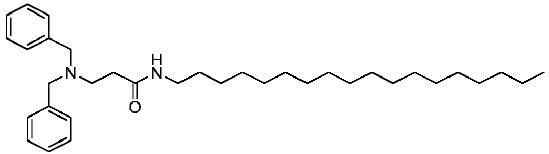 | 520 | MS+: 521.30 | 7.48 (d, J = 114.0 Hz, 1H), 7.31 (d, J = 9.0 Hz, 2H), 3.56 (s, 1H), |
| | | | MS-: 519.42 | 3.09 (dd, J = 13.4, 6.6 Hz, 1H), 2.72 (s, 1H), 2.43 (d, J = 5.6 Hz, 1H), 1.42 (s, 1H), 1.27 (s, 3H), 0.88 (t, J = 6.8 Hz, 1H). |
| | | | | Mp: 154°C to 161°C |
| 9 | 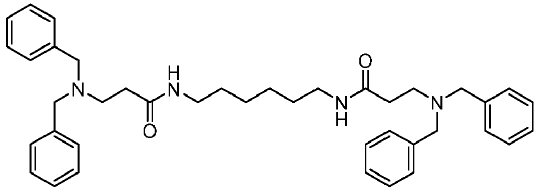 | 618 | MS+: 619.35, 641.44 | 7.63 (t, J = 5.4 Hz, 1H), 7.32 (ddd, J = 11.7, 7.3, 1.4 Hz, 2H), 3.56 (s, 2H), 3.10 (dd, J = 13.7, 6.5 Hz, 1H), 2.76 - 2.67 (m, 1H), 2.42 (t, J = 5.9 Hz, 1H), 1.53 - 1.24 (m, 2H). |
| | | | MS-: 617.24 | |
| | | | | Mp: 119°C to 122°C |
| 10 | 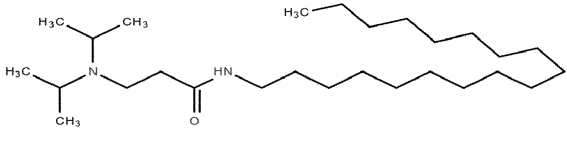 | 424 | MS+: 425.24 | N/A |
| | | | | Mp: 47°C to 70°C |
| 11 | 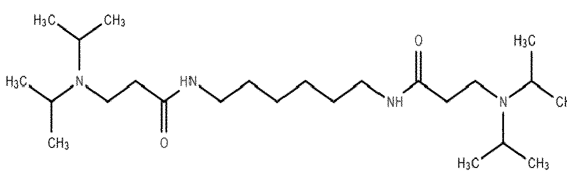 | 426 | MS+: 427.38 | N/A |
| | | | MS-: 425.37 | Mp: 39°C to 45°C |
| 12 | 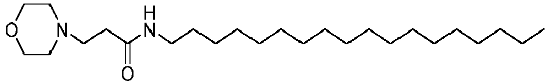 | 410 | MS+: 411.23 | 4.3 Hz, 6H), 2.40 (t, J = 7.1 Hz, 4H), 1.33 (d, J = 10.8 Hz, 47H), 0.93 (t, J = 6.9 Hz, 7H). |
| | | | Ms-: 409.15 | Mp: 75°C to 79°C |
| 13 | 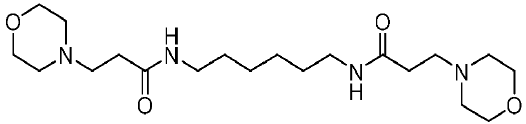 | 398 | MS+: 399.08 421.11 | (CD3OD) 8.04 (s, 2H), 4.22-4.02 (m, 4H), 3.81- 3.60 (m, 8H), 3.24 (d, J = 5.1 Hz, 4H), 2.75-2.32 (m, 4H), 2.01 (d, J = 25.8 Hz, 8H), 1.44-1.08 (m, 8H). |
| | | | MS-: 397.23 | |
| | | | | Mp: 123°C to 151°C |
| 14 | 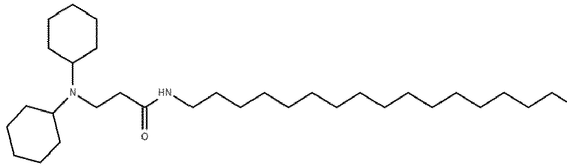 | 504 | MS+: 505.30 | N/A |
| | | | | Mp:58°C to 61°C |
| 15 | 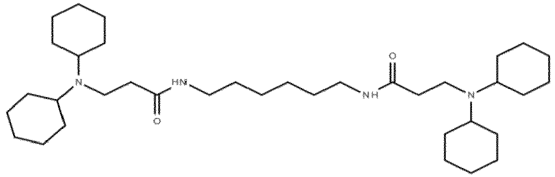 | 586 | MS+587.4 4 | N/A |
| | | | MS-: 585.47 | Mp: 97°C to 101°C |
| 16 | 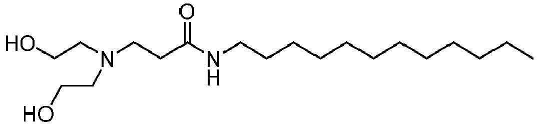 | 344 | MS+: 345.16 | (CD3OD) 7.86 (s, 1H), 3.22 (dd, J = 13.0, 6.6 Hz, 1H), 2.91 - 2.81 (m, 1H), 2.66 - 2.57 (m, 1H), 2.40 - 2.30 (m, 1H), 1.47 (d, J = 6.7 Hz, 2H), 1.27 (d, J = 10.4 Hz, 18H), 0.88 (t, J = 6.7 Hz, 3H). |
| | | | MS-: 344.23 | |
| | | | | Mp: 112°C to 116°C |
| 17 | 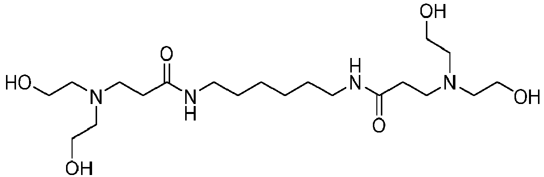 | 434 | MS+: 457.4 | (CD3OD) 3.64 (s, 2H), 3.49 (d, J = 1.5 Hz, 8H), 3.23 (s, 8H), 2.81 (s, 4H), 2.64 (s, 2H), 2.36 (s, 4H), 1.55 (d, J = 32.2 Hz, 8H). |
| | | | MS-: 433.21 | |
| | | | | Mp: 147°C to 151°C |
| 18 | 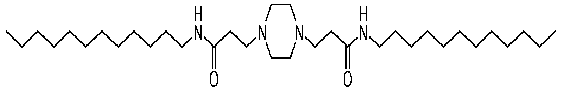 | 564 | MS⁺: 565.42 | N/A |
| | | | MS⁻: 563.48 | Mp: 138°C to 146°C |
| 19 | 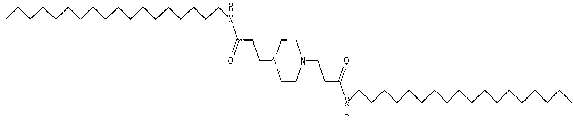 | 732 | MS+: 733.11 | N/A |
| | | | MS-: 731.42 | Mp: 137°C to 142°C |
| 19 | 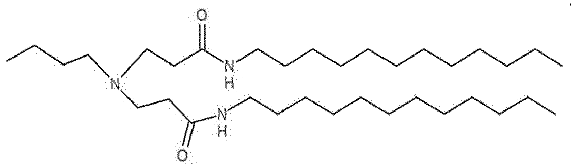 | 551 | MS⁺: 552.40 | (CDC13)7.85 (s, 2H), 3.22 (dd, J = 12.7, 7.0 Hz, 4H), 2.90-2.83 (m, 4H), 2.61 (t, J = 7.1 Hz, 4H), 2.39-2.31 (m, 4H), 1.48 (dd, J = 13.6, 6.8 Hz, 2H), 1.36-1.17 (m, 40H), 0.88 (t, J = 6.8 Hz, 9H). |
| | | | MS⁻: 550.28; 551.31 | |
| | | | | Mp: 102°C to 105°C |
| 20 | 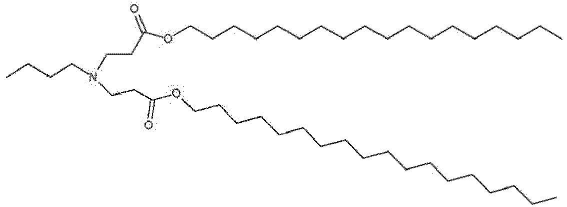 | 711 | MS+: 712.33 | (CDCl3)7.85 (s, 2H), 4.13 (dd, J = 12.7, 7.0 Hz, 4H), 3.11-2.83 (m, 6H), 2.71 (t, J = 7.1 Hz, 4H), 2.39-2.31 (m, 4H), 1.48 (dd, J = 13.6, 6.8 Hz, 2H), 1.36-1.17 (m, 68H), 0.88 (t, J = 6.8 Hz, 12H). |
| | | | | Mp: 86°C to 89°C |
| 21 | 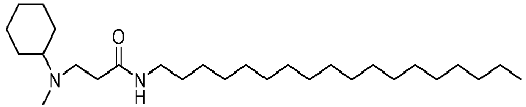 | 436 | MS+: 437.13 | 7.78 (s, 1H), 3.22 (dd, J = 12.7, 7.0 Hz, 1H), 2.94 - 2.85 (m, 1H), 2.64 (t, J = 7.0 Hz, 1H), 2.41 (d, J = 5.0 Hz, 1H), 1.57 - 1.42 (m, 2H), 1.38 - 1.18 (m, 18H), 0.88 (t, J = 6.8 Hz, 3H). |
| | | | MS-: 435.41 | |
| | | | | Mp: 77°C to 81°C |
| 22 | 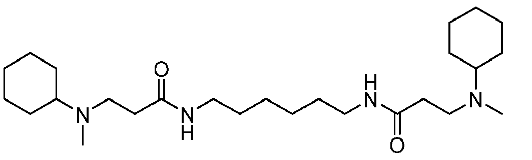 | 450 | MS+: 451.36, | N/A |
| | | | 923.24 | Mp: 103°C to 107°C |
| 23 | 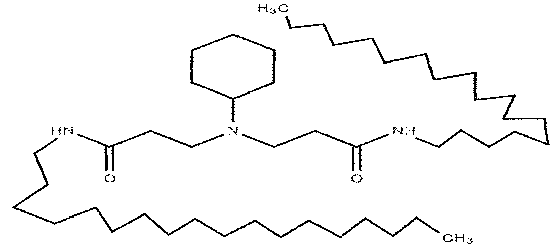 | 745 | MS+: 746.25 | N/A |
| | | | MS-: 744.43 | Mp: 102°C to 106°C |
| 24 | 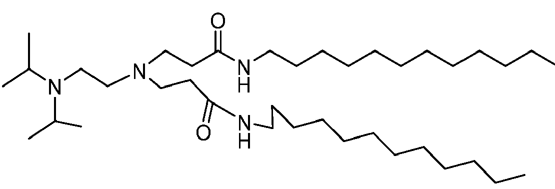 | 622 | MS⁺: 623.42 | N/A |
| | | | MS⁻: 621.38 | Mp: 108°C to 110°C |
| 25 | 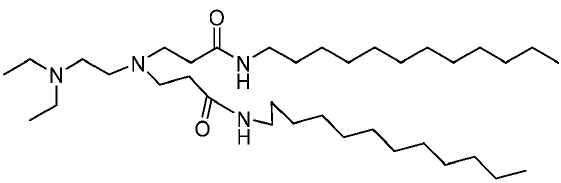 | 594 | MS⁻: 593.24 | N/A |
| | | | | Mp: 117°C to 120°C |
| 26 | 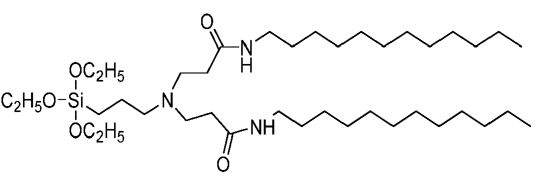 | 700 | MS⁺: 701.63 | N/A |
| | | | | Mp: 59°C to 63°C |
| 27 | 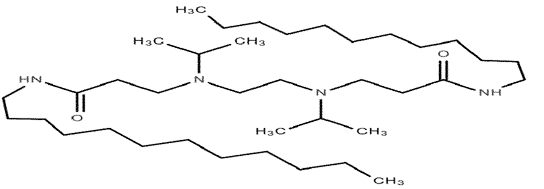 | 790 | MS⁺: 791.33 | N/A |
| | | | MS-: 789.48 | Mp: 91°C to 96°C |
| 28 | 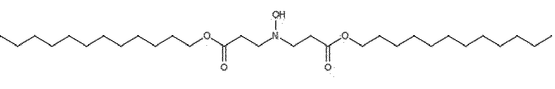 | 513 | MS+: 514.32 | N/A |
| | | | | Mp: 125°C to 131°C |
| 29 | 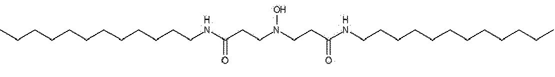 | 511 | MS+: 512.15 | N/A |
| | | | MS-: 510.32 | Mp: 131°C to 142°C |
| | | | | |
| 30 | 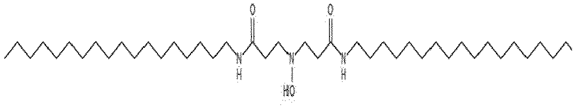 | 679 | MS+: 680.13 | N/A |
| | | | MS-: 678.36 | Mp: 131°C to 145°C |
| 31 | 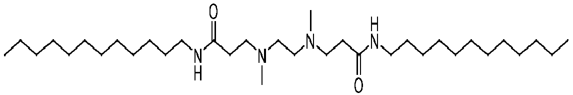 | 566 | MS+: 567.47 | N/A |
| | | | | Mp: 68°C to 93°C |
| 32 | 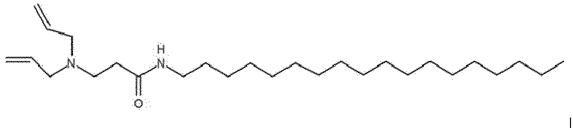 | 420 | MS+: 421.24 | N/A |
| | | | | Mp: 67°C to 71°C |
| 33 | 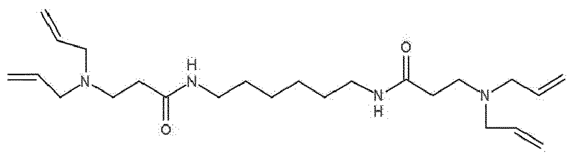 | 418 | MS+: 419.21 | N/A |
| | | | MS-: 417.11 | Mp: 97°C to 101°C |
| 34 | 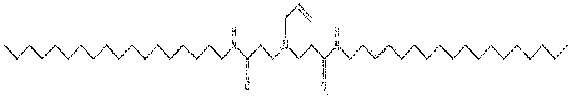 | 703 | MS+ | N/A |
| | | | MS-: | Mp: 83°C to 86°C |
| 35 | 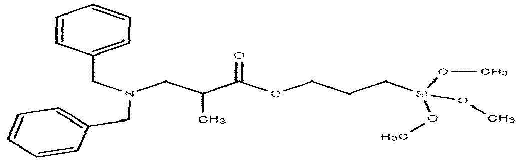 | 445 | MS+: 446.52 | N/A |
| | | | | Liquid |
| 36 | 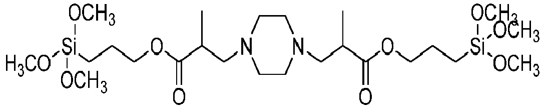 | 582 | MS+: 583.30; 605.20 | N/A |
| | | | | Liquid |
| 37 | 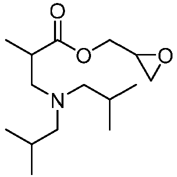 | 271 | MS+: 272.21 | 7.27(S, CDCl3), 4.05-4.25(dd, 2H), 3.62-3.85(dd, 3H), 2.31-2.48(m, 6H), 2.23(d, 3H), 1.69-1.82(m, 2H), 0.81-0.93(dd, 12H) |
| | | | | Liquid |
| 38 | 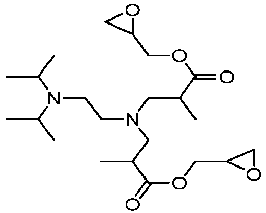 | 428 | MS+: 429.17 | N/A |
| | | | | Liquid |
| 39 | 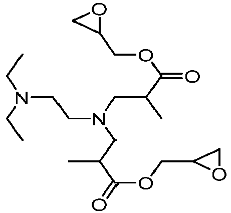 | 400 | MS+: 801.35 | N/A |
| | | | MS-: 422.84 | Liquid |
| 40 | 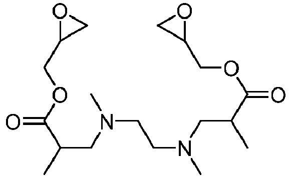 | 372 | MS+: 373.11 | N/A |
| | | | | Liquid |
| 41 | 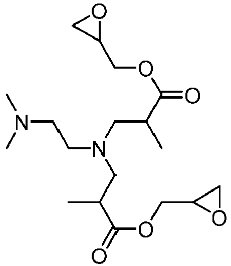 | 372 | MS+: 373.06 | N/A |
| | | | | Liquid |
| 42 | 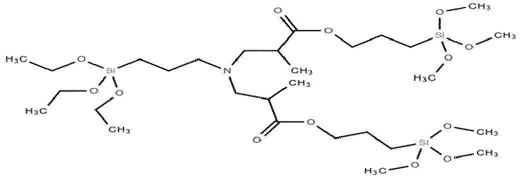 | 803 | MS+: 827.16 | N/A |
| | | | | Liquid |
| 43 | 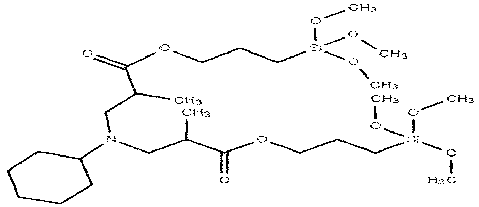 | 595 | MS-: 594.86 | |
| | | | | Liquid |
| 44 | 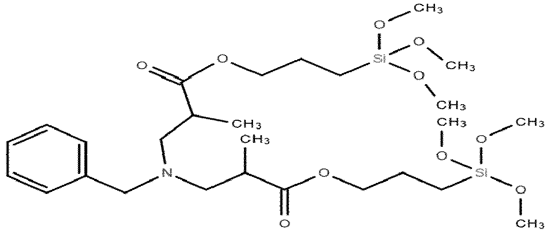 | 603 | MS-: 625.08 622.69 | N/A |
| | | | | Liquid |
| 45 | 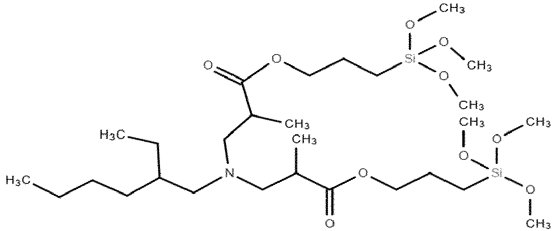 | 625 | MS+: 626.32 | N/A |
| | | | | Liquid |
| 46 | 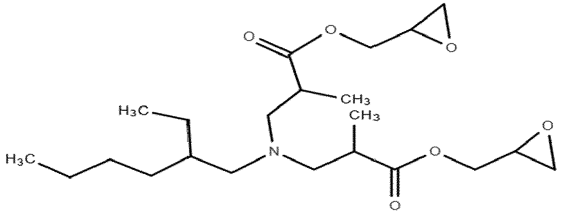 | 413 | MS+: 425.24 | N/A |
| | | | | Liquid |
| 47 | 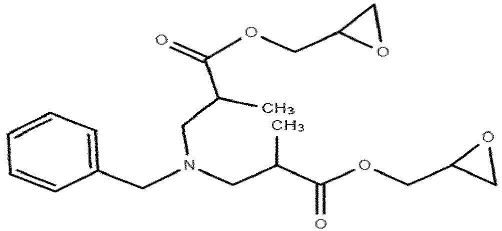 | 391 | MS+ | 7.27-7.38(m, 5H), 7.26(S, CDCl₃) 6.11 (d,2H), 5.59(d,2H), 4.16-4.23(m, 2H), 3.81-4.16(m, 4H), 3.62-3.81 (m, 4H), 2.72-2.89(m, 4H), 1.86-2.03(dd, 6H) |
| | | | | Liquid |
| 48 | 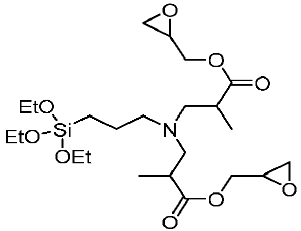 | 505 | MS+: 506.13; 528.29 | 7.27(S, CHCl3), 6.15-6.21 (d,2H), 5.03-5.16(d, 2H), 4.48(m, 1H), 3.28-4.23(m, 10H), 2.48-3.60(m, 8H), 1.61-1.78(m, 9H), 1.16-1.23(m, 10H) |
| | | | | Liquid |
| 49 | 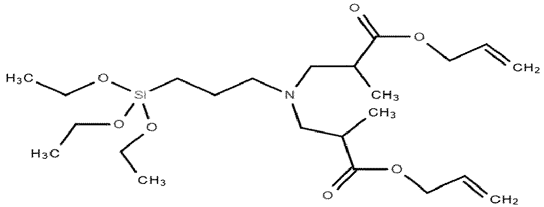 | 473 | MS+: 474.45 | N/A |
| | | | | Liquid |
| 50 | 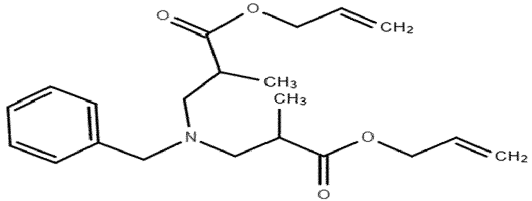 | 359 | MS+: 360.47 | N/A |
| | | | | Liquid |
| 51 | 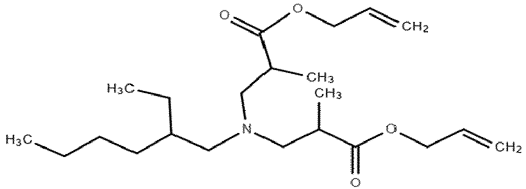 | 381 | MS+: 382.32 | N/A |
| | | | | Mp: 75°C to 79°C |
| 52 | 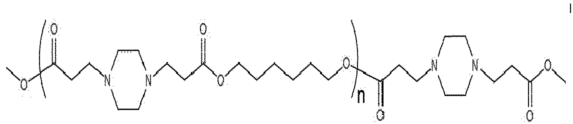 | N/A | N/A | 8.01 (BS, 1H), 7.27(s, CDCl3),4.13(t, 4H) 3.22(d,6H), 2.26-2.8(m, 10H), 1.42-1.71(m, 4H), 1,26-1.39(m,4H). |
| | | | | Mp: 147°C to 279°C |
| 53 | 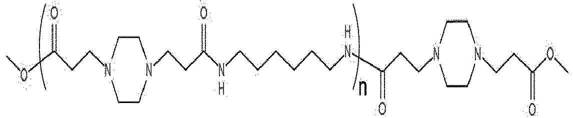 | N/A | N/A | 7.26(s,CDCl3), 3.67-3.81(m, 8H), 2.69(t, 4H), 2.36-2.58(m, 10H), 1.59-1.63(m, 4H), 41,36-1.46(m, 4H) |
| | | | | Mp: 177°C to 229°C |
| 54 | 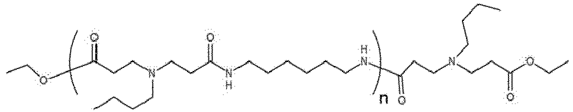 | N/A | N/A | 7.91(BS, 2H), 7.27(s, CDCl3), 3.13-3.31(d,10H), 2.39-2.23(m, 8H), 1.42-1.71(m, 8H), 1,26-1.39(m,5H). |
| | | | | Mp: 173°C to 196°C |
| 55 | 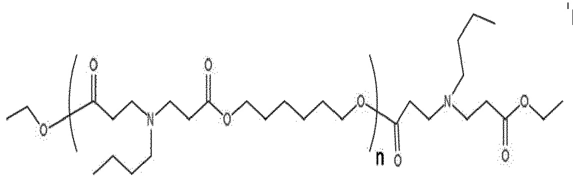 | N/A | N/A | 7.26(s,CDCl3), 4.17(t, 4H), 2.69(t, 4H), 2.87-3.13(m, 6H)2.26-2.39(m, 8H), 1.39-1.53(m, 6H), 0.98-1.26(m, 5H) |
| | | | | Mp: 169°C to 212°C |
| 56 | 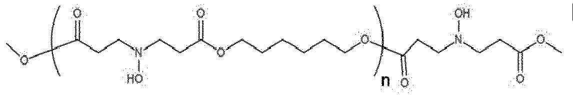 | N/A | N/A | 7.26(s,CDCl3), 4.34(SB,1H), 4.17(t, 4H), 3.39(t, 4H), 2.36-2.58(m, 4H), 1.59-1.63(m, 4H), 1,09-1.37(m, 4H) |
| | | | | Mp: 33°C to 46°C |
| 57 | 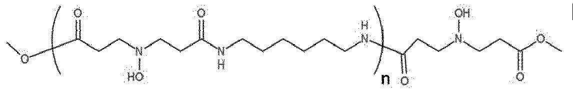 | N/A | N/A | 8.08(SB, 2H), 7.26(s, CDCl3), 4.53(SB,2H)3.42(d,4H) , 2.26-2.8(m, 12H), 1.42-1.71(m, 4H), 1,06-1.29(m,4H). |
| | | | | Mp: 169°C to 297°C |
| 58 | 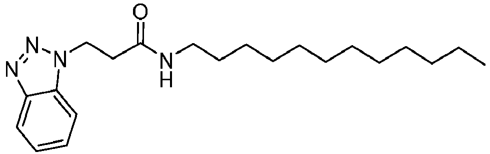 | 358 | MS+: 359.31 | (CDCl3) 8.03 (d, J = 8.3 Hz, 1H), 7.69 (d, J = 8.4 Hz, 1H), 7.51 (t, J = 7.6 Hz, 1H), 7.39 (t, J = 7.6 Hz, 1H), 4.97 (t, J = 6.4 Hz, 2H), 3.10 (dd, J = 12.2, 7.0 Hz, 2H), 2.96 (t, J = 6.4 Hz, 2H), 1.34 - 0.95 (m, 19H), 0.88 (t, J = 6.9 Hz, 3H). |
| | | | | Mp:106°C to 110°C |
| 59 | 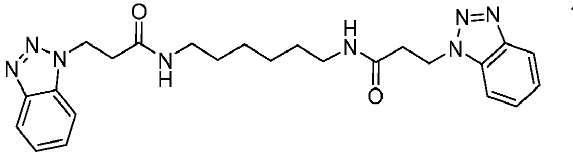 | 462 | MS+: 463.21 485.27 | (CDCl3) 8.04 - 7.96 (m, 1H), 7.86 - 7.80 (m, 1H), 7.69 (s, 1H), 7.50 (s, 1H), 7.38 (dd, J = 6.6, 3.1 Hz, 2H), 5.08 (t, J = 6.7 Hz, 1H), 4.99 (s, 2H), 3.06 (ddd, J = |
| 60 | 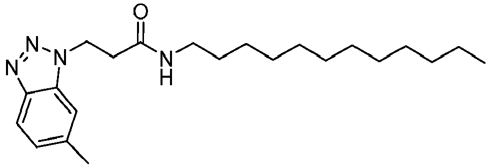 | 372 | MS+: 373.24 767.10 | CDCl3) 7.77 (s, 1H), 7.54 (d, J = 8.6 Hz, 1H), 7.34 - 7.30 (m, 1H), 4.96 - 4.90 (m, 1H), 3.10 (dd, J = 13.0, 7.0 Hz, 1H), 2.92 (dd, J = 8.2, 4.7 Hz, 1H), 2.51 (s, 1H), 1.37-1.03 (m, 11H), 0.89 (t, J = 6.9 Hz, 3H). |
| | | | | Mp: 84°C to 114°C |
| 61 | 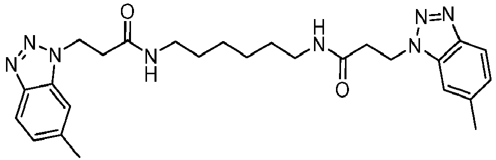 | 490 | MS+: 491.31; | N/A |
| | | | 980.11; 1003.20 | Mp: 145°C to 181°C |
| 62 | 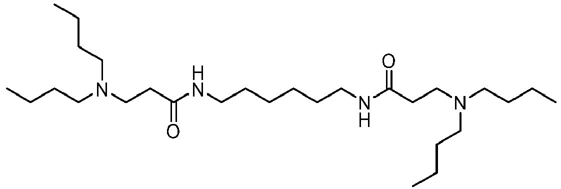 | 482 | MS+: 483.23 | (MeOD) δ 8.58 (s, 1H), 3.63 (q, J = 7.0 Hz, 1H), 3.34 (dt, J = |
| | | | MS-: 481.11 | 3.3, 1.6 Hz, 1H), 3.19 (t,J = 7.0 Hz, 1H), 2.93 - 2.70 (m, 2H), 2.47 (tdd, J = 16.2, 10.9, 5.7 Hz, 3H), 2.35 (t, J = 7.1 Hz, 1H), 1.70-1.25 (m, 8H), 1.04-0.89 (m, 4H). |
| | | | | Mp: 81°C to 87°C |
| 63 | 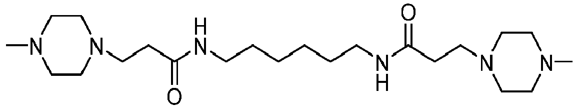 | 424 | MS+: 425.30, 870.99 | N/A |
| | | | MS-: 423.26 | Mp: 133°C to 138°C |
| 64 | 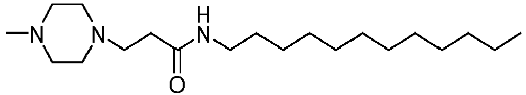 | 339 | MS+: 340.22, | N/A |
| | | | 701.09 | Mp: 74°C to 78°C |
| 65 | 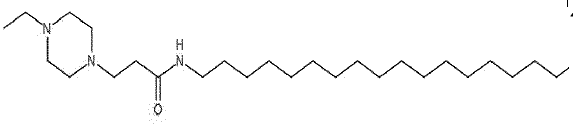 | 437 | MS+: 438.41 | N/A |
| | | | MS-: 436.11 | Mp: 106°C to 109°C |
| 66 | 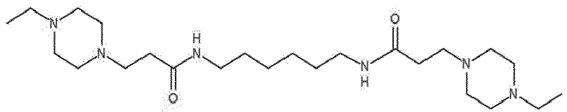 | 452 | MS+: 453.22 | N/A |
| | | | MS-: 451,46 | Mp: 149°C to 153°C |
| 67 | 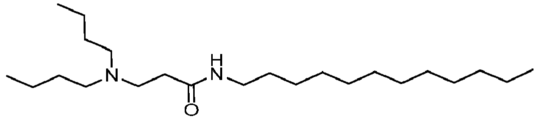 | 368 | MS+: | N/A |
| | | | 369.21 | Mp: 46°C to 49°C |
| 68 | 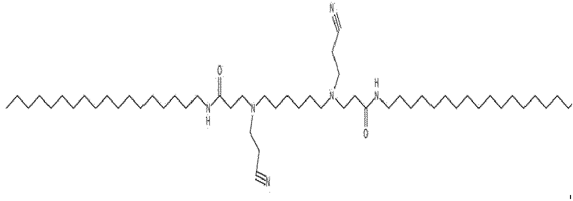 | 868 | MS+: 869.32 | N/A |
| | | | MS-: 867.47 | Mp: 155°C to 159°C |
| 69 | 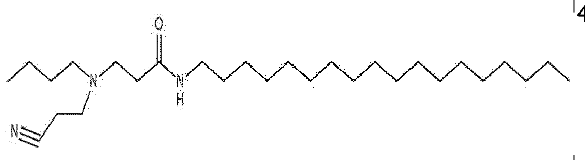 | 449 | MS+: 450.41 | N/A |
| | | | MS-: 448.04 | Mp: 92°C to 97°C |
| 70 | 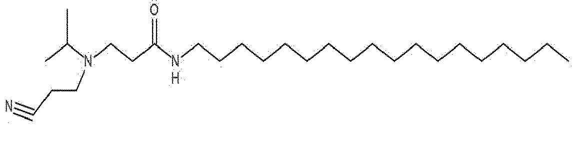 | 435 | MS+: 435.41 483.37 | N/A |
| | | | MS-: 434.17 | Mp: 83°C to 86°C |
| 71 | 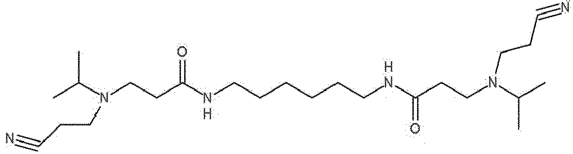 | 448 | MS+: 449.13 | N/A |
| | | | MS-: 447.24 | Mp: 137°C to 141°C |
| 72 | 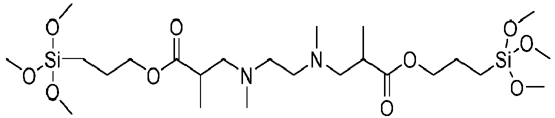 | 584 | MS+: 585.19 | N/A |
| | | | | Liquid |
| 73 | 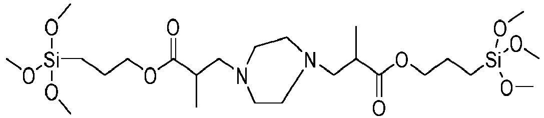 | 582 | MS+: 583.30 | N/A |
| | | | | Liquid |
| 74 | 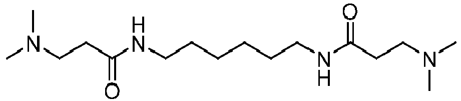 | 314 | MS+: 315.26 | N/A |
| | | | MS-: 313.17 | Liquid |
| 75 | 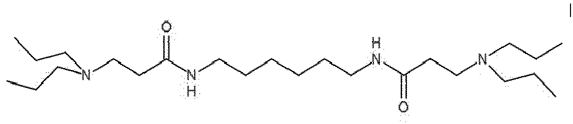 | 426 | MS+: 427.32 | N/A |
| | | | | Liquid |
| 76 | 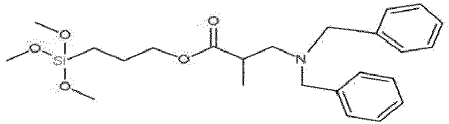 | 445 | MS+: 445.73 | N/A |
| | | | | Liquid |
| 77 | 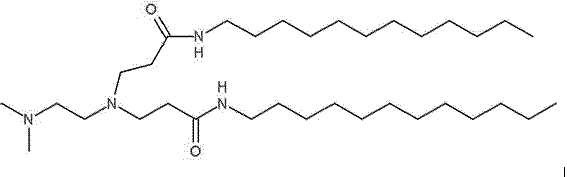 | 566 | MS+: 567.23 | N/A |
| | | | MS-: 565.21 | Mp: 68°C to 76°C |
| 78 | 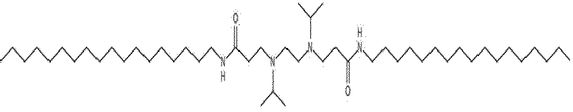 | 790 | MS+: 791.23 | N/A |
| | | | MS-: 789.46 | Mp: 151°C to 162°C |
| 79 | 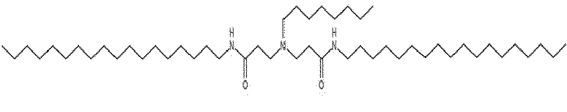 | 775 | MS+: 776.13 | N/A |
| | | | MS-: 774.36 | Mp:54°C to 61°C |
| 80 | 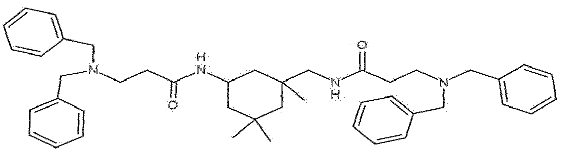 | 672 | MS+: 673.30 | N/A |
| | | | MS-: 671.07 | Mp: 146°C to 151°C |
| 81 | 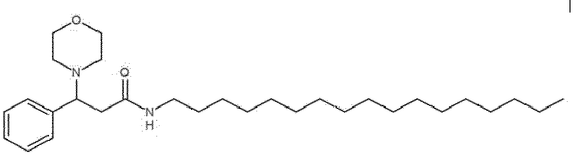 | 472 | MS+: 473.33 | N/A |
| | | | | Mp: 122°C to 126°C |
| 82 | 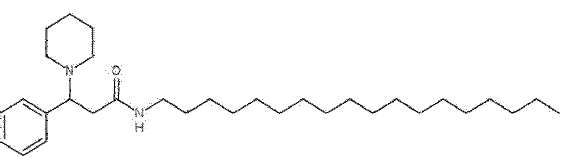 | 484 | MS+: 485.41 | N/A |
| | | | MS-: 483.23 | Mp: 93°C to 96°C |
| 83 | 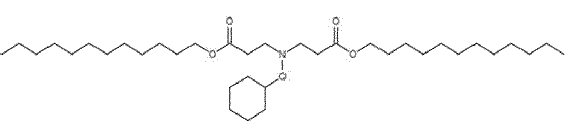 | 595 | MS+: 596.53 | N/A |
| | | | | Mp: 91°C to 95°C |
| 84 | 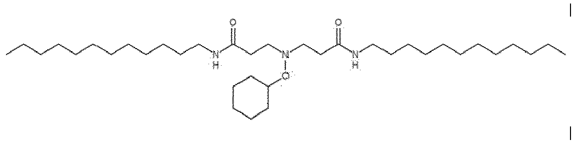 | 595 | MS+: 596.53 | N/A |
| | | | MS-: 592.11 | Mp: 129°C to 136°C |
| 85 | 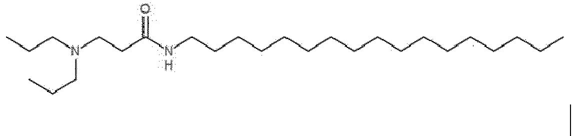 | 410 | MS+: 411.21 | N/A |
| | | | MS-: 409.05 | Mp: 43°C to 58°C |

The chemical structural formulas of the organic compounds in Table 1 include the example structures represented by the structural general formulas of seven types of innovative light stabilizers listed above in the patent, the synthesis method is a solvent-free green chemical synthesis method, and reaction activation energy thereof is reduced through the catalyst, so that synthesis of a target product is successfully completed.

### Synthesis method of novel light stabilizer:

### (I) Synthesis method of novel light stabilizer with structural general formula 1

### (1) Synthesis route of novel light stabilizer with structural general formula 1

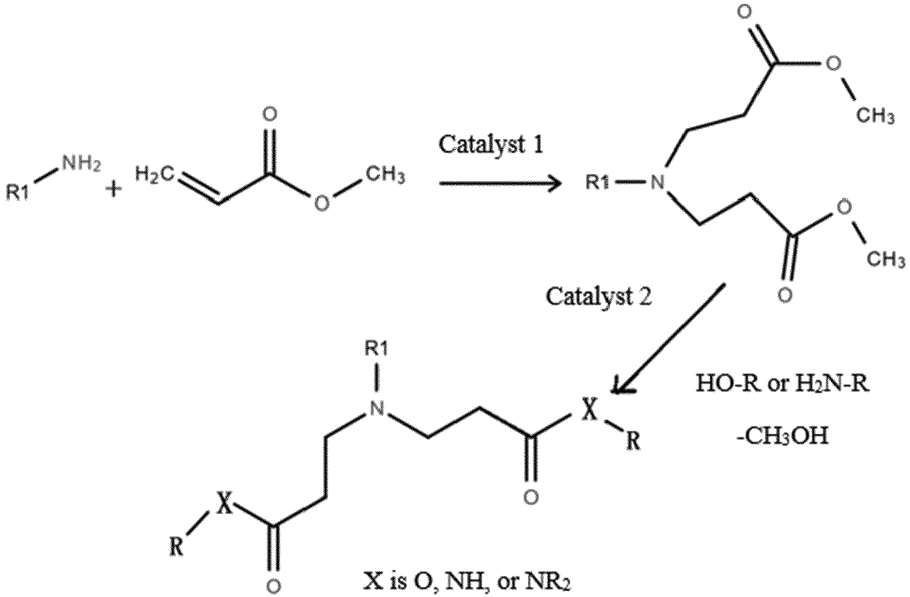

### (2) General synthesis method of structural general formula 1

I. Under the protection of nitrogen, methyl acrylate or methyl methacrylate (2.05 mmol to 3.5 mmol) was added into a reaction flask, and then stirred, 1 part to 3 parts of methanol, or ethanol, or acetone, or ethyl acetate, or dichloroethane, or DMF was added, or no solvent was added, and 0.05% to 30% of catalyst 1 was added (the catalyst 1 was acetic acid, acidic alumina, silica gel, ortho-methoxyhydroquinone, 4,4'-benzophenone, m-nitrophenol, or silica gel sulfate). The temperature was reduced to 5°C to 10°C, and then first amine (1.0 mmol to 1.5 mmol) was dropwise added slowly. The temperature was raised to a room temperature after dropwise adding, then the reagent was stirred for 3 hours to 5 hours, and heated to 40°C to 80°C to continue the reaction for 5 hours to 18 hours. The reaction process was monitored by TLC until the reaction was complete, excessive methyl acrylate was removed under vacuum, and the remaining reaction intermediate without further purification was used for next reaction.
II. Under the protection of nitrogen and stirring, 0.1% to 5% of catalyst 2 was added into the intermediate obtained in the reaction of the first step at the room temperature (the catalyst 2 could be sodium methoxide, sodium formate, diethyl tin oxide or aluminum isooctanol), and then n-dodecylamine, or n-octadecylamine, or n-dodecanol, or n-octadecanol (2.0 mmol) was added in batches. After charging, the temperature was raised to 40°C to 70°C for reaction for 5 hours to 16 hours, and continuously raised to 85°C to 120°C for reaction for 30 hours to 96 hours, and the reaction process was monitored by TLC until the reaction was complete. Ethanol, or ethyl acetate or petroleum ether containing 5% to 15% of water was added for recrystallizing, filtered to yield a white powder solid product, and dried, wherein a yield ranged from 85% to 97%.

### (3) Synthesis method of product example with structural general formula 1

### Synthesis method of example structure A:

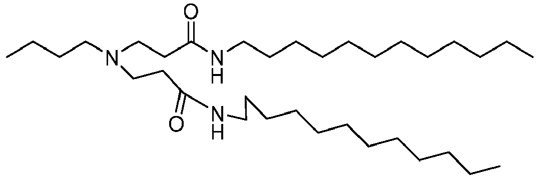

I. Methyl acrylate (2.02 mmol to 3.5 mmol) was added into a three-necked round-bottomed reaction flask, and 1 part to 3 parts of methanol, or ethanol, or dichloromethane, or dichloroethane, or acetone, or acetonitrile was added, or no solvent was added. The temperature was reduced to 7°C to 15°C, 5% to 30% (w/w) of silica gel was added and then stirred under the protection of nitrogen, and n-butylamine (1.0 mmol to 1.5 mmol) was dropwise added. The temperature was raised to the room temperature after dropwise adding, then the reagent was stirred for 5 hours to10 hours, continuously heated to 50°C to 70°C, stirred for 5 hours to 12 hours, and heated to 80°C to 100°C for reaction for 10 hours to 96 hours when necessary. After the completion of the reaction was confirmed by TLC, excessive methyl acrylate was removed under vacuum, and the reaction intermediate without further purification was directly used for a second step.
II. Under the protection of nitrogen, 0.1% to 1.0% of sodium formate or sodium methoxide or triethylamine or N,N-dimethyl pyridine was added into the reaction flask of the reaction intermediate in the first step, and then stirred, and n-octadecylamine (1.9 mmol to 2.5 mmol) was added in batches. The temperature was raised to 50°C, then the reagent was stirred for 3 hours, and continuously heated to 80°C and stirred for 5 hours, and then continuously heated to 106°C for reaction for 48 hours. The reaction was monitored by TLC until the reaction was complete. Petroleum ether was added for recrystallizing to yield a white powder solid, wherein a yield was 93%.
   m.p. 79°C to 80°C.

### Synthesis method of example structure B:

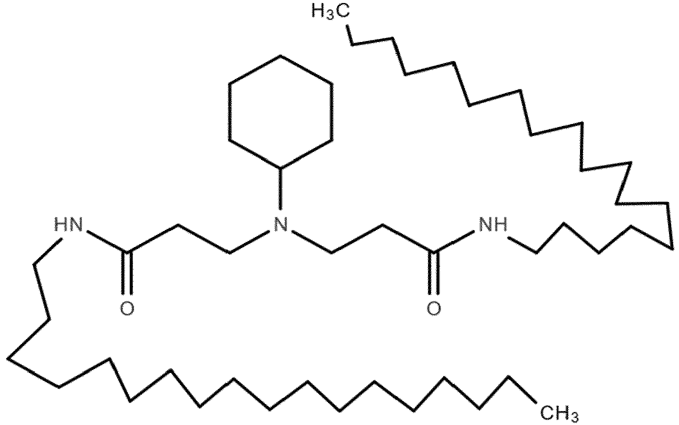

I. Methyl acrylate (2.0 mmol to 3.5 mmol) was added into a three-necked round-bottomed reaction flask, and 1 part to 3 parts of methanol, or ethanol, or acetone, or dichloroethane, or acetonitrile was added, or no solvent was added. The temperature was reduced to 10°C to 30°C, 200 ppm to 1000 ppm hydroquinone benzophenone, or m-nitrophenol, or ortho-methoxyhydroquinone was added, and then stirred under the protection of nitrogen, and cyclohexane (1.0 mmol to 1.5 mmol) was dropwise added. The temperature was raised to the room temperature after dropwise adding, then the reagent was stirred for 5 hours to10 hours, continuously heated to 40°C to 70°C, stirred for 5 hours to 12 hours, and heated to 80°C to 100°C for reaction for 10 hours to 48 hours when necessary. After the completion of the reaction was confirmed by TLC, excessive methyl acrylate was removed under vacuum, and the reaction intermediate without further purification was directly used for a second step.
II. Under the protection of nitrogen, 0.1% to 5% of sodium formate or sodium methoxide or a phosphorous ester ligand complex of Lewis acid tin or zinc was added into the reaction flask of the reaction intermediate in the first step, and then stirred, and n-octadecylamine (1.90 mmol to 2.5 mmol) was added in batches. The temperature was raised to 50°C, then the reagent was stirred for 5 hours, and continuously heated to 80°C and stirred for 5 hours, and then continuously heated to 100°C to 130°C for reaction for 48 hours. The reaction was monitored by TLC until the reaction was complete. Petroleum ether was added for recrystallizing to yield a white powder solid, wherein a yield was 95%.
   m.p. 79°C to 83°C, MS: 1H NMR:

### Synthesis method of example structure C:

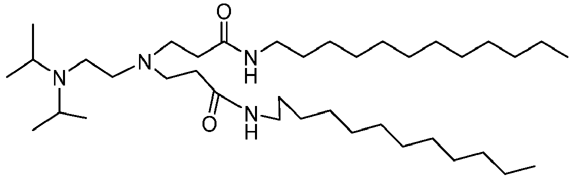

I. Methyl acrylate (2.05 mmol to 3.5 mmol) was added into a three-necked round-bottomed reaction flask, and ethanol, or acetone, or methanol, or acetonitrile, or dichloromethane was added, or no solvent was added. The reagent was stirred under the protection of nitrogen, the temperature was reduced to 10°C to 20°C, 200 ppm to 1500 ppm o-methoxyhydroquinone or 4,4-diphenol hydroxybenzophenone was added, and 2-iisopropylaminoethylamine (1.0 mmol to l.5 mmol) was dropwise added. The temperature was raised to the room temperature after dropwise adding, then the reagent was stirred for 5 hours to10 hours, continuously heated to 50°C to 70°C, stirred for 5 hours to 12 hours, and heated to 80°C to 100°C when necessary. The completion of the reaction was confirmed by TLC. Excessive methyl acrylate was removed under vacuum, and the reaction intermediate without further purification was directly used for a second step.
II. The reagent was stirred, and under the protection of nitrogen, n-octadecylamine (1.90 mmol to 2.5 mmol) was added in batches into the reaction flask of the reaction intermediate in the first step. The temperature was raised to 70°C, then the reagent was stirred for 5 hours, and continuously heated to 85°C and stirred for 5 hours, and then continuously heated to 100°C to 130°C for reaction for 48 hours to 96 hours. The reaction was monitored by TLC until the reaction was complete. Petroleum ether was added for recrystallizing to yield a white powder solid, wherein a yield was 91.5%.
   m.p.: 74°C to 77°C

### Synthesis method of example structure D:

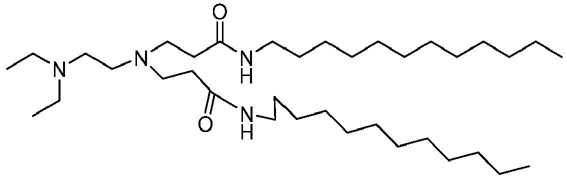

The first step of the synthesis method and the second step of the synthesis method are the same as those above.
A yield was 90.6%. m.p.:

### Synthesis method of example structure E:

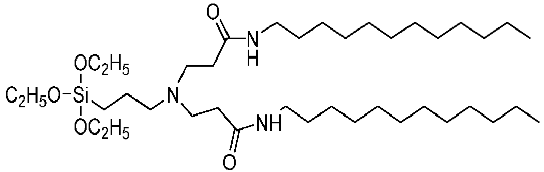

I. Under stirring and the protection of nitrogen, methyl acrylate (2.0 mmol to 3.5 mmol) was added into a three-necked round-bottomed reaction flask, and then ethanol, or acetone, or methanol, or dichloroethane was added, or no solvent was added. The temperature was reduced to 6°C to 15°C, 10% to 30% (400 mesh) of silica gel was added and then stirred under the protection of nitrogen, and KH550 (1.0 mmol to 1.6 mmol) was dropwise added. The temperature was raised to the room temperature after dropwise adding, and then the reagent was stirred for 5 hours to 7 hours, continuously heated to 50°C to 70°C, and stirred for 10 hours to 36 hours. The completion of the reaction was confirmed by TLC. Excessive methyl acrylate was removed under vacuum, and the reaction intermediate without further purification was directly used for a second step.
II. The reagent was stirred, and under the protection of nitrogen, n-octadecylamine (1.9 mmol to 2.5 mmol) was added in batches into the reaction flask of the reaction intermediate in the first step. The temperature was raised to 60°C, then the reagent was stirred for 5 hours, and continuously heated to 80°C and stirred for 5 hours, and then continuously heated to 100°C to 140°C for reaction for 48 hours to 96 hours. The reaction was monitored by TLC until the reaction was complete. Silica gel was filtered, and petroleum ether was recrystallized to yield a white powder solid, wherein a yield was 90.1%.
   m.p.: 67°C to 70°C

### Synthesis method of example structure F:

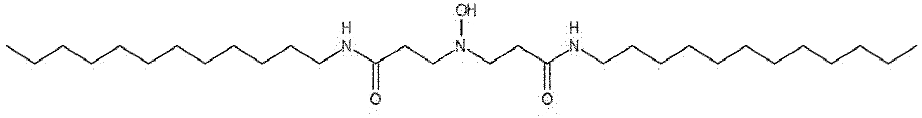

I. Under stirring and the protection of nitrogen, hydroxylamine sulfate or hydroxylamine hydrochloride (1.0 mmol to 1.3 mmol) was dissolved in water, methyl acrylate or ethyl acrylate (1.3 mmol to 3.5 mmol) dissolved in ethyl acetate, or dichloroethane, or dichloromethane, or ethanol, or methanol, or acetonitrile (1 part to 5 parts) or having no solvent was added, 0.1% to 5% of phase transfer catalyst tetrabutylammonium chloride, or tetrabutylammonium bromide, or 18-crown-6, or 15-crown-5, or glycol ether was added, and sodium hydroxide, or potassium hydroxide, or potassium carbonate (1.0 mmol to 3.0 mmol) was dropwise added, stirred at the room temperature for 1 hour to 5 hours, and heated to 28°C to 50°C to continue the reaction for 2 hours to 6 hours. The reaction process was monitored by TLC until the reaction of hydroxylamine was complete. The reagent was extracted with ethyl acetate, or dichloromethane, or dichloroethane, or methyl butyl ether, dried, and filtered to remove the solvent, and the solid without further purification was directly used in next step.
II. The reagent was stirred, and under the protection of nitrogen, n-octadecylamine (1.9 mmol to 2.5 mmol) was added in batches into the reaction flask of the reaction intermediate in the first step. The temperature was raised to 50°C to 60°C, then the reagent was stirred for 5 hours, and continuously heated to 70°C to 90°C and stirred for 15 hours to 32 hours, and then continuously heated to 95°C to 140°C for reaction for 48 hours to 96 hours. The reaction was monitored by TLC until the reaction was complete. Ethanol or methanol containing 3% to 15% of water was recrystallized to yield a white powder solid, wherein a yield was 87.9%.
   m.p.: 97°C to 99°C

### (II) Synthesis method of novel light stabilizer with structural general formula 2

### (1) Synthesis route of novel light stabilizer with structural general formula 2

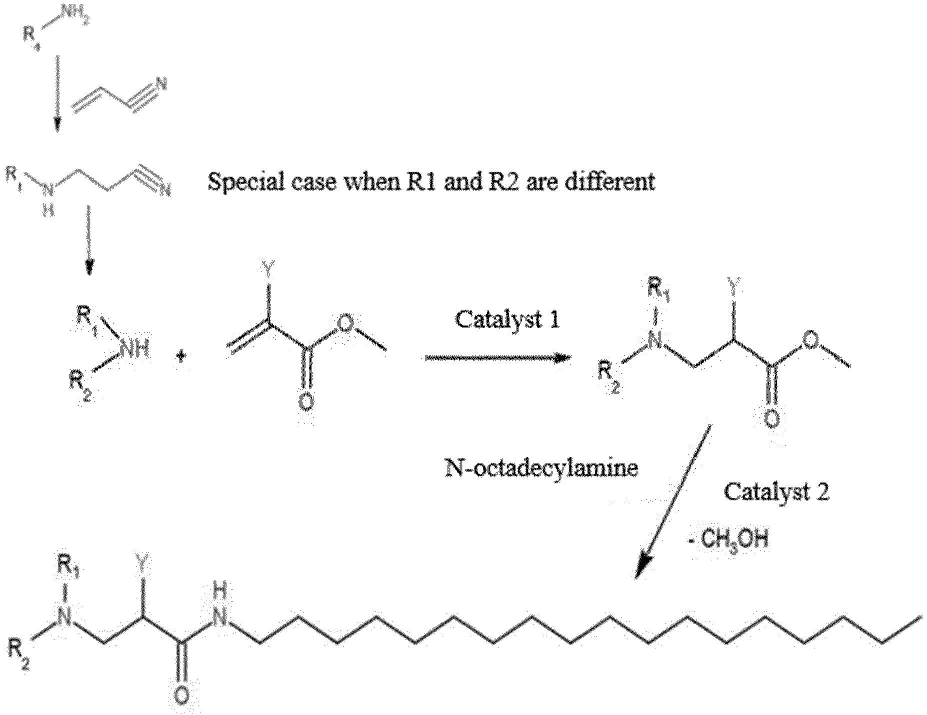

### (2) General synthesis method of structural general formula 2

I. Under the protection of nitrogen, methyl acrylate or methyl methacrylate (1.05 mmol to 3.3 mmol) was added into a reaction flask, and then stirred, 1 part to 3 parts of methanol, or ethanol, or ethyl acetate, or dichloroethane, or acetone, or acetonitrile, or DMF was added, or no solvent was added, and 0.02% to 30% of catalyst 1 was added (the catalyst 1 was acetic acid, acidic alumina, silica gel, ortho-methoxyhydroquinone, 4,4-diphenol hydroxy dibenzophenone, or m-nitrophenol). The temperature was reduced to 5°C to 10°C, and then second amine (1.0 mmol to 1.5 mmol) was dropwise added slowly. The temperature was raised to a room temperature after dropwise adding, and then the reagent was stirred for 6 hours to 24 hours, and if the reaction needs to be continued, the reagent was heated to 40°C to 80°C to continue the reaction for 5 hours to 18 hours. The reaction process was monitored by TLC until the reaction was complete, excessive methyl acrylate was removed under vacuum, and the remaining reaction intermediate without further purification was used for next reaction.
II. Under the protection of nitrogen and stirring, 0.1% to 5% of catalyst 2 was added into the intermediate obtained in the reaction of the first step at the room temperature (the catalyst 2 could be sodium methoxide, sodium formate, diethyl tin oxide or aluminum isooctanol), and then n-dodecylamine, or n-hexadecylamine, or n-octadecylamine, or n-dodecanol, or n-octadecanol (0.9 mmol to 1.2 mmol) was added in batches. After charging, the temperature was raised to 40°C to 70°C for reaction for 8 hours to 16 hours, and continuously raised to 85°C to 140°C for reaction for 48 hours to 96 hours, and the reaction process was monitored by TLC until the reaction was complete. The catalyst was removed, ethanol, or methanol, or ethyl acetate, or petroleum ether containing 0.5% to 20% of water was added for recrystallizing, filtered to yield a white powder solid product, and dried, wherein a yield ranged from 87% to 96%.

### (3) Synthesis method of product example with structural general formula 2

### Synthesis method of example structure A:

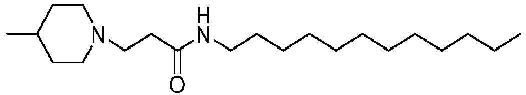

I. Under the protection of nitrogen, methyl acrylate (1.05 mmol to 3.3 mmol) was added into a reaction flask, and then stirred, 1 part to 3 parts of methanol, or ethanol, or ethyl acetate, or dichloroethane, or acetone, or acetonitrile, or DMF was added, or no solvent was added, and 100 ppm to 1000 ppm catalyst o-methoxyhydroquinone, or 4,4-diphenol hydroxy dibenzophenone, or m-nitrophenol was added. The temperature was reduced to 10°C to 15°C, and then 4-methylpiperidine amine (0.9 mmol to 1.5 mmol) was dropwise added slowly. The temperature was raised to a room temperature after dropwise adding, then the reagent was stirred for 10 hours to 20 hours, and heated to 40°C to 50°C to continue the reaction for 5 hours to 7 hours. The reaction process was monitored by TLC until the reaction was complete, excessive methyl acrylate was removed under vacuum, and the remaining reaction intermediate without further purification was directly used for next reaction.
II. Under the protection of nitrogen and stirring, 0.1% to 1% of sodium methoxide or sodium formate was added into the intermediate obtained in the reaction of the first step at the room temperature, and then n-octadecylamine (0.9 mmol to 1.2 mmol) was added in batches. After charging, the temperature was raised to 50°C to 70°C for reaction for 12 hours to 18 hours, and continuously raised to 85°C to 140°C for reaction for 48 hours to 96 hours, and the reaction process was monitored by TLC until the reaction was complete. The catalyst was removed, ethanol or methanol containing 5% of water was added for recrystallizing to yield a white powder solid product, and dried, wherein a yield was 91.2%.
   m.p.

### Synthesis method of example structure B:

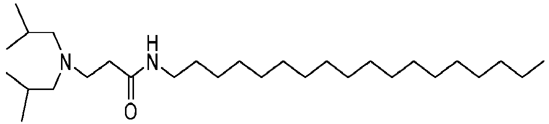

I. Under the protection of nitrogen, methyl acrylate (1.3 mmol to 3.5 mmol) was added into a reaction flask, and then stirred, 1 part to 3 parts of methanol, or ethanol, or ethyl acetate, or dichloroethane, or acetone, or acetonitrile, or DMF was added, or no solvent was added, and 30% silica gel (400 mesh) was added. The temperature was reduced to 15°C to 20°C, and then diisobutylamine (0.9 mmol to 1.5 mmol) was dropwise added slowly. The temperature was raised to a room temperature after dropwise adding, then the reagent was stirred for 15 hours to 20 hours, and heated to 40°C to 50°C to continue the reaction for 3 hours to 5 hours. The reaction process was monitored by TLC until the reaction was complete, excessive methyl acrylate was removed under vacuum, and the remaining reaction intermediate without further purification was directly used for next reaction.
II. Under the protection of nitrogen and stirring, 0.1% to 1% of sodium methoxide or sodium formate was added into the intermediate obtained in the reaction of the first step at the room temperature, and then n-octadecylamine (0.9 mmol to 1.2 mmol) was added in batches. After charging, the temperature was raised to 60°C to 70°C for reaction for 10 hours to 13 hours, and continuously raised to 80°C to 140°C for reaction for 48 hours to 96 hours, and the reaction process was monitored by TLC until the reaction was complete. The catalyst was removed, petroleum ether containing 0.5% of water was added for recrystallizing to yield a white powder solid product, and dried, wherein a yield was 89.3%.
   m.p. 79°C to 81°C

### Synthesis method of example structure C:

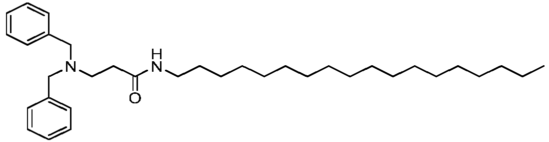

I. Under the protection of nitrogen, methyl acrylate (1.3 mmol to 3.5 mmol) was added into a reaction flask, and then stirred, 1 part to 3 parts of methanol, or ethanol, or ethyl acetate, or dichloroethane, or acetone, or acetonitrile, or DMF was added, or no solvent was added, and 300 ppm to 700 ppm 4,4'-diphenol hydroxybenzophenone was added. The temperature was reduced to 10°C to 15°C, and then dibenzyl amine (0.9 mmol to 1.5 mmol) was dropwise added slowly. The temperature was raised to a room temperature after dropwise adding, then the reagent was stirred for 18 hours to 20 hours, and heated to 50°C to 60°C to continue the reaction for 5 hours to 7 hours. The reaction process was monitored by TLC until the reaction was complete, excessive methyl acrylate was removed under vacuum, and the remaining reaction intermediate without further purification was directly used for next reaction.
II. Under the protection of nitrogen and stirring, 0.1% to 1% of sodium methoxide or sodium formate was added into the intermediate obtained in the reaction of the first step at the room temperature or no catalyst was added, and then n-octadecylamine (0.9 mmol to 1.2 mmol) was added in batches. After charging, the temperature was raised to 50°C to 70°C for reaction for 15 hours to 18 hours, and continuously raised to 85°C to 140°C for reaction for 24 hours to 96 hours, and the reaction process was monitored by TLC until the reaction was complete. The catalyst was removed, ethanol containing 5% of water was added for recrystallizing to yield a white powder solid product, and dried, wherein a yield was 92%.
   m.p. 140°C to 143°C

### Synthesis method of example structure D:

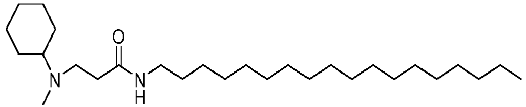

I. Under the protection of nitrogen, methyl acrylate (1.3 mmol to 3.5 mmol) was added into a reaction flask, and then stirred, 1 part to 3 parts of methanol, or ethanol, or ethyl acetate, or dichloroethane, or acetone, or acetonitrile, or DMF was added, or no solvent was added, and 200 ppm to 600 ppm 4,4'-diphenol hydroxybenzophenone or o-methoxyhydroquinone was added. The temperature was reduced to 15°C to 18°C, and then methylcyclohexylamine (0.95 mmol to 1.2 mmol) was dropwise added slowly. The temperature was raised to a room temperature after dropwise adding, then the reagent was stirred for 15 hours to 20 hours, and heated to 50°C to 60°C to continue the reaction for 2 hours to 3 hours. The reaction process was monitored by TLC until the reaction was complete, excessive methyl acrylate was removed under vacuum, and the remaining reaction intermediate without further purification was directly used for next reaction.
II. Under the protection of nitrogen and stirring, 0.1% to 1% of sodium methoxide or sodium formate was added into the intermediate obtained in the reaction of the first step at the room temperature or no catalyst was added, and then n-octadecylamine (0.95 mmol to 1.0 mmol) was added in batches. After charging, the temperature was raised to 50°C to 60°C for reaction for 5 hours to 8 hours, and continuously raised to 85°C to 140°C for reaction for 24 hours to 72 hours, and the reaction process was monitored by TLC until the reaction was complete. The catalyst was removed, ethanol containing 5% to 10% of water was added for recrystallizing to yield a white powder solid product, and dried, wherein a yield was 87%.
   m.p. 67°C to 70°C

### Synthesis method of example structure E:

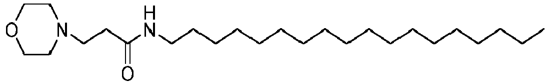

I. Under the protection of nitrogen, methyl acrylate (1.5 mmol to 3.0 mmol) was added into a reaction flask, and then stirred, 1 part to 3 parts of methanol, or ethanol, or ethyl acetate, or dichloroethane, or acetone, or acetonitrile, or DMF was added, or no solvent was added, and 100 ppm to 600 ppm 4,4'-diphenol hydroxybenzophenone or o-methoxyhydroquinone was added. Morpholine (0.97 mmol to 1.3 mmol) was dropwise added slowly at a room temperature. The reagent was stirred for 18 hours to 36 hours at the room temperature (25°C), and heated to 40°C to 60°C to continue the reaction for 3 hours to 6 hours. The reaction process was monitored by TLC until the reaction was complete, excessive methyl acrylate was removed under vacuum, and the remaining reaction intermediate without further purification was directly used for next reaction.
II. Under the protection of nitrogen and stirring, 0.1% to 1% of sodium methoxide or sodium formate was added into the intermediate obtained in the reaction of the first step at the room temperature or no catalyst was added, and then n-octadecylamine (0.90 mmol to 1.10 mmol) was added in batches. After charging, the temperature was raised to 55°C to 70°C for reaction for 6 hours to 8 hours, and continuously raised to 80°C to 140°C for reaction for 24 hours to 72 hours, and the reaction process was monitored by TLC until the reaction was complete. The catalyst was removed, ethanol containing 5% of water was added for recrystallizing to yield a white powder solid product, and dried, wherein a yield was 89.3%.
   m.p.

### Synthesis method of example structure F:

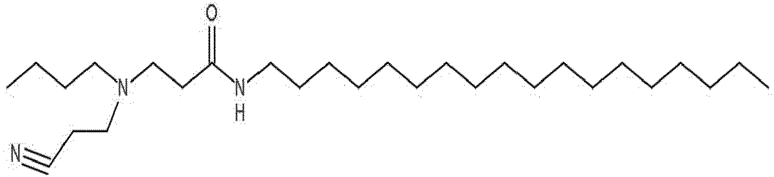

I. Under the protection of nitrogen, n-butylamine (1.2 mmol to 1.5 mmol) was added into a reaction flask, 1 part to 3 parts of methanol, or ethanol, or dichloroethane, or acetonitrile was added, and then 15% to 30% of silica gel or acidic alumina or 0.03% to 0.7% of 4,4-diphenol hydroxybenzophenone or o-hydroxyhydroquinone was added. Acrylonitrile (1.0 mmol) was dropwise added at a room temperature, stirred for 2 hours to 5 hours at the room temperature after dropwise adding, and then heated to 30°^{C} to 65°^{C} to continue the reaction for 6 hours to 15 hours. The reaction process was monitored by TLC until the reaction was complete. Excessive n-butylamine was removed under vacuum, and the remaining oily matter without further purification was directly used in a second step.
II. Under the protection of nitrogen and stirring, 1 part to 3 parts of methanol, or ethyl acetate, or dichloroethane was added into the oily matter (1.0 mmol) in the above step, and then methyl acrylate (1.5 mmol to 3.0 mmol) was dropwise added into the reaction flask. The reagent was stirred for 10 hours to 36 hours at the room temperature (25°C), and heated to 40°C to 60°C to continue the reaction for 5 hours to 15 hours. The reaction process was monitored by TLC until the reaction was complete, excessive methyl acrylate was removed under vacuum, and the remaining reaction intermediate oily matter without further purification was directly used for next reaction.

### Reaction of third step:

Under the protection of nitrogen and stirring, 0.1% to 1% of sodium methoxide or sodium formate was added into the intermediate oily matter obtained in the reaction of the second step at the room temperature or no catalyst was added, and then n-octadecylamine (0.90 mmol to 1.10 mmol) was added in batches. After charging, the temperature was raised to 55°C to 75°C for reaction for 8 hours to 16 hours, and continuously raised to 80°C to 140°C for reaction for 24 hours to 72 hours, and the reaction process was monitored by TLC until the reaction was complete. The catalyst was removed, ethanol containing 5% of water was added for recrystallizing to yield a white powder solid product, and dried, wherein a yield was 86.5%.
m.p.

### (III) Synthesis method of novel light stabilizer with structural general formula 3

### (1) Synthesis route of novel light stabilizer with structural general formula 3

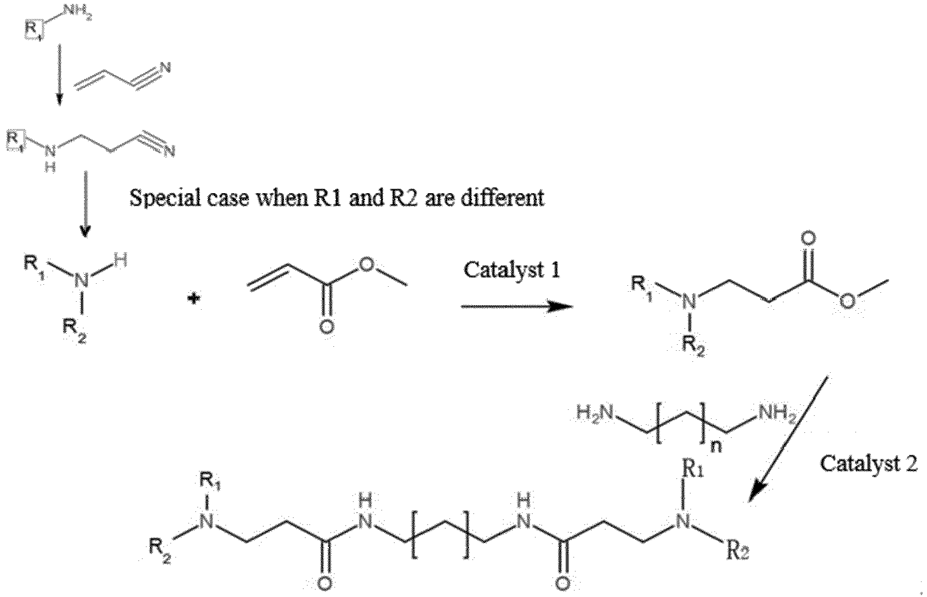

### (2) General synthesis method of structural general formula 3

I. Under the protection of nitrogen, methyl acrylate or methyl methacrylate (2.5 mmol to 4.5 mmol) was added into a reaction flask, and then stirred, 1 part to 3 parts of methanol, or ethanol, or ethyl acetate, or dichloroethane, or acetone, or acetonitrile, or DMF was added, or no solvent was added, and 0.01% to 30% of catalyst 1 was added (the catalyst 1 was acetic acid, acidic alumina, silica gel, ortho-methoxyhydroquinone, 4,4-diphenol hydroxy dibenzophenone, or m-nitrophenol). The temperature was reduced to 5°C to 20°C, and then second amine (1.90 mmol to 2.05 mmol) was dropwise added slowly. The temperature was raised to a room temperature after dropwise adding, and then the reagent was stirred for 5 hours to 24 hours, and heated to 40°C to 80°C if the reaction needs to continue the reaction for 5 hours to 18 hours. The reaction process was monitored by TLC until the reaction was complete, excessive methyl acrylate was removed under vacuum, and the remaining reaction intermediate without further purification was used for next reaction.
II. Under the protection of nitrogen and stirring, alkyl diamine, such as ethylenediamine, or butanediamine, or hexamethylenediamine, or decanediamine (1.0 mmol to 1.2 mmol) was added in batches first into the intermediate obtained in the first step at a room temperature, and then 0.1% to 5% of catalyst 2 was added (the catalyst 2 was sodium methoxide, sodium formate, diethyl tin oxide or aluminum isooctanol). After charging, the temperature was raised to 50°C to 70°C for reaction for 6 hours to 16 hours, and continuously raised to 85°C to 120°C for reaction for 48 hours to 96 hours, and the reaction process was monitored by TLC until the reaction was complete. The catalyst was removed, ethanol, or methanol, or ethyl acetate, or petroleum ether containing 0.5% to 20% of water was added for recrystallizing, filtered to yield a white powder solid product, and dried, wherein a yield ranged from 85% to 95%.

### (3) Synthesis method of product example with structural general formula 3

### Synthesis method of example structure A:

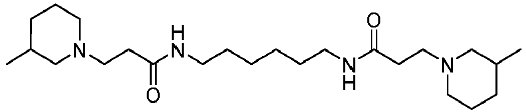

I. Under the protection of nitrogen, methyl acrylate (2.2 mmol to 3.5 mmol) was added into a reaction flask, and then stirred, 1 part to 3 parts of methanol, or ethanol, or ethyl acetate, or dichloroethane, or acetone, or acetonitrile, or DMF was added, or no solvent was added, and then 30% silica gel (400 mesh) was added. The temperature was reduced to 15°C to 20°C, and then 3-methylpiperidine amine (1.95 mmol to 2.01 mmol) was dropwise added slowly. The temperature was raised to a room temperature after dropwise adding, then the reagent was stirred for 5 hours to 7 hours, and continuously heated to 40°C to 50°C to continue the reaction for 5 hours to 10 hours. The reaction process was monitored by TLC until the reaction was complete, excessive methyl acrylate was removed under vacuum, and the remaining reaction intermediate without further purification was used for next reaction.
II. Under the protection of nitrogen and stirring, hexanediamine (1.0 mmol to 1.05 mmol) was added in batched first into the intermediate obtained in the reaction of the first step at the room temperature, and then 0.1% of sodium methoxide or sodium formate was added. After charging, the temperature was raised to 50°C to 60°C for reaction for 10 hours to 15 hours, and continuously raised to 85°C to 120°C for reaction for 48 hours to 96 hours, and the reaction process was monitored by TLC until the reaction was complete. The catalyst was removed, methanol, or ethyl acetate, or petroleum ether containing 0.5% to 5% of water was added for recrystallizing, filtered to yield a white powder solid product, and dried, wherein a yield was 83.9%.
   m.p.

### Synthesis method of example structure B:

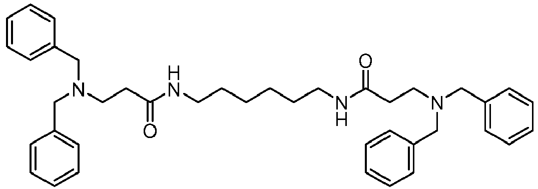

I. Under the protection of nitrogen, methyl acrylate (2.5 mmol to 4.5 mmol) was added into a reaction flask, and then stirred, 1 part to 3 parts of methanol, or ethanol, or ethyl acetate, or dichloroethane, or acetone, or acetonitrile, or DMF was added, or no solvent was added, and 100 ppm to 600 ppm 4,4'-diphenol hydroxybenzophenone, or o-methoxyhydroquinone, or m-nitrophenol was added. The temperature was reduced to 20°C, and then dibenzylamine (1.96 mmol to 2.03 mmol) was dropwise added slowly. The reagent was stirred for 5 hours at the room temperature after dropwise adding, and heated to 40°C to 60°C to continue the reaction for 10 hours to 32 hours. The reaction process was monitored by TLC until the reaction was complete, excessive methyl acrylate was removed under vacuum, and the remaining reaction intermediate without further purification was used for next reaction.
II. Under the protection of nitrogen and stirring, hexanediamine (1.05 mmol to 1.15 mmol) was added in batched first into the intermediate obtained in the reaction of the first step at the room temperature, and then 0.1% of sodium methoxide or sodium formate was added, or no catalyst was added. After charging, the temperature was raised to 50°C to 70°C for reaction for 8 hours to 10 hours, and continuously raised to 80°C to 125°C for reaction for 32 hours to 72 hours, and the reaction process was monitored by TLC until the reaction was complete. The catalyst was removed, ethanol, or ethyl acetate, or petroleum ether containing 5% of water was added for recrystallizing, filtered to yield a white powder solid product, and dried, wherein a yield was 90.3%.
   m.p. 140°C to 143°C

### Synthesis method of example structure C:

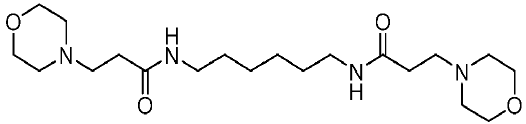

I. Under the protection of nitrogen, methyl acrylate (2.5 mmol to 3.8 mmol) was added into a reaction flask, and then stirred, 1 part to 3 parts of methanol, or ethanol, or ethyl acetate, or dichloroethane, or acetone, or acetonitrile, or DMF was added, or no solvent was added, then 200 ppm to 700 ppm 4,4'-diphenol hydroxybenzophenone, or o-methoxyhydroquinone, or m-nitrophenol, or m-nitrophenol was added, and then dibenzylamine (1.96 mmol to 2.03 mmol) was dropwise added slowly at a room temperature. The reagent was stirred for 18 hours at the room temperature after dropwise adding, and heated to 40°C to 60°C if necessary to continue the reaction for 5 hours to 15 hours. The reaction process was monitored by TLC until the reaction was complete, excessive methyl acrylate was removed under vacuum, and the remaining reaction intermediate without further purification was used for next reaction.
II. Under the protection of nitrogen and stirring, hexanediamine (1.05 mmol to 1.15 mmol) was added in batched first into the intermediate obtained in the reaction of the first step at the room temperature, and then 0.1% of sodium methoxide or sodium formate was added, or no catalyst was added. After charging, the temperature was raised to 50°C to 70°C for reaction for 6 hours to 8 hours, and continuously raised to 80°C to 125°C for reaction for 48 hours to 72 hours, and the reaction process was monitored by TLC until the reaction was complete. The catalyst was removed, ethanol, or ethyl acetate, or petroleum ether containing 5% of water was added for recrystallizing, filtered to yield a white powder solid product, and dried, wherein a yield was 92.6%.
   m.p. 122°C to 125°C

### Synthesis method of example structure D:

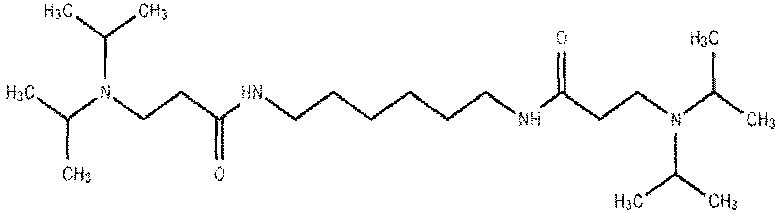

I. Under the protection of nitrogen, methyl acrylate (2.80 mmol to 4.8 mmol) was added into a reaction flask, and then stirred, 1 part to 3 parts of methanol, or ethanol, or ethyl acetate, or dichloroethane, or acetone, or acetonitrile, or DMF was added, or no solvent was added, then 200 ppm to 700 ppm 4,4'-diphenol hydroxybenzophenone, or o-methoxyhydroquinone, or m-nitrophenol was added, and then diisopropylamine (1.95 mmol to 2.01 mmol) was dropwise added slowly at a room temperature. The reagent was stirred for 18 hours at the room temperature after dropwise adding, and heated to 50°C to 60°C to continue the reaction for 18 hours to 32 hours. The reaction process was monitored by TLC until the reaction was complete, excessive methyl acrylate was removed under vacuum, and the remaining reaction intermediate without further purification was used for next reaction.
II. Under the protection of nitrogen and stirring, hexanediamine (1.05 mmol to 1.15 mmol) was added into the intermediate obtained in the reaction of the first step at the room temperature, and then 0.1% of sodium methoxide or sodium formate was added, or no catalyst was added. After charging, the temperature was raised to 60°C to 70°C for reaction for 8 hours to 10 hours, and continuously raised to 80°C to 125°C for reaction for 48 hours to 72 hours, and the reaction process was monitored by TLC until the reaction was complete. The catalyst was removed, ethyl acetate or petroleum ether containing 0.5% of water was added for recrystallizing, filtered to yield a white powder solid product, and dried, wherein a yield was 86%.
   m.p. 115°C to 118°C

### Synthesis method of example structure E:

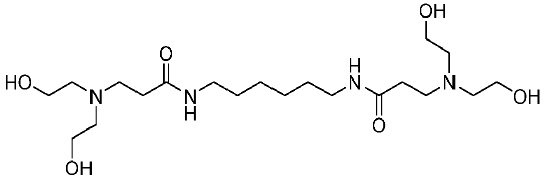

I. Under the protection of nitrogen, methyl acrylate (2.8 mmol to 3.8 mmol) was added into a reaction flask, and then stirred, 1 part to 3 parts of methanol, or ethanol, or ethyl acetate, or dichloroethane, or acetone, or acetonitrile, or DMF was added, or no solvent was added, then 20% of acidic alumina was added, and then bis(2-hydroxyethyl)amine (1.97 mmol to 2.0 mmol) was dropwise added slowly at a room temperature. The reagent was stirred for 5 hours at the room temperature after dropwise adding, and heated to 35°C to 60°C to continue the reaction for 15 hours to 24 hours. The reaction process was monitored by TLC until the reaction was complete, excessive methyl acrylate was removed under vacuum, and the remaining reaction intermediate without further purification was used for next reaction.
II. Under the protection of nitrogen and stirring, hexanediamine (1.0 mmol to 1.1 mmol) was added in batched first into the intermediate obtained in the reaction of the first step at the room temperature, and then 0.1% of sodium methoxide or sodium formate was added, or no catalyst was added. After charging, the temperature was raised to 50°C to 70°C for reaction for 6 hours to 8 hours, and continuously raised to 80°C to 125°C for reaction for 48 hours to 72 hours, and the reaction process was monitored by TLC until the reaction was complete. The catalyst was removed, ethanol or methanol containing 5% to 10% of water was added for recrystallizing, and filtered to yield a colorless oily liquid product, and dried, wherein a yield was 95.6%.
   m.p.: 147°C to 151°C

### Synthesis method of example structure F:

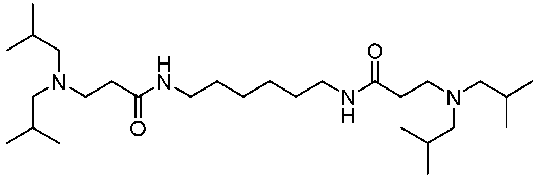

I. Under the protection of nitrogen, methyl acrylate (2.6 mmol to 3.5 mmol) was added into a reaction flask, and then stirred, 1 part to 3 parts of methanol, or ethanol, or ethyl acetate, or dichloroethane, or acetone, or acetonitrile, or DMF was added, or no solvent was added, and then 4,4-diphenol hydroxybenzophenone or o-methoxyhydroquinone was added or no catalyst was added. Diisobutyl amine (1.98 mmol to 2.0 mmol) was dropwise added slowly at a room temperature. The reagent was stirred for 5 hours at the room temperature after dropwise adding, and heated to 30°C to 60°C to continue the reaction for 15 hours to 24 hours. The reaction process was monitored by TLC until the reaction was complete, excessive methyl acrylate was removed under vacuum, and the remaining reaction intermediate without further purification was used for next reaction.
II. Under the protection of nitrogen and stirring, hexanediamine (0.95 mmol to 1.1 mmol) was added in batched first into the intermediate obtained in the reaction of the first step at the room temperature, and then 0.1% of sodium methoxide or sodium formate was added, or no catalyst was added. After charging, the temperature was raised to 50°C to 70°C for reaction for 6 hours to 8 hours, and continuously raised to 80°C to 120°C for reaction for 48 hours to 72 hours, and the reaction process was monitored by TLC until the reaction was complete. The catalyst was removed, ethanol or methanol containing 5% to 15% of water was added for recrystallizing, and filtered to yield a white powder solid product, and dried, wherein a yield was 93.5%.
   m.p. 105°C to 107°C

### (IV) Synthesis method of novel light stabilizer with structural general formula 4

### (1) Synthesis route of novel light stabilizer with structural general formula 4

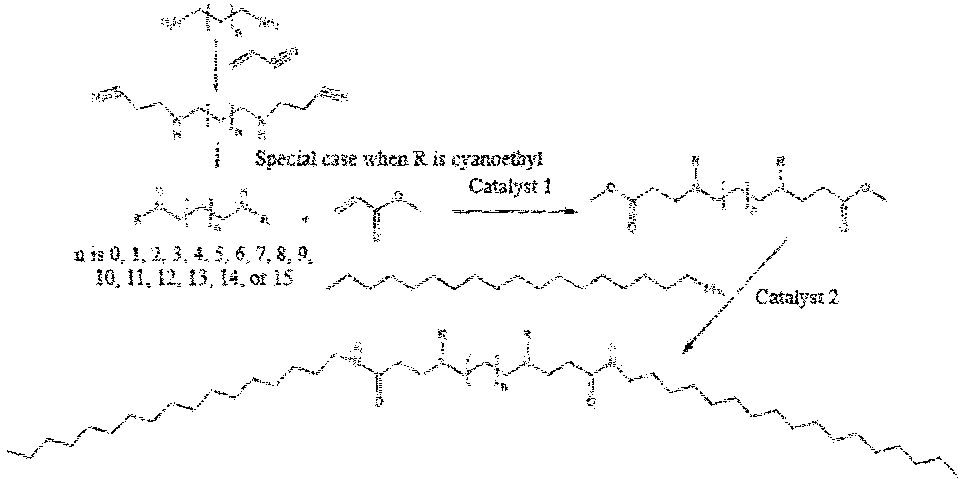

### (2) General synthesis method of structural general formula 4

I. Under the protection of nitrogen, methyl acrylate or methyl methacrylate (2.5 mmol to 4.5 mmol) was added into a reaction flask, and then stirred, 1 part to 3 parts of methanol, or ethanol, or ethyl acetate, or dichloroethane, or acetone, or acetonitrile, or DMF was added, or no solvent was added, and then 100 ppm to 1000 ppm o-methoxyhydroquinone, or 4,4-diphenol hydroxy dibenzophenone, or m-nitrophenol was added. The temperature was reduced to 5°C to 20°C, and then second amine (0.90 mmol to 1.15 mmol) was dropwise added slowly. The temperature was raised to a room temperature after dropwise adding, and then the reagent was stirred for 8 hours to 32 hours, and if the reaction needs to be continued, the reagent was heated to 40°C to 60°C to continue the reaction for 5 hours to 24 hours. The reaction process was monitored by TLC until the reaction was complete, excessive methyl acrylate was removed under vacuum, and the remaining reaction intermediate without further purification was used for next reaction.
II. Under the protection of nitrogen and stirring, dodecylamine, or hexadecylamine, or octadecylamine, or hexadecanol, or octadecanol, or dodecanol (1.95 mmol to 2.20 mmol) was added in batched first into the intermediate obtained in the first step at a room temperature, and then 0.02% to 5% of catalyst 2 was added (the catalyst 2 was sodium methoxide, sodium formate, diethyl tin oxide or aluminum isooctanol). After charging, the temperature was raised to 50°C to 70°C for reaction for 8 hours to 10 hours, and continuously raised to 80°C to 120°C for reaction for 48 hours to 96 hours, and the reaction process was monitored by TLC until the reaction was complete. The catalyst was removed, ethanol, or methanol, or ethyl acetate, or petroleum ether containing 0.5% to 20% of water was added for recrystallizing, filtered to yield a white powder solid product, and dried, wherein a yield ranged from 83% to 96%.

### (3) Synthesis method of product example with structural general formula 4

### Synthesis method of example structure A:

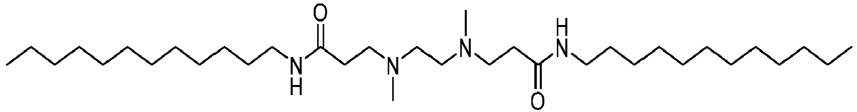

I. Under the protection of nitrogen, methyl acrylate (2.5 mmol to 3.8 mmol) was added into a reaction flask, and then stirred, 1 part to 3 parts of methanol, or ethanol, or ethyl acetate, or dichloroethane, or acetone, or acetonitrile, or DMF was added, or no solvent was added, and then 300 ppm to 700 ppm o-methoxyhydroquinone, or 4,4-diphenol hydroxy dibenzophenone, or m-nitrophenol was added. The temperature was reduced to 15°C to 20°C, and then N,N'-dimethyl ethylenediamine (1.0 mmol to 1.1 mmol) was dropwise added slowly. The temperature was raised to a room temperature after dropwise adding, then the reagent was stirred for 8 hours to 10 hours, and continuously heated to 40°C to 60°C to continue the reaction for 10 hours to 24 hours. The reaction process was monitored by TLC until the reaction was complete, excessive methyl acrylate was removed under vacuum, and the remaining reaction intermediate without further purification was used for next reaction.
II. Under the protection of nitrogen and stirring, octadecylamine (1.95 mmol to 2.0 mmol) was added in batched first into the intermediate obtained in the reaction of the first step at the room temperature, and then 0.02% to 5% of sodium methoxide or sodium formate was added. After charging, the temperature was raised to 50°C to 60°C for reaction for 5 hours to 8 hours, and continuously raised to 80°C to 120°C for reaction for 48 hours to 72 hours, and the reaction process was monitored by TLC until the reaction was complete. The catalyst was removed, ethanol, or methanol, or ethyl acetate, or petroleum ether containing 0.5% to 20% of water was added for recrystallizing, filtered to yield a white powder solid product, and dried, wherein a yield was 93%.
   m.p. 90°C to 93°C

### Synthesis method of example structure B:

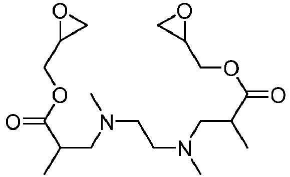

I. Under the protection of nitrogen, glycidyl methacrylate (1.1 mmol to 1.5 mmol) was added into a reaction flask, and then stirred, 1 part to 3 parts of ethyl acetate, or dichloroethane, or acetone, or acetonitrile, or DMF was added, or no solvent was added, and then 200 ppm to 600 ppm o-methoxyhydroquinone, or 4,4-diphenol hydroxy dibenzophenone, or m-nitrophenol was added. The temperature was reduced to 20°C, and then N,N'-dimethyl ethylenediamine (0.95 mmol to 1.05 mmol) was dropwise added slowly. The temperature was raised to a room temperature after dropwise adding, then the reagent was stirred for 10 hours to 18 hours, and continuously heated to 40°C to 60°C to continue the reaction for 10 hours to 20 hours. The reaction process was monitored by TLC until the reaction was complete, and excessive epoxypropylmethyl acrylate was removed under vacuum to yield an oily product, wherein a yield was 96%.

### Synthesis method of example structure C:

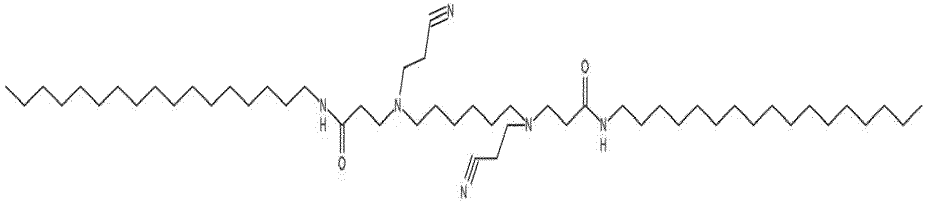

I. Under the protection of nitrogen, acrylonitrile (1.95 mmol to 1.98 mmol) was dropwise added into a reaction flask with hexamethylenediamine (1.0 mmol to 1.05 mmol), stirred for 10 hours to 18 hours at a room temperature, and heated to 40°C to 70°C to continue the reaction for 8 hours to 15 hours. The reaction process was monitored by TLC until the reaction was complete. Excessive hexamethylenediamine was removed to yield the oily intermediate, and the oily intermediate without further purification was directly used for a second step.
II. Under the protection of nitrogen and stirring, 1 part to 3 parts of methanol, or ethanol, or ethyl acetate, or acetonitrile, or dichloromethane was added into the intermediate (0.95 mmol to 1.0 mmol) obtained in the above step, then 300 ppm to 700 ppm o-methoxyhydroquinone, or 4,4-diphenol hydroxy dibenzophenone, or m-nitrophenol was added, and methyl acrylate (2.6 mmol to 3.9 mmol) was dropwise added into the reaction flask. The temperature was raised to a room temperature after dropwise adding, then the reagent was stirred for 10 hours to 18 hours, and continuously heated to 40°C to 70°C to continue the reaction for 24 hours to 36 hours. The reaction process was monitored by TLC until the reaction was complete, excessive methyl acrylate was removed under vacuum, and the remaining reaction oily intermediate without further purification was used for next reaction.
III. Under the protection of nitrogen and stirring, octadecylamine (1.93 mmol to 1.97 mmol) was added in batched first into the oily intermediate (0.95 mmol to 1.0 mmol) obtained in the reaction of the second step at the room temperature, and then 0.02% to 5% of sodium methoxide or sodium formate was added. After charging, the temperature was raised to 50°C to 60°C for reaction for 5 hours to 8 hours, and continuously raised to 80°C to 130°C for reaction for 48 hours to 72 hours, and the reaction process was monitored by TLC until the reaction was complete. The catalyst was removed, ethanol, or methanol, or ethyl acetate, or petroleum ether containing 0.5% to 20% of water was added for recrystallizing, filtered to yield a white powder solid product, and dried, wherein a yield was 89.6%.
   m.p. 96°C to 99°C

### (V) Synthesis method of novel light stabilizer with structural general formula 5

### (1) Synthesis route of novel light stabilizer with structural general formula 5

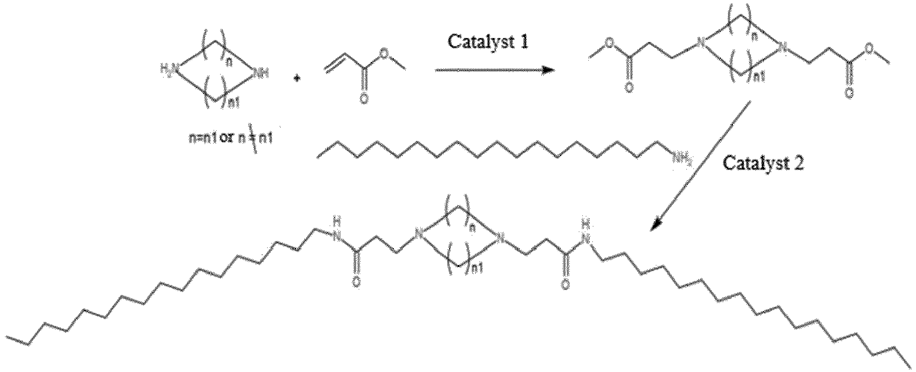

### (2) General synthesis method of structural general formula 5

I. Under the protection of nitrogen, methyl acrylate or methyl methacrylate (1.5 mmol to 3.5 mmol) was added into a reaction flask, and then stirred, 1 part to 3 parts of methanol, or ethanol, or ethyl acetate, or dichloroethane, or acetone, or acetonitrile, or DMF was added, or no solvent was added, and then 100 ppm to 1000 ppm o-methoxyhydroquinone, or 4,4-diphenol hydroxy dibenzophenone, or m-nitrophenol was added. The temperature was reduced to 10°C to 20°C, and then cyclic alkyl diamine (0.90 mmol to 1.15 mmol) was dropwise added slowly. The temperature was raised to a room temperature after dropwise adding, then the reagent was stirred for 3 hours to 5 hours, and heated to 40°C to 60°C to continue the reaction for 10 hours to 24 hours. The reaction process was monitored by TLC until the reaction was complete, excessive methyl acrylate was removed under vacuum, and the remaining reaction intermediate without further purification was used for next reaction.
II. Under the protection of nitrogen and stirring, octadecylamine, or dodecylamine, or hexadecylamine (1.95 mmol to 2.20 mmol) was added in batched first into the intermediate obtained in the reaction of the first step at the room temperature, and then 0.1% to 0.5% of sodium methoxide or sodium formate was added, or no catalyst was added. After charging, the temperature was raised to 50°C to 60°C for reaction for 5 hours to 8 hours, and continuously raised to 80°C to 120°C for reaction for 48 hours to 72 hours, and the reaction process was monitored by TLC until the reaction was complete. The catalyst was removed, ethanol or methanol containing 5% to 10% of water was added for recrystallizing, and filtered to yield a white powder solid product, and dried, wherein a yield ranged from 83% to 92%.

### (3) Synthesis method of product example with structural general formula 5

### Synthesis method of example structure A:

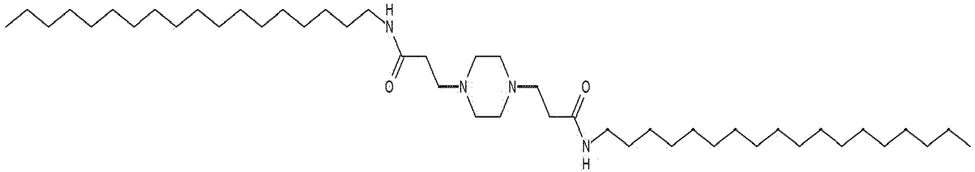

I. Under the protection of nitrogen, methyl acrylate (1.3 mmol to 3.5 mmol) was added into a reaction flask, and then stirred, 1 part to 3 parts of methanol, or ethanol, or ethyl acetate, or dichloroethane, or acetone, or acetonitrile, or DMF was added, or no solvent was added, and 200 ppm to 600 ppm 4,4'-diphenol hydroxy dibenzophenone or m-nitrophenol was added. The temperature was reduced to 15°C to 20°C, and then piperazine (0.90 mmol to 1.05 mmol) was dropwise added slowly. The temperature was raised to a room temperature after dropwise adding, then the reagent was stirred for 3 hours to 5 hours, and heated to 40°C to 60°C to continue the reaction for 15 hours to 24 hours. The reaction process was monitored by TLC until the reaction was complete, excessive methyl acrylate was removed under vacuum, and the remaining reaction intermediate without further purification was used for next reaction.
II. Under the protection of nitrogen and stirring, octadecylamine (2.0 mmol to 2.05 mmol) was added in batched first into the intermediate obtained in the reaction of the first step at the room temperature, and then 0.1% to 0.5% of sodium methoxide or sodium formate was added, or no catalyst was added. After charging, the temperature was raised to 50°C to 60°C for reaction for 6 hours to 8 hours, and continuously raised to 80°C to 120°C for reaction for 48 hours to 72 hours, and the reaction process was monitored by TLC until the reaction was complete. The catalyst was removed, ethanol or methanol containing 5% to 10% of water was added for recrystallizing, and filtered to yield a white powder solid product, and dried, wherein a yield was 91%.

### Synthesis method of example structure B:

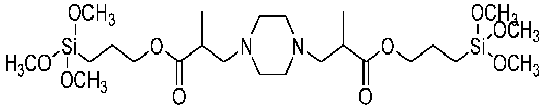

I. Under the protection of nitrogen, 3-(trimethoxysilyl)propyl acrylate (2.03 mmol to 2.5 mmol) was added into a reaction flask, and then stirred, 1 part to 3 parts of methanol or ethanol was added, or no solvent was added, and 100 ppm to 700 ppm 4,4'-diphenol hydroxy dibenzophenone, or m-nitrophenol, or o-methoxyhydroquinone was added. The temperature was reduced to 20°C, and then piperazine (0.95 mmol to 1.0 mmol) was dropwise added slowly. The temperature was raised to a room temperature after dropwise adding, then the reagent was stirred for 5 hours to 7 hours, and heated to 40°C to 60°C to continue the reaction for 24 hours to 48 hours. The reaction process was monitored by TLC until the reaction was complete, and excessive 3-(trimethoxysilyl)propyl acrylate was removed under vacuum to yield an oily product, wherein a yield was 93%.

### (VI) Synthesis method of novel light stabilizer with structural general formula 6

### (1) Synthesis route of novel light stabilizer with structural general formula 6

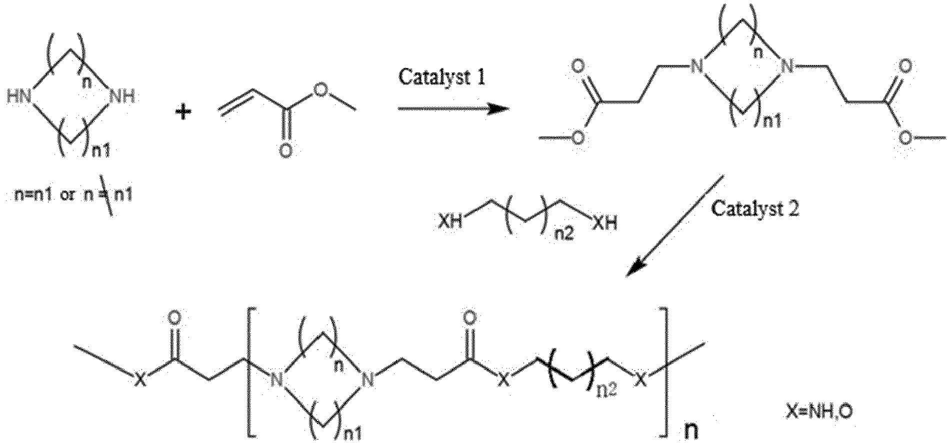

### (2) General synthesis method of structural general formula 6

I. Under the protection of nitrogen, methyl acrylate or methyl methacrylate (1.5 mmol to 3.5 mmol) was added into a reaction flask, and then stirred, 1 part to 3 parts of methanol, or ethanol, or ethyl acetate, or dichloroethane, or acetone, or acetonitrile, or DMF was added, or no solvent was added, and then 100 ppm to 1000 ppm o-methoxyhydroquinone, or 4,4-diphenol hydroxy dibenzophenone, or m-nitrophenol was added. The temperature was reduced to 10°C to 20°C, and then alkyl cyclic diamine (0.90 mmol to 1.15 mmol) was dropwise added slowly. The temperature was raised to a room temperature after dropwise adding, then the reagent was stirred for 5 hours to 8 hours, and heated to 45°C to 70°C to continue the reaction for 10 hours to 24 hours. The reaction process was monitored by TLC until the reaction was complete, excessive methyl acrylate was removed under vacuum, and the remaining reaction intermediate without further purification was used for next reaction.
II. Under the protection of nitrogen and stirring, pentanediamine, or hexamethylenediamine, or decanediamine, or hexanediol, or octanediol, or decanediol (1.95 mmol to 2.20 mmol) was added in batched first into the intermediate obtained in the reaction of the first step at the room temperature, and then 0.01% to 5% of sodium methoxide or sodium formate was added, or no catalyst or solvent diethyltin oxide or aluminum alkoxide was added. After charging, the temperature was raised to 50°C to 70°C for reaction for 5 hours to 8 hours, and continuously raised to 80°C to 130°C for reaction for 48 hours to 96 hours, and the reaction process was monitored by TLC until the reaction was complete. The catalyst was removed, ethanol or methanol containing 5% to 10% of water was added for recrystallizing, and filtered to yield a white powder solid product, and dried, wherein a yield ranged from 83% to 91%.

### (3) Synthesis method of product example with structural general formula 6

### Synthesis method of example structure A:

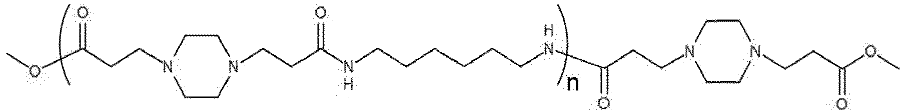

I. Under the protection of nitrogen, methyl acrylate (1.5 mmol to 3.5 mmol) was added into a reaction flask, and then stirred, 1 part to 3 parts of methanol, or ethanol, or ethyl acetate, or dichloroethane, or acetone, or acetonitrile, or DMF was added, or no solvent was added, and 200 ppm to 700 ppm o-methoxyhydroquinone, or 4,4-diphenol hydroxy dibenzophenone, or m-nitrophenol was added. The temperature was reduced to 15°C to 20°C, and then piperazine (0.97 mmol to 1.05 mmol) was dropwise added slowly. The temperature was raised to a room temperature after dropwise adding, then the reagent was stirred for 5 hours to 8 hours, and heated to 45°C to 60°C to continue the reaction for 15 hours to 24 hours. The reaction process was monitored by TLC until the reaction was complete, excessive methyl acrylate was removed under vacuum, and the remaining reaction intermediate without further purification was used for next reaction.
II. Under the protection of nitrogen and stirring, propylene diamine, or pentanediamine, or hexamethylenediamine, or decanediamine (0.95 mmol to 1.02 mmol) was added in batched first into the intermediate obtained in the reaction of the first step at the room temperature, and then 0.01% to 5% of sodium methoxide or sodium formate was added, or no catalyst was added. After charging, the temperature was raised to 50°C to 60°C for reaction for 5 hours to 6 hours, and continuously raised to 80°C to 110°C for reaction for 48 hours to 96 hours, and the reaction process was monitored by TLC until the reaction was complete. The catalyst was removed, ethanol or methanol containing 5% to 10% of water was added for recrystallizing, and filtered to yield a white powder solid product, and dried, wherein a yield was 91%.
   m.p.

### Synthesis method of example structure B:

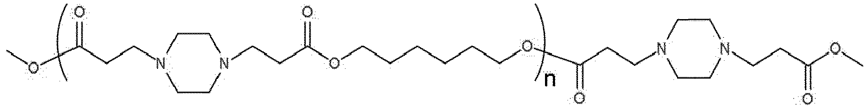

I. Under the protection of nitrogen, methyl acrylate (1.8 mmol to 3.5 mmol) was added into a reaction flask, and then stirred, 1 part to 3 parts of methanol, or ethanol, or ethyl acetate, or dichloroethane, or acetone, or acetonitrile, or DMF was added, or no solvent was added, and 300 ppm to 700 ppm 4,4-diphenol hydroxy dibenzophenone or m-nitrophenol was added. The temperature was reduced to 18°C to 20°C, and then piperazine (0.97 mmol to 1.05 mmol) was dropwise added slowly. The temperature was raised to a room temperature after dropwise adding, then the reagent was stirred for 5 hours to 6 hours, and heated to 45°C to 60°C to continue the reaction for 18 hours to 24 hours. The reaction process was monitored by TLC until the reaction was complete, excessive methyl acrylate was removed under vacuum, and the remaining reaction intermediate without further purification was used for next reaction.
II. Under the protection of nitrogen and stirring, propylene glycol, or pentanediol, or hexanediol, or decanediol (0.95 mmol to 1.02 mmol) was added in batched first into the intermediate obtained in the reaction of the first step at the room temperature, and then diethyltin oxide and aluminum alkoxide (3:1) were added as a catalyst. After charging, the temperature was raised to 80°C to 90°C for reaction for 5 hours to 6 hours, generated methanol micromolecules were removed by a negative pressure, and the temperature was continuously raised to 90°C to 130°C for reaction for 48 hours to 96 hours, and the reaction process was monitored by TLC until the reaction was complete. The catalyst was removed, ethanol or methanol containing 5% to 10% of water was added for recrystallizing, and filtered to yield a white powder solid product, and dried, wherein a yield was 87.7%.
   m.p.

### (VII) Synthesis method of novel light stabilizer with structural general formula 7

### (1) Synthesis route of novel light stabilizer with structural general formula 7

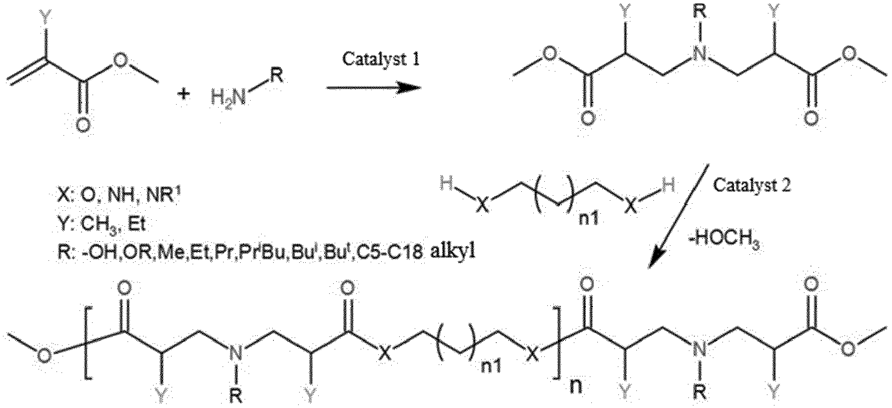

### (2) General synthesis method of structural general formula 7

I. Under the protection of nitrogen and stirring, alkylamine, or aryl substituted alkylamine, or hydroxylamine, or alkoxyamine, or aryl substituted alkoxyamine (0.90 mmol to 1.15 mmol) was added into a reaction flask, then 1 part to 3 parts of methanol, or ethanol, or ethyl acetate, or dichloroethane, or acetone, or acetonitrile, or DMF was added, or no solvent was added, and then 100 ppm to 1000 ppm o-methoxyhydroquinone, or 4,4-diphenol hydroxy dibenzophenone or m-nitrophenol, or 0.1% to 5% of NaOH or K2CO3, or 10% to 30% of silica gel or acidic alumina was added, or no catalyst was added. The temperature was reduced to 5°C to 10°C, and then methyl acrylate or methyl methacrylate (2.1 mmol to 4.5 mmol) was dropwise added slowly. The temperature was raised to a room temperature after dropwise adding, then the reagent was stirred for 5 hours to 18 hours, and heated to 30°C to 70°C to continue the reaction for 5 hours to 24 hours. The reaction process was monitored by TLC until the reaction was complete, excessive methyl acrylate, and solvent and catalyst were removed under vacuum, and the remaining reaction intermediate without further purification was used for next reaction.
II. Under the protection of nitrogen and stirring, pentanediamine, or hexamethylenediamine, or decanediamine, or hexanediol, or octanediol, or decanediol (1.95 mmol to 2.20 mmol) was added in batched first into the intermediate obtained in the reaction of the first step at the room temperature, and then 0.01% to 5% of sodium methoxide or sodium formate was added, or no catalyst or solvent diethyltin oxide or aluminum alkoxide was added. After charging, the temperature was raised to 50°C to 70°C for reaction for 10 hours to 18 hours, and continuously raised to 80°C to 140°C for reaction for 24 hours to 96 hours, and the reaction process was monitored by TLC until the reaction was complete. The catalyst was removed, ethanol, or methanol, or ethyl acetate, or petroleum ether containing 5% to 10% of water was added for recrystallizing, filtered to yield a white powder solid product, and dried, wherein a yield ranged from 80% to 90%.

### (3) Synthesis method of product example with structural general formula 7

### Synthesis method of example structure A:

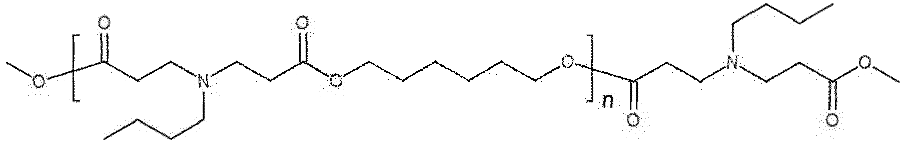

I. Under the protection of nitrogen, n-butylamine (1.5 mmol to 3.5 mmol) was added into a reaction flask, and then stirred, 1 part to 3 parts of methanol, or ethanol, or ethyl acetate, or dichloroethane, or acetone, or acetonitrile, or DMF was added, or no solvent was added, and 100 ppm to 700 ppm o-methoxyhydroquinone, or 4,4-diphenol hydroxy dibenzophenone, or m-nitrophenol was added, or no catalyst was added. The temperature was reduced to 10°C to 20°C, and then methyl acrylate (2.1 mmol to 4.0 mmol) was dropwise added slowly. The temperature was raised to a room temperature after dropwise adding, then the reagent was stirred for 10 hours to 18 hours, and heated to 35°C to 60°C to continue the reaction for 15 hours to 24 hours. The reaction process was monitored by TLC until the reaction was complete, excessive methyl acrylate was removed under vacuum, and the remaining reaction intermediate without further purification was used for next reaction.
II. Under the protection of nitrogen and stirring, hexanediol (0.95 mmol to 1.02 mmol) was added in batched first into the intermediate obtained in the reaction of the first step at the room temperature, and then dibutyltin oxide or tetrabutoxy aluminum was added. After charging, the temperature was raised to 50°C to 80°C for reaction for 8 hours to 12 hours, and continuously raised to 90°C to 150°C for reaction for 24 hours to 96 hours, and the reaction process was monitored by TLC until the reaction was complete. The catalyst was removed, ethanol, or methanol, or ethanol, or methyl butyl ether, or petroleum ether containing 5% to 10% of water was added for recrystallizing, filtered to yield a white powder solid product, and dried, wherein a yield was 90.6%.

### Synthesis method of example structure B:

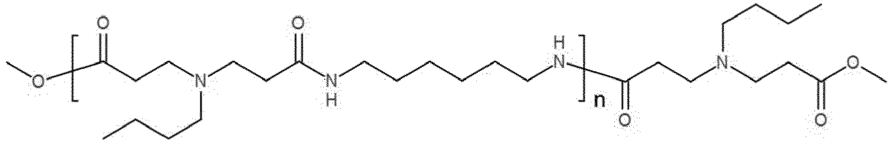

I. Under the protection of nitrogen, n-butylamine (1.5 mmol to 3.5 mmol) was added into a reaction flask, and then stirred, 1 part to 3 parts of methanol, or ethanol, or ethyl acetate, or dichloroethane, or acetone, or acetonitrile, or DMF was added, or no solvent was added, and 100 ppm to 700 ppm o-methoxyhydroquinone, or 4,4-diphenol hydroxy dibenzophenone, or m-nitrophenol was added, or no catalyst was added. The temperature was reduced to 10°C to 20°C, and then methyl acrylate (2.1 mmol to 4.0 mmol) was dropwise added slowly. The temperature was raised to a room temperature after dropwise adding, then the reagent was stirred for 10 hours to 18 hours, and heated to 35°C to 60°C to continue the reaction for 15 hours to 24 hours. The reaction process was monitored by TLC until the reaction was complete, excessive methyl acrylate was removed under vacuum, and the remaining reaction intermediate without further purification was used for next reaction.
II. Under the protection of nitrogen and stirring, hexanediamine (0.95 mmol to 1.02 mmol) was added in batched first into the intermediate obtained in the reaction of the first step at the room temperature, and then sodium formate or sodium methoxide was added. After charging, the temperature was raised to 60°C to 80°C for reaction for 18 hours to 24 hours, and continuously raised to 90°C to 130°C for reaction for 48 hours to 96 hours, and the reaction process was monitored by TLC until the reaction was complete. The catalyst was removed, ethanol, or methanol, or ethanol, or methyl butyl ether, or petroleum ether containing 5% to 10% of water was added for recrystallizing, filtered to yield a white powder solid product, and dried, wherein a yield was 89.3%.

### Synthesis method of example structure C:

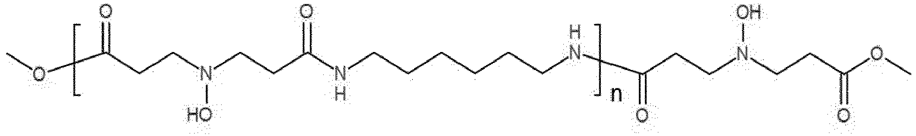

I. Under the protection of nitrogen, hydroxylamine hydrochloride or hydroxylamine sulfate (1.1 mmol to 1.5 mmol) was added into a reaction flask, and stirred, then 1 part to 3 parts of methanol solution or ethanol solution (2:1) was added, then sodium hydroxide or potassium carbonate (1.2 mmol to 2 mmol) was added at 5°C to 15°C, and then methyl acrylate (2.1 mmol to 4.0 mmol) was dropwise added slowly. The temperature was raised to a room temperature after dropwise adding, and then the reagent was stirred for 15 hours to 24 hours. The reaction process was monitored by TLC until the reaction was complete, ethyl acetate, or dichloromethane, or methyl butyl ether was added for extracting a target intermediate, and dried, excessive methyl acrylate and solvent were removed under vacuum, and the remaining reaction intermediate without further purification was used for next reaction.
II. Under the protection of nitrogen and stirring, hexanediamine (0.95 mmol to 1.02 mmol) was added in batched first into the intermediate obtained in the reaction of the first step at the room temperature, and 0.1% to 2% of sodium formate or sodium methoxide was added. After charging, the temperature was raised to 50°C to 70°C for reaction for 15 hours to 24 hours, and continuously raised to 70°C to 130°C for reaction for 72 hours to 96 hours, and the reaction process was monitored by TLC until the reaction was complete. The catalyst was removed, ethanol, or methanol, or ethanol, or methyl butyl ether, or petroleum ether containing 5% to 10% of water was added for recrystallizing, filtered to yield a white powder solid product, and dried, wherein a yield was 92.5%.

### Synthesis method of example structure D:

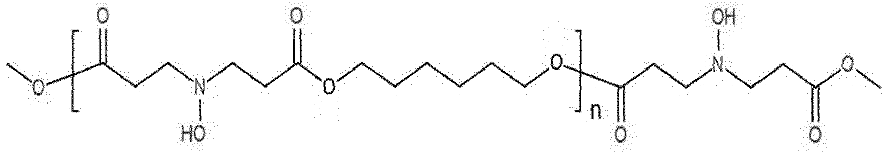

### Reaction of first step:

Under the protection of nitrogen and stirring, hydroxylamine hydrochloride or hydroxylamine sulfate (1.1 mmol to 1.5 mmol) was added into a reaction flask, and stirred, then 1 part to 3 parts of methanol solution or ethanol solution (2:1), or ethyl acetate, or dichloroethane was added, or no solvent was added, then sodium hydroxide or potassium carbonate (1.2 mmol to 2 mmol) or triethylamine was added at 5°C to 15°C, and then methyl acrylate (2.0 mmol to 4.0 mmol) was dropwise added slowly. The temperature was raised to a room temperature after dropwise adding, and then the reagent was stirred for 15 hours to 24 hours. The reaction process was monitored by TLC until the reaction was complete, ethyl acetate, or dichloromethane, or methyl butyl ether was added for extracting a target intermediate, and dried, excessive methyl acrylate and solvent were removed under vacuum, and the remaining reaction intermediate without further purification was used for next reaction.

### Reaction of second step:

Under the protection of nitrogen and stirring, hexanediol (0.98 mmol to 1.3 mmol) was added in batched first into the intermediate obtained in the reaction of the first step at the room temperature, and 0.1% to 5% of trioctyloxy aluminum and dibutyltin oxide were added. Then, the temperature was raised to 70°C to 90°C for reaction for 15 hours to 24 hours, and continuously raised to 100°C to 150°C for reaction for 48 hours to 96 hours, and the reaction process was monitored by TLC until the reaction was complete. The catalyst was removed, ethanol, or methanol, or ethanol, or methyl butyl ether, or petroleum ether containing 5% to 10% of water was added for recrystallizing, filtered to yield a white powder solid product, and dried, wherein a yield was 90.1%.

Thermal aging property tests and UVB and 300 W ultraviolet light aging property tests of the innovative light stabilizer of the patent in different polymer materials, and comparison tests with light stabilizers of common brands in the international market were carried out, and these tests were completed in PP, ABS and PC polymer materials respectively.

### (1) Comparison test of thermal aging and ultraviolet light aging properties of steric hindrance adjustable light stabilizer in PP-T20

### a. Twin-screw machining, extrusion and granulation

Granulation was completed by extrusion with a twin-screw extruder (Nanjing Keya AK36):

### Parameters of extruder:

Temperatures of first to tenth regions (°C): 160, 190, 210, 220, 220, 220, 210, 210, 210, and 200
Rotating speed: 300 rpm 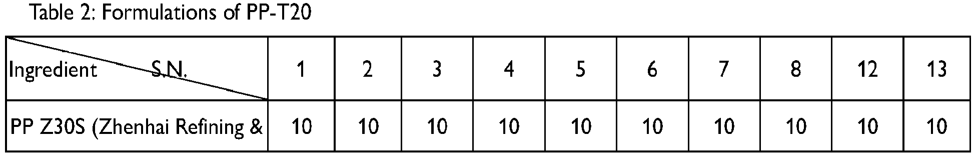 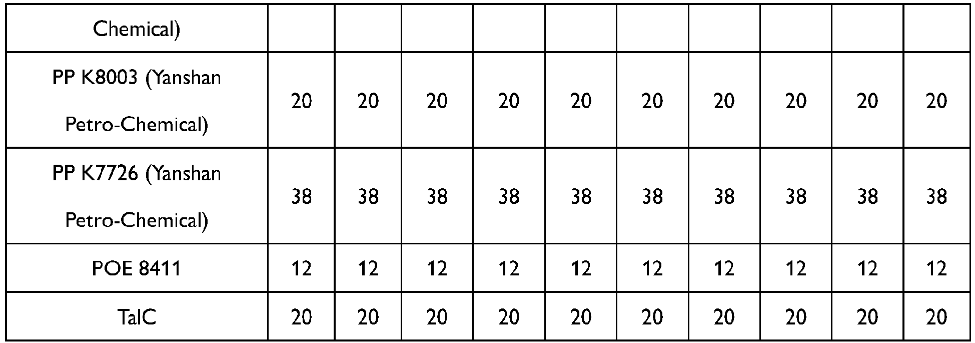
Main antioxidant: 0.2%; (See FIG. 1 for grades)
Light stabilizer: 0.1%; (See FIG. 1 for grades)

### b. Preparation of PP-T20 splines

The PP-T20 splines were completed with Haitian injection molding machine SA1200 device.

### Machining parameters of injection molding machine:

Temperatures of first to fifth segments (°C): 200, 210, 210, 205, and 190;
Injection molding pressure: 58 bar

### c. Thermal aging test in oven at 150°C

The thermal aging in oven was completed in a thermal aging oven according to a GB/T 7141-2008 plastic thermal aging test method.

### d. UVB light aging test

The UVB light aging test was completed in an UVB ultraviolet light aging test box (Q-Lab Suzhou Guangjun) according to a general principle in a first part of a GB/T 16422.1-2006 plastic laboratory light source exposure test method and a fluorescent ultraviolet lamp principle in a third part of a GB/T 16422.3-2014 plastic laboratory light source exposure test method.

### e. Test results of thermal aging in oven at 150°C and UVB ultraviolet light aging (FIG. 1)

### f. Result discussion

It can be seen from the results of the thermal aging in oven at 150°C of the PP-T20 splines that colors of a B1 (3853) spline, a B2 (770) spline and a B3 (622) spline of products in the international market are obviously darker than those of splines (B4 to B13) added with products of the patent, wherein the spline (B2) added with the light stabilizer 770 has a deepest color, the spline added with the light stabilizer 622 has a lighter color, and the spline (B1) added with the light stabilizer 3853 has a lightest color in products of the three major international companies. In comparison, the colors of the splines (B4 to B13) added with the light stabilizers of the patent are lighter than those of the splines added with the light stabilizers in the international market.

It can be seen from the results of the VUB ultraviolet light aging test that B7 and B13 splines have a lightest color, B8 and B12 splines have a similar whiteness to those added with 3853 (B1), 770 (B2) and 622 (B3), and B4, B5 and B6 splines have a darker color.

The steric hindrance adjustable weak base light stabilizer of the patent for invention has better compatibility and matching with the polymer materials. Therefore, the steric hindrance adjustable weak base light stabilizer can not only provide better light stability protection, but also play a better role in thermal aging resistance and yellowing resistance.

### Results of mechanical property test

**Table 3: Comparison test of tensile properties of innovative light stabilizers of Rycom in PP-T20**

| (Comparison samples: 3853, 770, and 622) | | | | | | | |
|---|---|---|---|---|---|---|---|
| Batch number: PPT20 RK-PT-T20201014 | | | | | | | |
| Aging at 150°C | 0 hour constant temperature and humidity 48 hours | 288 hours constant temperature and humidity 48 hours | Thermal aging in oven at 150°C for 288 hours | | UVB ultraviolet aging for 207 hours | UVB ultraviolet aging for 207 hours | |
| Serial No. | Tensile/MPa | Tensile/MPa | Change rate/MPa | Retention | Tensile/MPa | Change rate/MPa | Retention |
| 1 3853 | 20.8 | 19.9 | -0.9 | 0.96 | 19.3 | -1.5 | 0.93 |
| 2 770 | 20.8 | 20.6 | -0.5 | 0.98 | 19.6 | -1.5 | 0.93 |
| 3 622 | 21.0 | 20.7 | -0.7 | 0.97 | 20.2 | -1.2 | 0.95 |
| 4 RK-AB75S | 20.8 | 20.6 | -0.4 | 0.98 | 19.6 | -1.2 | 0.95 |
| 5 RK-AB76S | 20.7 | 20.8 | -0.2 | 0.99 | 19.4 | -1.3 | 0.94 |
| 6 RK-AB77 | 20.9 | 20.7 | -0.2 | 0.99 | 19.8 | -1.1 | 0.96 |
| 7 RK-ABUV721 | 20.3 | 20.8 | 0.5 | 1.02 | 19.1 | -1.2 | 0.94 |
| 8 RK-AB71L | 20.8 | 20.9 | 0.1 | 1.00 | 19.3 | -1.5 | 0.93 |
| 9 RK-AB78 | 20.9 | 20.6 | -0.3 | 0.98 | 19.6 | -1.3 | 0.95 |
| 10 RK-AB79 | 20.9 | 20.7 | -0.4 | 0.98 | 19.7 | -1.5 | 0.96 |

| Serial No. | Modulus/MPa | Modulus/MPa | Change value/MPa | Retention | Modulus/MPa | Change value/MPa | Retention |
|---|---|---|---|---|---|---|---|
| 1 3853 | 2373 | 2240 | -133 | 0.94 | 2720 | 347 | 1.15 |
| 2 770 | 2677 | 2390 | -287 | 0.89 | 3770 | 1093 | 1.41 |
| 3 622 | 2503 | 2207 | -297 | 0.88 | 1600 | -903 | 0.64 |
| 4 RK-AB75S | 2037 | 2020 | -17 | 0.99 | 2580 | 543 | 1.27 |
| 5 RK-AB76S | 2455 | 2440 | -15 | 0.99 | 2820 | 365 | 1.15 |
| 6 RK-AB77 | 2340 | 2620 | 280 | 1.12 | 2350 | 10 | 1.03 |
| 7 RK-ABUV721 | 2490 | 2397 | -93 | 0.96 | 1710 | -780 | 0.69 |
| 8 RK-AB71L | 2380 | 2260 | -120 | 0.95 | 2210 | -160 | 0.91 |
| 9 RK-AB78 | 2823 | 2630 | -193 | 0.93 | 2230 | -593 | 0.79 |
| 10 RK-AB79 | 2397 | 2440 | 43 | 1.02 | 2399 | 20 | 1.01 |

| Aging at 150°C | 0 hour constant temperature and humidity 48 hours | 288 hours constant temperature and humidity 48 hours | Aging at 150°C for 288 hours | | UVB ultraviolet aging for 207 hours | UVB ultraviolet aging for 207 hours | |
|---|---|---|---|---|---|---|---|
| Serial number | Elongation/% | Elongation/% | Change value/% | Retention | Elongation/% | Change value/% | Retention |
| 1 3853 | 3.1 | 2.9 | -0.2 | 0.94 | 2.1 | -1.0 | 0.68 |
| 2 770 | 3.0 | 2.8 | -0.2 | 0.94 | 1.5 | -1.5 | 0.51 |
| 3 622 | 2.5 | 2.9 | 0.3 | 1.13 | 1.6 | -0.9 | 0.63 |
| 4 RK-AB75S | 2.5 | 2.4 | -0.1 | 0.97 | 2.3 | -0.2 | 0.92 |
| 5 RK-AB76S | 2.6 | 2.8 | 0.2 | 1.06 | 2.0 | -0.6 | 0.77 |
| 6 RK-AB77 | 2.9 | 2.5 | -0.4 | 0.86 | 2.0 | -0.9 | 0.70 |
| 7 RK-ABUV721 | 2.8 | 2.8 | 0.0 | 1.01 | 1.9 | -0.9 | 0.69 |
| 8 RK-AB71L | 3.0 | 2.9 | -0.2 | 0.95 | 2.1 | -0.9 | 0.69 |
| 9 RK-AB78 | 3.0 | 2.3 | -0.7 | 0.76 | 2.2 | -0.8 | 0.73 |
| 10 RK-AB79 | 3.2 | 2.7 | -0.6 | 0.82 | 2.3 | -0.9 | 0.71 |

### Test condition:

Laboratory environment: temperature: 23°C, humidity: 45% RH; and state adjustment: 23°C, and 50% RH;
Executive standard: GB/T1040.2-2006/ISO 527-2: 1993;
Tensile speed: 50 mm/min
Note: the light stabilizer having an RK grade with a mantissa followed by an English letter L is a liquid antioxidant.

According to mechanical tensile data, the results of the mechanical tensile property test of the patent in Table 1 are compared with those of the products of the major international companies in the market, and the light stabilizers of some grades show an outstanding property in maintaining the mechanical property.

According to the results of comparing tensile strengths before and after thermal and light aging of the light stabilizers of some grades of the patent with those of the optimal light stabilizers 3853, 770 and 622 in PP in the market, the light stabilizers of some grades of the patent show less influence of a change in mechanical property during parallel aging, for example, tensile retention rates of RK-AB75S, RK-AB-76S, RK-AB77, RK-ABUV721, and RK-AB71L are excellent after thermal aging for 288 hours and light aging for 207 hours. According to the data, there is no obvious aging influence, and the tensile retention rates basically have no obvious change. According to the results of thermal aging, the retention rates are better than those of comparison standard samples.

In terms of moduli before and after aging and tensile elongations, the light stabilizer products of the patent show slightly better results.

### (2) Comparison test of thermal aging and ultraviolet light aging properties of steric hindrance adjustable light stabilizer of the patent in ABS

a. Twin-screw machining, extrusion and granulation

Granulation was completed by extrusion with a twin-screw extruder (Nanjing Keya AK36):

### Parameters of extruder:

Temperatures of first to tenth regions: 200, 205, 215, 215, 215, 215, 210, 210, 205, 200
Rotating speed: 300 m/s
Main antioxidant: 0.2%; light stabilizer: 0.1%
Standard comparison samples: C1 spline: 3808, C2 spline: 770, and C3 spline: 622.

### b. Preparation of ABS splines

The ABS splines were completed with Haitian injection molding machine SA1200 device.

### Machining parameters of injection molding machine:

Temperatures of first to fifth segments: 205, 220, 220, 215, and 200;
Injection molding pressure: 62 bar

### c. Thermal aging test in oven at 110°C

The thermal aging of all ABS splines was completed in a thermal aging oven according to a GB/T 7141-2008 plastic thermal aging test method at 110°C.

### d. UVB light aging test

The UVB light aging test was completed in an UVB ultraviolet light aging test box (Q-Lab Suzhou Guangjun) according to a general principle in a first part of a GB/T 16422.1-2006 plastic laboratory light source exposure test method and a fluorescent ultraviolet lamp principle in a third part of a GB/T 16422.3-2014 plastic laboratory light source exposure test method.

### e. Test results of thermal aging at 110°C and UVB light aging of ABS splines (FIG. 2)

### f. Result discussion

ABS is resin sensitive to yellowing caused by light aging. According to the test results of thermal aging in oven at 110°C and UVB ultraviolet light aging of the ABS splines, a spline C7 added with the innovative light stabilizer with the grade RK-AB79 of the patent and having a main antioxidant RK-701 has the best yellowing resistance, followed by a spline C4 (added with the innovative light stabilizer RK-AB65S in the patent), and then followed by splines C9, C8 and C5. Compared with splines for the comparison test, these splines show obviously better thermal aging resistance and yellowing resistance. On the whole, the steric hindrance adjustable weak base antioxidant of the patent can still show obvious property advantages of thermal yellowing resistance and light-induced yellowing resistance in ABS sensitive to yellowing.

### (3) Comparison test of thermal aging and UVB ultraviolet light aging properties of the steric hindrance adjustable light stabilizer of the patent in PC

### Preparation of sample plates

PC resin material: PC2805 Shanghai Covestro

PC sample plates were prepared with Haitian injection molding machine SA1200 device

### Machining parameters of PC sample plates

PC sample plates were prepared with Haitian injection molding machine SA1200 device
Machining parameters: temperatures of first to fifth segments (°C) 266, 273, 273, 268, and 265
Pressure: 90 bar
Speed: 44 rpm

### Aging test of PC sample plates

### (I) Test results of thermal aging in oven at 150°C

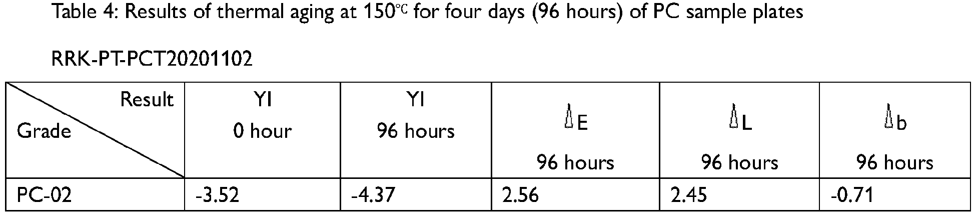

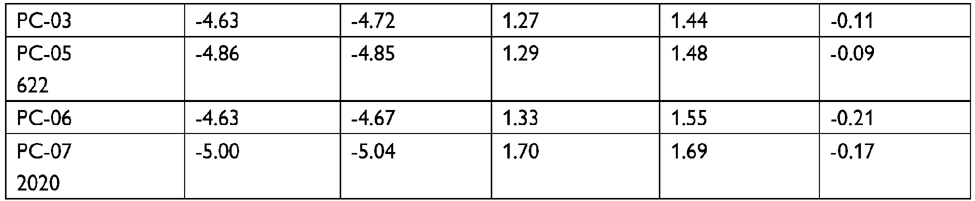

### (II) UVB ultraviolet light aging test

### Results 1 of UVB ultraviolet light aging at 70°C for 47 hours of PC sample plates (FIG. 3)

Yellowing degrees of the PC sample plates after light aging were sorted as follows:
C2(PC-03) & C1(PC-02) < C3(622) < C5(PC-06) < C4(2020)

### Results 2 of UVB ultraviolet light aging at 70°C for 17 hours of PC sample plates (FIG. 4)

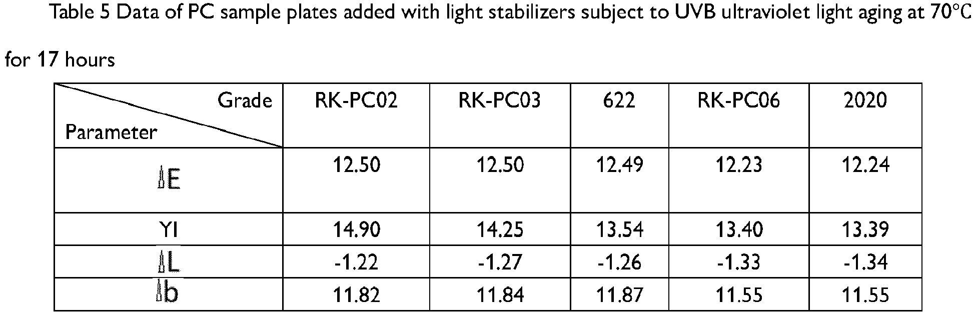

### Results 3 of ultraviolet light aging at 70°C of PC sample plates (FIG. 5)

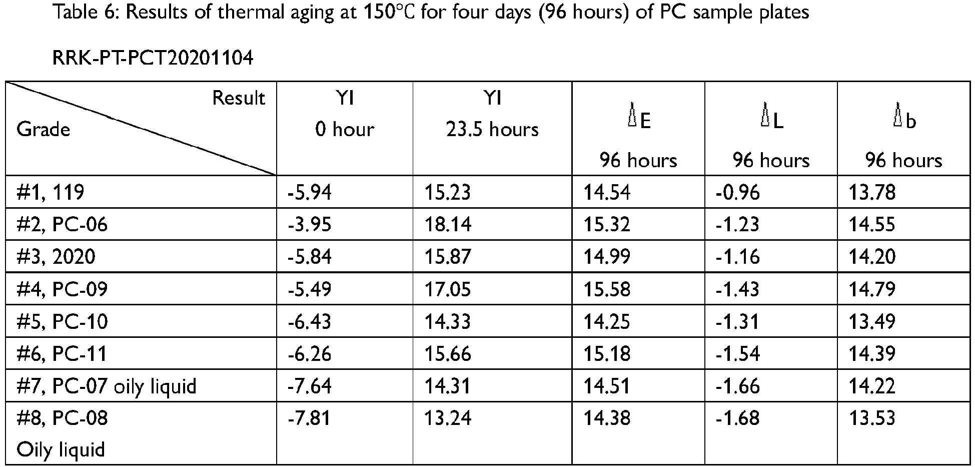

### Data results in Table 6:

Color difference: PC-10 < PC-08 < PC-07 < UV119 < UV2020 < PC-11 < PC-06 < PC-09
YI: 13.24 (PC-08) < 14.31 (PC-07) < 14.33 (PC-10) < 15.23 (UV119) < 15.66 (PC-11) < 15.87 (UV2020) < 17.05 (PC-09) < 18.14 (PC-06)

### Result discussion

Polycarbonate (PC) is the most sensitive material to light-induced yellowing. During thermal aging in oven at 150°C for 4 days (96 hours) of the above PC sample plates (see Tables 4 to 6), the color differences all range from 1 to 2.6, and are no more than 3. However, the test results of UVB light aging for 4 days (96 hours) show that the color differences are obviously larger, and all range from 14 to 16. Compared with the international brands UV119 and UV2020, the light stabilizers with grades PC-10, PC-08 and PC-07 of the patent all show better light-induced yellowing resistance. It can also be seen from visual color comparison of the sample plates before and after aging in FIG. 8 that the PC sample plates of the light stabilizers with the three grades have a lighter color after light aging.

On the whole, the steric hindrance adjustable structural light stabilizer of the patent has great advantages in structural steric hindrance adjustment, an electronegative environment around the nitrogen atoms may also be adjusted, and the production process is green and easy to operate, thus providing a variety of selective products capable of reducing a cost for polymer weather-resistant products.

## Claims

1. A structure of a steric hindrance adjustable weak base light stabilizer, wherein structural formulas are as follows:
structural general formula 1: 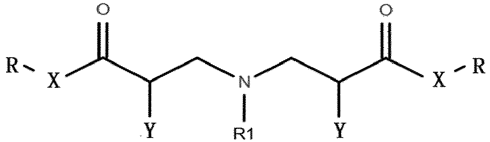
wherein, in the structural general formula 1, X is NH, NR3 or O;
Y is H, methyl or other alkyl;
R is 5-22 linear alkyl, branched alkyl, or -(CH₂)nSi(OMe)₃, or -(CH₂)nSi(OEt)₃, and n is 2, 3, 4, or 5;
R1 is C1-C20 linear alkyl, or branched alkyl, or double-bond substituted alkyl, or heteroatom substituted alkyl, or hydroxyl or alkoxy; or i-Pr, i-Bu, isoamyl, isooctyl, cyclohexyl, substituted cyclohexyl, cyclopentyl, benzyl, substituted benzyl, allyl, substituted allyl, double-bond contained alkyl, or aryl substituted alkyl, or -(CH2)n-NR4R5; R4 and R5 are methyl, ethyl, propyl, isopropyl, butyl, isobutyl, cyclohexyl, or benzyl; or 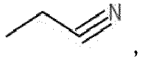 or -(CH2)n-Si(OEt)₃, and n is 2, 3, 4, or 5; and
when R1 is neither -(CH2)n-NR4R5 nor -(CH2)n-Si(OR)₃, R and R1 are the same or different;
or
structural general formula 2: 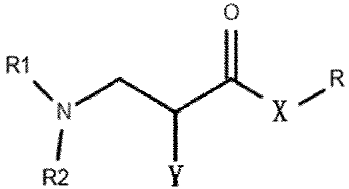
wherein, in the structural formula 2, X is NH, NR3, or O;
Y is H, methyl, ethyl, or other alkyl side chains;
R is 5-22 carbon linear or branched alkyl;
R1 and R2 are linear or branched alkyl, or i-Pr, i-Bu, isoamyl, isooctyl, cyclohexyl, substituted cyclohexyl, cyclopentyl, benzyl, substituted benzyl, allyl, substituted allyl, double-bond contained alkyl, aryl substituted alkyl, or 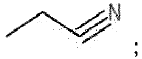 or R1 and R2 are both 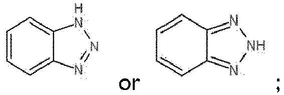
R1 and R2 are the same or different; and R is the same as or different from R1 and R2;
when R1 and R2 are different, R1 is Et, i-Pr, n-Pr, Bu, i-Bu, or C5-12 alkyl; and
R2 is
or 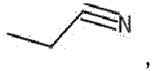 hydroxyethyl, or hydroxypropyl;
structural general formula 3: 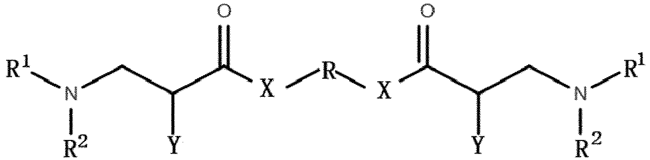
wherein, in the structural formula 3, X is NH, NR3, or O;
Y is H, methyl, ethyl, or other alkyl;
R is -(CH2)n-, wherein n ranges from 2 to 22, or R is alkyl, or aryl side chain substituted -(CH2)n-, or dibenzylamine;
R1 and R2 are alkyl, or i-Pr, i-Bu, isoamyl, isooctyl, cyclohexyl, substituted cyclohexyl, cyclopentyl, benzyl, substituted benzyl, allyl, substituted allyl, double-bond contained alkyl, aryl substituted alkyl, or 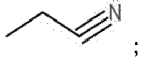 R1 and R2 are both 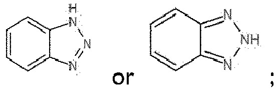
R1 and R2 are the same or different; when R1 and R2 are different, R1 is Et, i-Pr, n-Pr, Bu, i-Bu, or C5-12 alkyl; and
R2 is 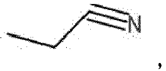 or hydroxyethyl, or hydroxypropyl;
or
structural general formula 4: 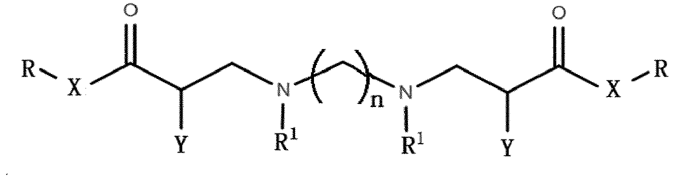
wherein, in the structural formula 4, X is NH, NR3, or O;
Y is H, methyl, ethyl, or other alkyl;
n is 2-18 linear paraffin -(CH2)n-, or side chain alkyl or aryl substituted alkane;
R1 is methyl, ethyl, propyl, or butyl; or i-Pr, i-Bu, cyclohexyl, substituted cyclohexyl, cyclopentyl, benzyl, substituted benzyl, allyl, substituted allyl, double-bond contained alkyl, or aryl substituted alkyl; or R1 is 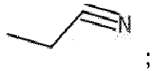
R is C5-C20 linear or branched alkyl, or alkyl side chain and aryl substituted alkyl; or -CH2CH2CH2-Si(OMe)₃, or -CH2CH2CH2-Si(OEt)₃;
or
structural general formula 5: 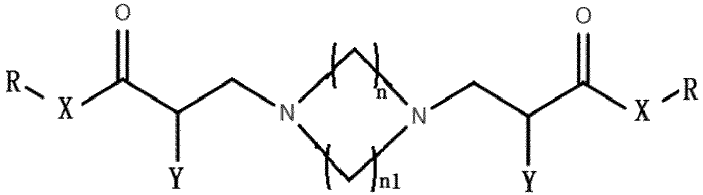
wherein, in the structural formula 5, X is NH, NR3, or O;
Y is H, methyl, or other alkyl;
n is 1, 2, 3, 4, or 5; and n1 is 1, 2, 3, 4, or 5;
n is equal to n1, or n is not equal to n1;
R is C5-C22 linear or branched alkyl, or alkyl side chain and aryl substituted alkyl; and
R is -CH2CH2CH2-Si(OMe)₃ or -CH2CH2CH2-Si(OEt)₃;
or
structural general formula 6: 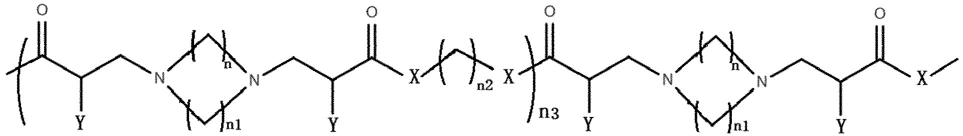
wherein, in the structural formula 6, X is NH, NR3, or O;
Y is H, methyl, ethyl, or other alkyl;
n is 1, 2, 3, 4, or 5; and n1 is 1, 2, 3, 4, or 5;
n is equal to n1, or n is not equal to n1;
n2 ranges from 2 to 18; and
n3 ranges from 2 to 35, which is a polymerization degree of an oligomer;
or
structural general formula 7: 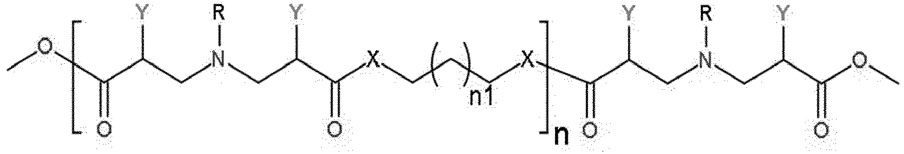
wherein, in the structural formula 7, X is NH, NR3, or O;
Y is H, methyl, ethyl, or other alkyl;
n1 is 1,2, 3, 4, 5, 6, 7, 8, 9, 10, 11, 12, 13, 14, 15, 16, 17, 18, 19, 20, 21, or 22;
n ranges from 1 to 15, which is a polymerization degree of an oligomer;
n is equal to n1, or n is not equal to n1;
R is C2-C18 linear or branched alkane; or isopropyl, isobutyl, isopentyl, isohexyl, isooctyl, or isodecyl; and
R is double-bond contained alkyl; or heteroatom contained substituent, or -OH, -OR1, ester group, carboxyl, or nitrile group.

2. A preparation method of the structure of the steric hindrance adjustable weak base light stabilizer according to claim 1, comprising the reaction formulas as follows:
structural general formula 1: 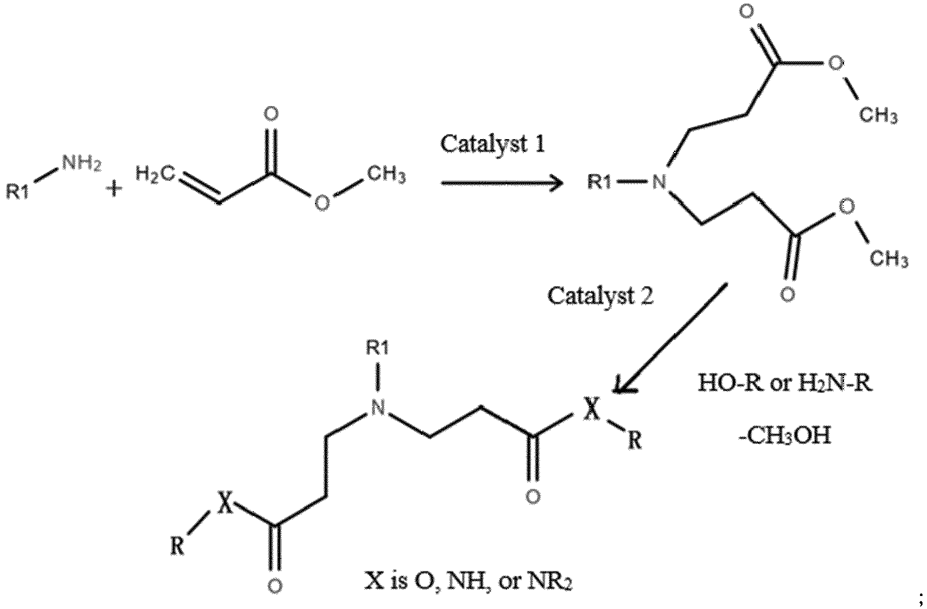
structural general formula 2: 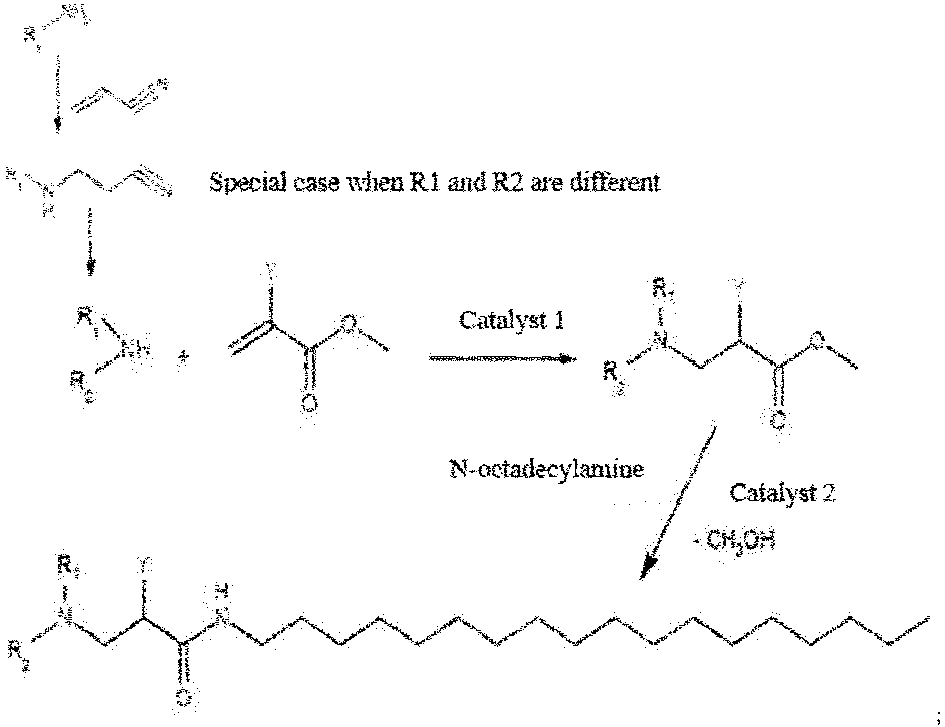
structural general formula 3: 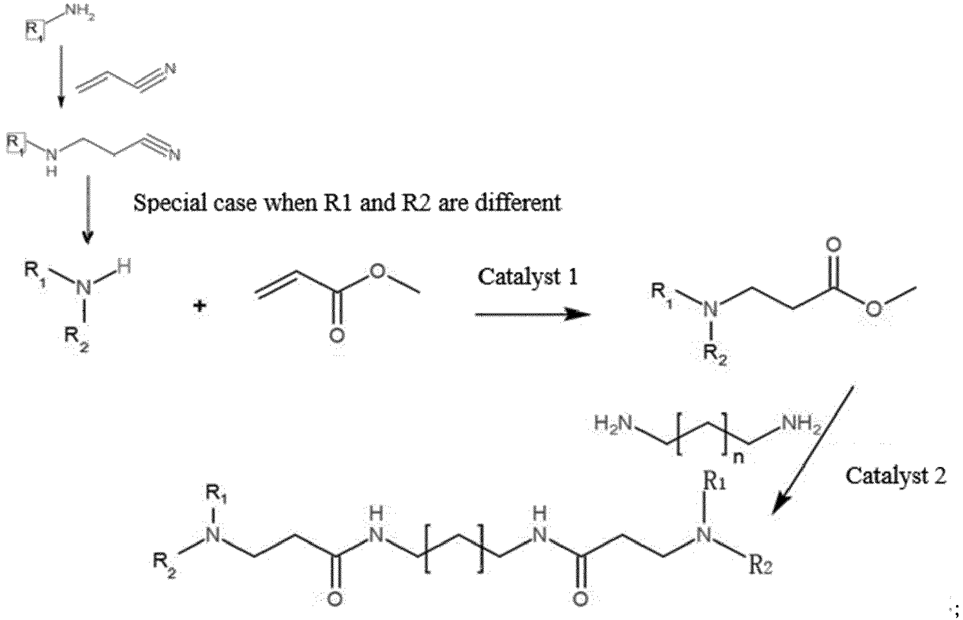
structural general formula 4: 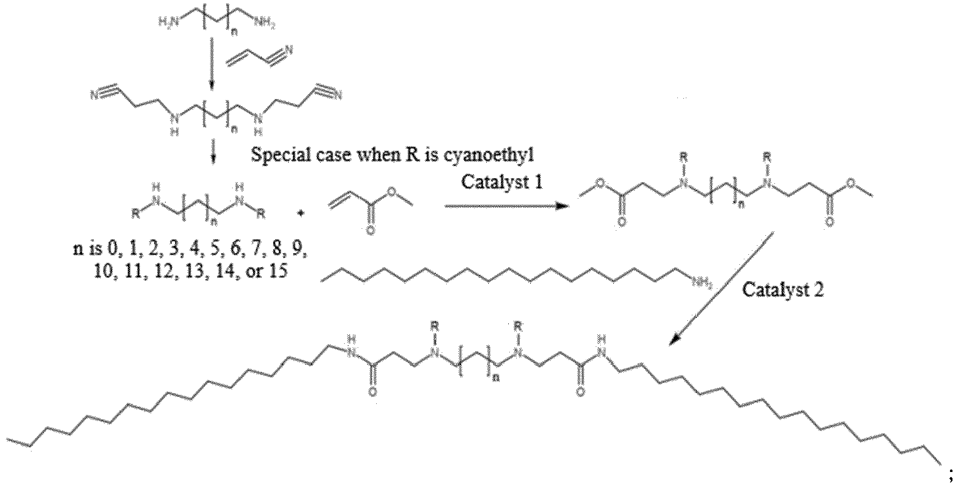
structural general formula 5: 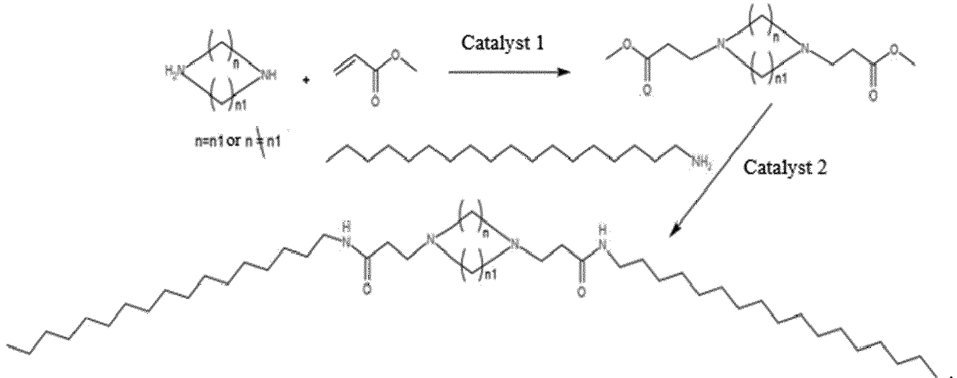
structural general formula 6: 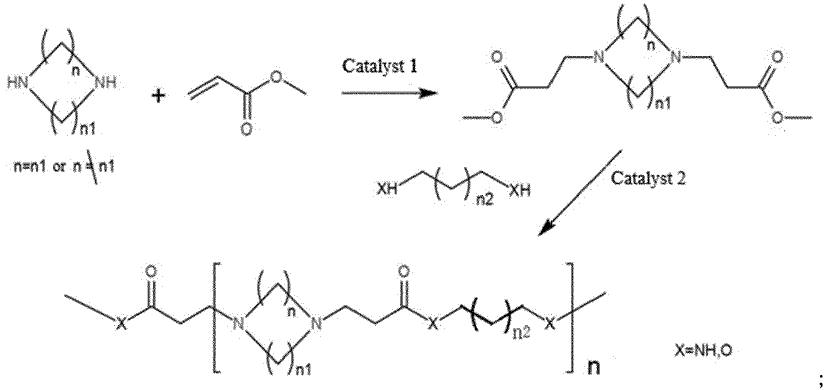
structural general formula 7:

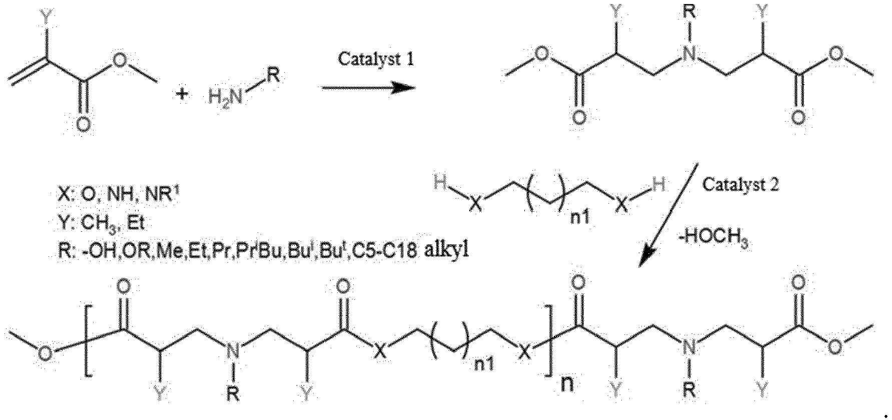

3. The preparation method of the structure of the steric hindrance adjustable weak base light stabilizer according to claim 1, comprising the specific steps as follows:
a preparation method of the structural general formula 1:
(1) under the protection of nitrogen, adding methyl acrylate or methyl methacrylate into a reaction flask, starting stirring, adding methanol, or ethanol, or acetone, or ethyl acetate, or dichloroethane, or DMF, or not adding any solvent, adding a catalyst 1, wherein the catalyst 1 is acetic acid, or acidic alumina, or silica gel, or ortho-methoxyhydroquinone, or 4,4'-benzophenone, or m-nitrophenol, or silica gel sulfate, cooling to 5°C to 10°C, and then dropwise adding first amine slowly; heating to a room temperature after dropwise adding, stirring, and heating to continue the reaction; monitoring the reaction process by TLC until the reaction is complete, removing excessive methyl acrylate under vacuum, and using the remaining reaction intermediate without further purification for next reaction;
(2) under the protection of nitrogen and stirring, adding 0.1% to 5% of catalyst 2 into the intermediate obtained in the first step at a room temperature, wherein the catalyst 2 is sodium methoxide, sodium formate, diethyl tin oxide or aluminum isooctanol, then adding n-dodecylamine, or n-octadecylamine, or n-dodecanol, or n-octadecanol in batches, after finishing adding, heating to 40°C to 70°C for reaction for 5 hours to 16 hours, continuously heating to 85°C to 120°C for reaction for 30 hours to 96 hours, and monitoring the reaction process by TLC until the reaction is complete; and adding ethanol, or ethyl acetate or petroleum ether containing 5% to 15% of water for recrystallizing, filtering to yield a white powder solid product, and drying, wherein a yield ranges from 85% to 97%;
a preparation method of the structural general formula 2:
(1) under the protection of nitrogen, adding methyl acrylate or methyl methacrylate into a reaction flask, starting stirring, then adding 1 part to 3 parts of methanol, or ethanol, or ethyl acetate, or dichloroethane, or acetone, or acetonitrile, or DMF, or not adding any solvent, adding 0.02% to 30% of catalyst 1, wherein the catalyst 1 is acetic acid, or acidic alumina, or silica gel, or ortho-methoxyhydroquinone, or 4,4-diphenol hydroxy dibenzophenone, or m-nitrophenol, cooling to 5°C to 10°C, and then dropwise adding second amine slowly; heating to a room temperature after dropwise adding, stirring for 6 hours to 24 hours, and if the reaction needs to be continued, continuously heating to 40°C to 80°C to continue the reaction for 5 hours to 18 hours; monitoring the reaction process by TLC until the reaction is complete, removing excessive methyl acrylate under vacuum, and using the remaining reaction intermediate without further purification for next reaction;
(2) under the protection of nitrogen and stirring, adding 0.1% to 5% of catalyst 2 into the intermediate obtained in the first step at a room temperature, wherein the catalyst 2 is sodium methoxide, sodium formate, diethyl tin oxide or aluminum isooctanol, then adding n-dodecylamine, or n-hexadecylamine, or n-octadecylamine, or n-dodecanol, or n-octadecanol in batches, after finishing adding, heating to 40°C to 70°C for reaction for 8 hours to 16 hours, continuously heating to 85°C to 140°C for reaction for 48 hours to 96 hours, and monitoring the reaction process by TLC until the reaction is complete; and removing the catalyst, adding ethanol, or methanol, or ethyl acetate, or petroleum ether containing 0.5% to 20% of water for recrystallizing, filtering to yield a white powder solid product, and drying, wherein a yield ranges from 87% to 96%;
a preparation method of the structural general formula 3:
(1) under the protection of nitrogen, adding methyl acrylate or methyl methacrylate into a reaction flask, starting stirring, then adding 1 part to 3 parts of methanol, or ethanol, or ethyl acetate, or dichloroethane, or acetone, or acetonitrile, or DMF, or not adding any solvent, then adding 0.01% to 30% of catalyst 1, wherein the catalyst 1 is acetic acid, or acidic alumina, or silica gel, or ortho-methoxyhydroquinone, or 4,4-diphenol hydroxy dibenzophenone, or m-nitrophenol, cooling to 5°C to 20°C, and then dropwise adding second amine slowly; heating to a room temperature after dropwise adding, stirring for 5 hours to 24 hours, and if the reaction needs to be continued, continuously heating to 40°C to 80°C to continue the reaction for 5 hours to 18 hours; monitoring the reaction process by TLC until the reaction is complete, removing excessive methyl acrylate under vacuum, and using the remaining reaction intermediate without further purification for next reaction;
(2) under the protection of nitrogen and stirring, adding alkyl diamine, such as ethylenediamine, or butanediamine, or hexamethylenediamine, or decanediamine in batches first into the intermediate obtained in the first step at a room temperature, then adding 0.1% to 5% of catalyst 2, wherein the catalyst 2 is sodium methoxide, sodium formate, diethyl tin oxide or aluminum isooctanol, after finishing adding, heating to 50°C to 70°C for reaction for 6 hours to 16 hours, continuously heating to 85°C to 120°C for reaction for 48 hours to 96 hours, and monitoring the reaction process by TLC until the reaction is complete; and removing the catalyst, adding ethanol, or methanol, or ethyl acetate, or petroleum ether containing 0.5% to 20% of water for recrystallizing, filtering to yield a white powder solid product, and drying, wherein a yield ranges from 85% to 95%;
a preparation method of the structural general formula 4:
(1) under the protection of nitrogen, adding methyl acrylate or methyl methacrylate into a reaction flask, starting stirring, then adding 1 part to 3 parts of methanol, or ethanol, or ethyl acetate, or dichloroethane, or acetone, or acetonitrile, or DMF, or not adding any solvent, then adding 100 ppm to 1000 ppm o-methoxyhydroquinone, or 4,4-diphenol hydroxy dibenzophenone, or m-nitrophenol, cooling to 5°C to 20°C, and then dropwise adding second amine slowly; heating to a room temperature after dropwise adding, stirring for 8 hours to 32 hours, and if the reaction needs to be continued, continuously heating to 40°C to 60°C to continue the reaction for 5 hours to 24 hours; monitoring the reaction process by TLC until the reaction is complete, removing excessive methyl acrylate under vacuum, and using the remaining reaction intermediate without further purification for next reaction;
(2) under the protection of nitrogen and stirring, adding dodecylamine, or hexadecylamine, or octadecylamine, or hexadecanol, or octadecanol, or dodecanol in batches first into the intermediate obtained in the first step at a room temperature, then adding 0.02% to 5% of catalyst 2, wherein the catalyst 2 is sodium methoxide, or sodium formate, or diethyl tin oxide, or aluminum isooctanol, after finishing adding, heating to 50°C to 70°C for reaction for 8 hours to 10 hours, continuously heating to 80°C to 120°C for reaction for 48 hours to 96 hours, and monitoring the reaction process by TLC until the reaction is complete; and removing the catalyst, adding ethanol, or methanol, or ethyl acetate, or petroleum ether containing 0.5% to 20% of water for recrystallizing, filtering to yield a white powder solid product, and drying, wherein a yield ranges from 83% to 96%;
a preparation method of the structural general formula 5:
(1) under the protection of nitrogen, adding methyl acrylate or methyl methacrylate into a reaction flask, starting stirring, then adding 1 part to 3 parts of methanol, or ethanol, or ethyl acetate, or dichloroethane, or acetone, or acetonitrile, or DMF, or not adding any solvent, then adding 100 ppm to 1000 ppm o-methoxyhydroquinone, or 4,4-diphenol hydroxy dibenzophenone, or m-nitrophenol, cooling to 10°C to 20°C, and then dropwise adding cyclic alkyl diamine slowly; heating to a room temperature after dropwise adding, stirring for 3 hours to 5 hours, heating to 40°C to 60°C to continue the reaction for 10 hours to 24 hours; monitoring the reaction process by TLC until the reaction is complete, removing excessive methyl acrylate under vacuum, and using the remaining reaction intermediate without further purification for next reaction;
(2) under the protection of nitrogen and stirring, adding octadecylamine, or dodecylamine, or hexadecylamine in batches first into the intermediate obtained in the first step at a room temperature, then adding 0.1% to 0.5% of sodium methoxide or sodium formate, or not adding any catalyst, after finishing adding, heating to 50°C to 60°C for reaction for 5 hours to 8 hours, continuously heating to 80°C to 120°C for reaction for 48 hours to 72 hours, and monitoring the reaction process by TLC until the reaction is complete; and removing the catalyst, adding ethanol or methanol containing 5% to 10% of water for recrystallizing, filtering to yield a white powder solid product, and drying, wherein a yield ranges from 83% to 92%;
a preparation method of the structural general formula 6:
(1) under the protection of nitrogen, adding methyl acrylate or methyl methacrylate into a reaction flask, starting stirring, then adding 1 part to 3 parts of methanol, or ethanol, or ethyl acetate, or dichloroethane, or acetone, or acetonitrile, or DMF, or not adding any solvent, then adding 100 ppm to 1000 ppm o-methoxyhydroquinone, or 4,4-diphenol hydroxy dibenzophenone, or m-nitrophenol, cooling to 10°C to 20°C, and then dropwise adding alkyl cyclic diamine slowly; heating to a room temperature after dropwise adding, stirring for 5 hours to 8 hours, heating to 45°C to 70°C to continue the reaction for 10 hours to 24 hours; monitoring the reaction process by TLC until the reaction is complete, removing excessive methyl acrylate under vacuum, and using the remaining reaction intermediate without further purification for next reaction;
(2) under the protection of nitrogen and stirring, adding pentanediamine, or hexamethylene diamine, or decanediamine, or hexanediol, or octanediol, or decanediol in batches first into the intermediate obtained in the first step at a room temperature, then adding 0.01% to 5% of sodium methoxide or sodium formate, or not adding any catalyst, or diethyl tin oxide, or aluminum alkoxide, after finishing adding, heating to 50°C to 70°C for reaction for 5 hours to 8 hours, continuously heating to 80°C to 130°C for reaction for 48 hours to 96 hours, and monitoring the reaction process by TLC until the reaction is complete; and removing the catalyst, adding ethanol or methanol containing 5% to 10% of water for recrystallizing, filtering to yield a white powder solid product, and drying, wherein a yield ranges from 83% to 91%; and
a preparation method of the structural general formula 7:
(1) under the protection of nitrogen and stirring, adding alkylamine, or aryl substituted alkylamine, or hydroxylamine, or alkoxyamine, or aryl substituted alkoxyamine into a reaction flask, then adding 1 part to 3 parts of methanol, or ethanol, or ethyl acetate, or dichloroethane, or acetone, or acetonitrile, or DMF, or not adding any solvent, then adding 100 ppm to 1000 ppm o-methoxyhydroquinone, or 4,4-diphenol hydroxy dibenzophenone, or m-nitrophenol, or 0.1% to 5% of NaOH, or K2CO3, or 10% to 30% of silica gel, or acidic alumina, or not adding any catalyst, cooling to 5°C to 10°C, and then dropwise adding methyl acrylate or methyl methacrylate slowly; heating to a room temperature after dropwise adding, stirring for 5 hours to 18 hours, heating to 30°C to 70°C to continue the reaction for 5 hours to 24 hours; monitoring the reaction process by TLC until the reaction is complete, removing excessive methyl acrylate, solvent and catalyst under vacuum, and using the remaining reaction intermediate without further purification for next reaction;
(2) under the protection of nitrogen and stirring, adding pentanediamine, or hexamethylene diamine, or decanediamine, or hexanediol, or octanediol, or decanediol in batches first into the intermediate obtained in the first step at a room temperature, then adding 0.01% to 5% of sodium methoxide or sodium formate, or not adding any catalyst, or diethyl tin oxide, or aluminum alkoxide, after finishing adding, heating to 50°C to 70°C for reaction for 10 hours to 18 hours, continuously heating to 80°C to 140°C for reaction for 24 hours to 96 hours, and monitoring the reaction process by TLC until the reaction is complete; and removing the catalyst, adding ethanol, or methanol, or ethyl acetate, or petroleum ether containing 5% to 10% of water for recrystallizing, filtering to yield a white powder solid product, and drying, wherein a yield ranges from 80% to 90%.

4. An application of the structure of the steric hindrance adjustable weak base light stabilizer according to claim 1 to a so-called polymer material to provide effective light stability protection and antioxidant stability protection.
